# EUROPÄISCHE PATENTANMELDUNG

(11) **EP 0 678 500 A1**
(43) Veröffentlichungstag der Anmeldung: **25.10.1995**
(21) Anmeldenummer: 95810238.6
(22) Anmeldetag: 07.04.1995
(51) Int. Cl.: C07C 229/36, C07C 271/22, C07C 271/16, C07C 271/18, C07D 319/18, C07D 241/08

(54) **Alpha-Aminoalkansäuren und Reduktionsprodukte als Zwischen-Produkte in der Herstellung von Renininhibitoren**

(30) Priorität: 18.04.1994 CH 1169/94; 30.01.1995 CH 247/95
(71) Anmelder: CIBA-GEIGY AG, CH-4002 Basel (CH)
(72) Erfinder: Göschke, Richard, Dr., CH-4103 Bottmingen (CH)

(57) **Zusammenfassung**

Verbindungen der Formel I 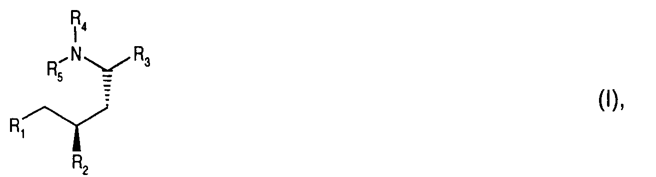
worin
R₁ einen aliphatischen, cycloaliphatischen, cycloaliphatisch-aliphatischen, aromatischen oder heteroaromatischen Rest, eine aliphatisch, araliphatisch oder heteroarylaliphatisch veretherte oder durch eine Hydroxyschutzgruppe geschützte Hydroxygruppe oder eine aliphatisch veretherte Mercaptogruppe und R₂ einen aliphatischen, cycloaliphatischen, cycloaliphatisch-aliphatischen, araliphatischen oder heteroarylaliphatischen Rest bedeutet oder R₁ und R₂ gemeinsam einen zweiwertigen aliphatischen Rest darstellen, R₃ für gegebenenfalls aliphatisch, araliphatisch oder aromatisch verestertes Carboxy, Formyl oder Hydroxymethyl steht, R₄ Wasserstoff, einen aliphatischen oder araliphatischen Rest oder eine Aminoschutzgruppe bedeutet und R₅ Wasserstoff oder einen aliphatischen Rest darstellt,
und ihre Salze können als Zwischenprodukte für die Herstellung von Arzneimittelwirkstoffen verwendet werden.

## Beschreibung

Die Erfindung betrifft Verbindungen der Formel I 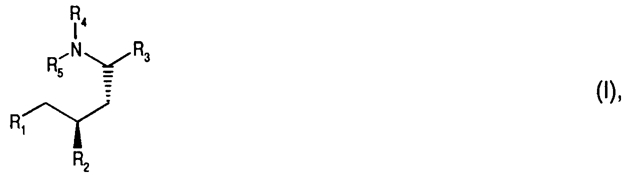
worin
R₁ einen aliphatischen, cycloaliphatischen, cycloaliphatisch-aliphatischen, aromatischen oder heteroaromatischen Rest, eine aliphatisch, araliphatisch oder heteroarylaliphatisch veretherte oder durch eine Hydroxyschutzgruppe geschützte Hydroxygruppe oder eine aliphatisch veretherte Mercaptogruppe und
R₂ einen aliphatischen, cycloaliphatischen, cycloaliphatisch-aliphatischen, araliphatischen oder heteroarylaliphatischen Rest bedeutet oder
R₁ und R₂ gemeinsam einen zweiwertigen aliphatischen Rest darstellen,
R₃ für gegebenenfalls aliphatisch, araliphatisch oder aromatisch verestertes Carboxy, Formyl oder Hydroxymethyl steht,
R₄ Wasserstoff, einen aliphatischen oder araliphatischen Rest oder eine Aminoschutzgruppe bedeutet und
R₅ Wasserstoff oder einen aliphatischen Rest darstellt,
und ihre Salze, Verfahren zur Herstellung der erfindungsgemäßen Verbindungen und ihre Verwendung als Zwischenprodukte für die Herstellung von Arzneimittelwirkstoffen.

Aliphatische Reste sind beispielsweise Niederalkylreste, im Falle von R₁ auch Niederalkenylreste.

Cycloaliphatische Reste sind beispielsweise Cycloalkylreste.

Cycloaliphatisch-aliphatische Reste sind beispielsweise Cycloalkylniederalkylreste.

Araliphatische Reste sind beispielsweise gegebenenfalls substituierte Phenyl- oder Naphthylniederalkylreste. Als Substituenten kommen dabei beispielsweise 1, 2 oder 3, insbesondere 1 oder 2 Substituenten, ausgewählt z.B. aus Niederalkyl, Niederalkoxy, Halogen und/oder Trifluormethyl, in Betracht.

Aromatische Reste sind beispielsweise gegebenenfalls substituierte Phenyl- oder Naphthylrest. Als Substituenten von aromatischen Resten R₁ kommen beispielsweise 1, 2 oder 3, insbesondere 2 oder 3 Substituenten, ausgewählt aus Aminoniederalkoxy, Aminoniederalkyl, Arylniederalkoxy, Carbamoylniederalkoxy, Carbamoylniederalkyl, Carboxyniederalkoxy, Cyanoniederalkoxy, Cyanoniederalkyl, Cycloalkoxy, Cycloalkoxyniederalkoxy, Cycloalkoxyniederalkyl, Cycloalkyl, Diniederalkylamino, Diniederalkylaminoniederalkoxy, Diniederalkylaminoniederalkyl, gegebenenfalls partiell hydriertes Heteroarylniederalkoxy, gegebenenfalls N-oxidiertes Pyridylthioniederalkoxy, gegebenenfalls N-oxidiertes Pyridylthioniederalkyl, Halogen(hydroxy)niederalkoxy, Halogen, Hydroxy, Hydroxyniederalkoxy, Hydroxyniederalkyl, Imidazolylthioniederalkoxy, Imidazolylthioniederalkyl, Morpholinoniederalkoxy, Morpholinoniederalkyl, N-Mono- oder N,N-Diniederalkylcarbamoylniederalkoxy, N-Mono- oder N,N-Diniederalkylcarbamoylniederalkyl, gegebenenfalls N-oxidierte Pyridylniederalkyl, N'-Niederalkylpiperazino- bzw. N'-Niederalkanoylpiperazinoniederalkoxy, N'-Niederalkylpiperazino- bzw. N'-Niederalkanoylpiperazinoniederalkyl, Naphthyl, Naphthylniederalkoxy, Niederalkanoylaminoniederalkoxy, Niederalkanoylaminoniederalkyl, Niederalkanoylniederalkoxy, Niederalkanoyloxyniederalkyl, Niederalkansulfonyl(hydroxy)niederalkoxy, Niederalkansulfonylaminoniederalkoxy, Niederalkansulfonylaminoniederalkyl, Niederalkansulfonylniederalkoxy, Niederalkansulfonylnieder alkyl, Niederalkenyloxy, Niederalkenyloxyniederalkoxy, Niederalkenyloxyniederalkyl, Niederalkoxy, Niederalkoxycarbonylaminoniederalkoxy, Niederalkoxycarbonylaminoniederalkyl, Niederalkoxycarbonylniederalkoxy, Niederalkoxycarbonylniederalkyl, Niederalkoxyiminoniederalkyl, Niederalkoxyniederalkenyl, Niederalkoxyniederalkenyloxy, Niederalkoxyniederalkoxy, Niederalkoxyniederalkoxyniederalkyl, Niederalkoxyniederalkyl, Niederalkyl, Niederalkylamino, Niederalkylaminoniederalkoxy, Niederalkylaminoniederalkyl, Niederalkylthio(hydroxy)niederalkoxy, Niederalkylthioniederalkoxy, Niederalkylthioniederalkoxy, Niederalkylthioniederalkyl, Oxoniederalkoxy, Priperazinoniederalkoxy, Piperazinoniederalkyl, Piperidinoniederalkoxy, Piperidinoniederalkyl, Poyhalogenniederalkansulfonylaminoniederalkoxy, Polyhalogenniederalkansulfonylaminoniederalkyl, Polyhalogenniederalkoxy, Polyhalogenniederalkyl, Pyrimidinylthioniederalkoxy, Pyrimidinylthioniederalkoxy, Pyrimidinylthioniederalkyl, Pyrrolidinoniederalkoxy, Pyrrolidinoniederalkyl, S,S-Dioxothiomorpholinoniederalkoxy, S,S-Dioxothiomorpholinoniederalkyl, S-Oxothiomorpholinoniederalkoxy, S-Oxothiomorpholinoniederalkyl, Thiazolylthioniederalkoxy, Thiazolinylthioniederalkoxy, Thiazolylthioniederalkyl, Thiazolinylthioniederalkyl und/oder Thiomorpholino sowie ankondensierten Benzo- oder Cyclohexenoringen, in Betracht.

Aromatische Reste R₁ sind beispielsweise solche der Formel 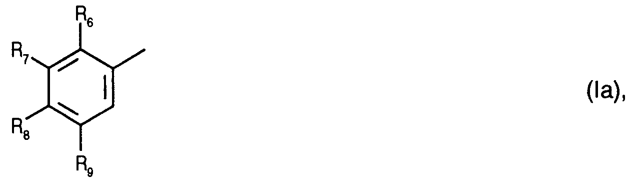
worin
R₆ Wasserstoff, Hydroxy, Niederalkoxy, Cycloalkoxy, Niederalkoxyniederalkoxy, Carboxyniederalkoxy, Niederalkoxycarbonylniederalkoxy, Carbamoylniederalkoxy oder N-Mono- oder N,N- Diniederalkylcarbamoylniederalkoxy bedeutet,
R₇ Wasserstoff, Niederalkyl, Cycloalkyl, Niederalkoxyniederalkyl, Niederalkoxyniederalkoxyniederalkyl, Cycloalkoxyniederalkyl, Hydroxy, Niederalkanoyloxyniederalkyl, Hydroxyniederalkoxy, Halogen(hydroxy)niederalkoxy, Niederalkansulfonyl(hydroxy)niederalkoxy, Aminoniederalkyl, Niederalkylaminoniederalkyl, Diniederalkylaminoniederalkyl, Niederalkanoylaminoniederalkyl, Niederalkoxycarbonylaminoniederalkyl, Niederalkoxyiminoniederalkyl, Aminoniederalkoxy, Niederalkylaminoniederalkoxy, Diniederalkylaminoniederalkoxy, Niederalkanoylaminoniederalkoxy, Niederalkoxycarbonylaminoniederalkoxy, Oxoniederalkoxy, Niederalkoxy, Cycloalkoxy, Niederalkenyloxy, Cycloalkoxyniederalkoxy, Niederalkoxyniederalkoxy, Niederalkoxyniederalkenyl, Niederalkenyloxyniederalkoxy, Niederalkoxyniederalkenyloxy, Niederalkenyloxyniederalkyl, Niederalkanoylniederalkoxy, Niederalkylthioniederalkoxy, Niederalkansulfonylniederalkoxy, Niederalkylthio(hydroxy)niederalkoxy, Arylniederalkoxy, Thiazolylthioniederalkoxy bzw. Thiazolinylthioniederalkoxy, midazolylthioniederalkoxy, gegebenenfalls N-oxidierte Pyridylthioniederalkoxy, Pyrimidinylthioniederalkoxy, Cyanoniederalkoxy, Niederalkoxycarbonylniederalkoxy, Carbamoylniederalkoxy, N-Mono- oder N,N-Diniederalkylcarbamoylniederalkoxy, Carboxyniederalkyl, Niederalkoxycarbonylniederalkyl, Carbamoylniederalkyl oder N-Mono- oder N,N-Diniederalkylcarbamoylniederalkyl bedeutet,
R₈ Niederalkyl, Polyhalogenniederalkyl, Niederalkoxyniederalkyl, Cycloalkoxyniederalkyl, Hydroxyniederalkyl, Niederalkylthioniederalkyl, Niederalkansulfonylniederalkyl, gegebenenfalls partiell hydriertes oder N-oxidierte Pyridylniederalkyl, Thiazolylthioniederalkyl bzw. Thiazolinylthioniederalkyl, Imidazolylthioniederalkyl, gegebenenfalls N-oxidiertes Pyridylthioniederalkyl, Pyrimidinylthioniederalkyl, Aminoniederalkyl, Niederalkylaminoniederalkyl, Diniederalkylaminoniederalkyl, Niederalkanoylaminoniederalkyl, Niederalkansulfonylaminoniederalkyl, Polyhalogenniederalkansulfonylaminoniederalkyl, Pyrrolidinoniederalkyl, Piperidinoniederalkyl, Piperazino-, N'-Niederalkylpiperazino- bzw. N'-Niederalkanoylpiperazinoniederalkyl, Morpholinoniederalkyl, Thiomorpholino-, S-Oxothiomorpholino- oder S,S-Dioxothiomorpholinoniederalkyl, Cyanoniederalkyl, Carboxyniederalkyl, Niederalkoxycarbonylniederalkyl, Carbamoylniederalkyl, N-Mono- oder N,N-Diniederalkylcarbamoylniederalkyl, Cycloalkyl, unsubstituiertes oder durch Niederalkyl, Niederalkoxy, Hydroxy, Niederalkylamino, Diniederalkylamino, Halogen und/oder Trifluormethyl mono-, di- oder trisubstituiertes Phenyl oder Naphthyl, Hydroxy, Niederalkoxy, Cycloalkoxy, Niederalkoxyniederalkoxy, Cycloalkoxyniederalkoxy, Hydroxyniederalkoxy, unsubstituiertes oder durch Niederalkyl, Niederalkoxy, Hydroxy, Niederalkylamino, Diniederalkylamino, Halogen und/oder Trifluormethyl mono-, di- oder trisubstituiertes Phenylniederalkoxy oder Naphthylniederalkoxy, Niederalkoxy, Polyhalogenniederalkoxy, Niederalkylthioniederalkoxy, Niederalkansulfonylnieralkoxy, gegebenenfalls hydriertes Heteroarylniederalkoxy, gegebenenfalls partiell oder vollständig hydriertes Heteroarylthioniederalkoxy, wie Thiazolylthioniederalkoxy bzw. Thiazolinylthioniederalkoxy, Imidazolylthioniederalkoxy, gegebenenfalls N-oxidierte Pyridylthioniederalkoxy, Pyrimidinylthioniederalkoxy, Aminoniederalkoxy, Niederalkylaminoniederalkoxy, Diniederalkylaminoniederalkoxy, Niederalkanoylaminoniederalkoxy, Niederalkansulfonylaminoniederalkoxy, Polyhalogenniederalkansulfonylaminoniederalkoxy, Pyrrolidinoniederalkoxy, Piperidinoniederalkoxy, Piperazino-, N'-Niederalkylpiperazino- bzw. N'-Niederalkanoylpiperazinoniederalkoxy, Morpholinoniederalkoxy, Thiomorpholino-, S-Oxothiomorpholino- oder S,S-Dioxothiomorpholinoniederalkoxy, Cyanoniederalkoxy, Carboxyniederalkoxy, Niederalkoxycarbonylniederalkoxy, Carbamoylniederalkoxy oder N-Mono- oder N,N-Diniederalkylcarbamoylniederalkoxy bedeutet oder gemeinsam mit R₉ Niederalkylendioxy oder einen ankondensierten Benzo- oder Cyclohexenoring darstellt,
R₉ gemeinsam mit R₈ Niederalkylendioxy oder einen ankondensierten Benzo- oder Cyclohexenoring darstellt oder für Wasserstoff, Niederalkyl, Hydroxy, Niederalkoxy oder Cycloalkoxy steht.

Heteroaromatische Reste sind beispielsweise gegebenenfalls substituierte 5- oder 6-gliedrige monocyclische oder aus 5- oder 6-gliedrigen Ringen zusammengesetzte Heteroarylreste, wie Furyl, Thienyl, Pyridyl, Pyrimidinyl, Indolyl oder Chinolinyl. Als Substituenten kommen dabei beispielsweise 1, 2 oder 3, insbesondere 1 oder 2 Substituenten, ausgewählt z.B. aus Niederalkyl, Niederalkoxy, Halogen und/oder Trifluormethyl, in Betracht.

Gegebenenfalls hydriertes Heteroarylniederalkoxy ist beispielsweise gegebenenfalls partiell hydriertes oder N-oxidiertes Pyridylniederalkoxy, Thiazolylniederalkoxy oder vor allem Morpholinoniederalkoxy.

Aliphatisch veretherte Hydroxygruppen sind beispielsweise Niederalkoxy- oder Niederalkenyloxygruppen.

Araliphatisch veretherte Hydroxygruppen sind beispielsweise unsubstituierte oder im Phenylteil substituierte Phenylniederalkoxygruppen. Als Substituenten kommen dabei beispielsweise 1, 2 oder 3, insbesondere 1 oder 2 Substituenten, ausgewählt z.B. aus Niederalkyl, Niederalkoxy, Halogen und/oder Trifluormethyl, in Betracht.

Heteroarylaliphatisch veretherte Hydroxygruppen sind beispielsweise mit einem gegebenenfalls substituierten 5- oder 6-gliedrige monocyclischen oder aus 5- oder 6-gliedrigen Ringen zusammengesetzten heteroarylaliphatischen Alkohol veretherte Hydroxygruppen, wie gegebenenfalls substituierte Furylniederalkoxy-, Thienylniederalkoxy-, Pyridylniederalkoxy- oder Chinolinylniederalkoxygruppen. Als Substituenten kommen dabei beispielsweise 1, 2 oder 3, insbesondere 1 oder 2 Substituenten, ausgewählt z.B. aus Niederalkyl, Niederalkoxy, Halogen und/oder Trifluormethyl, in Betracht.

Durch eine Hydroxyschutzgruppe geschützte Hydroxygruppen sind beispielsweise durch eine von einer aliphatischen oder aromatischen Carbonsäure oder einem aliphatischen oder araliphatischen Halbester der Kohlensäure abgeleiteten Acylgruppe oder einer aliphatisch und/oder araliphatisch substituierte Silylgruppe geschützt. Zu nennen sind beispielsweise Niederalkanoyloxy, Trihalogenniederalkanoyloxy, wie Trifluoracetoxy, gegebenenfalls, z.B. durch Niederalkyl, Niederalkoxy, Halogen, Trifluormethyl und/oder Nitro, substituierte Benzoyloxy- oder Phenylniederalkoxycarbonyloxygruppen, Triniederalkylsilyloxy oder Benzyl(diniederalkyl)silyloxy.

Aliphatisch veretherte Mercaptogruppen sind beispielsweise Niederalkylthiogruppen.

Araliphatische Reste sind beispielsweise Phenylniederalkyl- oder Naphthylniederalkylreste. Als Substituenten kommen dabei beispielsweise 1, 2 oder 3, insbesondere 1 oder 2 Substituenten, ausgewählt z.B. aus Niederalkyl, Niederalkoxy, Halogen und/oder Trifluormethyl, in Betracht.

Heteroarylaliphatische Reste sind beispielsweise gegebenenfalls substituierte 5- oder 6- gliedrige monocyclische oder aus 5- oder 6-gliedrigen Ringen zusammengesetzte Heteroarylniederalkylreste, wie Furylniederalkyl Thienylniederalkyl, gegebenenfalls partiell hydriertes oder N-oxidiertes Pyridylniederalkyl, Pyrimidinylniederalkyl oder Chinolinylniederalkyl. Als Substituenten kommen dabei beispielsweise 1, 2 oder 3, insbesondere 1 oder 2 Substituenten, ausgewählt z.B. aus Niederalkyl, Niederalkoxy, Halogen und/oder Trifluormethyl, in Betracht.

Durch R₁ und R₂ gemeinsam dargestellte zweiwertigen aliphatische Reste sind beispielsweise Niederalkylenreste, deren freie Valenzen von benachbarten oder zueinander 1,3-, 1,4- oder 1,5-ständigen C-Atomen ausgehen.

Gegebenenfalls aliphatisch, araliphatisch oder aromatisch verestertes Carboxy ist beispielsweise Carboxy, Niederalkoxycarbonyl, Niederalkenyloxycarbonyl oder gegebenenfalls substituierte Phenylniederalkoxycarbonyl- oder Phenyloxycarbonylreste. Als Substituenten der beiden letztgenannten kommen beispielsweise 1, 2 oder 3, insbesondere 1 oder 2 Substituenten, ausgewählt z.B. aus Niederalkyl, Niederalkoxy, Halogen und/oder Trifluormethyl, in Betracht.

Aminoschutzgruppen sind beispielsweise von einer aliphatischen oder aromatischen Carbonsäure oder einem aliphatischen oder araliphatischen Halbester der Kohlensäure abgeleiteten Acylgruppen oder aliphatisch und/oder araliphatisch substituierte Silylgruppen. Zu nennen sind beispielsweise Niederalkanoyl, Trihalogenniederalkanoyl, wie Trifluoracetyl, gegebenenfalls, z.B. durch Niederalkyl, Niederalkoxy, Halogen, Trifluormethyl und/oder Nitro, substituierte Benzoyl- oder Phenylniederalkoxycarbonylgruppen, Triniederalkylsilyl oder Benzyl(diniederalkyl)silyl.

Vor- und nachstehend sind unter niederen Resten und Verbindungen beispielsweise solche zu verstehen, die bis und mit 7, vorzugsweise bis und mit 4, Kohlenstoffatome (C-Atome) aufweisen.

Aminoniederalkyl ist beispielsweise Amino-C₁-C₄-alkyl, wie 2-Aminoethyl, 3-Aminopropyl oder 4-Aminobutyl.

Arylniederalkoxy ist beispielsweise Phenyl-C₁-C₄-alkoxy, wie Benzyloxy, 1- oder 2-Phenylethoxy, 3-Phenylpropyloxy oder 4-Phenylbutyloxy.

Benzyl(diniederalkyl)silyl ist beispielsweise Benzyl(di-C₁-C₄-alkyl)silyl, wie insbesondere Benzyl(dimethyl)silyl.

Carbamoylniederalkoxy ist beispielsweise Carbamoyl-C₁-C₄-alkoxy, wie Carbamoylmethoxy, 2-Carbamoylethoxy, 3-Carbamoylpropyloxy oder 4-Carbamoylbutyloxy, insbesondere Carbamoylmethoxy.

Carbamoylniederalkyl ist beispielsweise Carbamoyl-C₁-C₇-alkyl, wie Carbamoylmethyl, 2-Carbamoylethyl, 3-Carbamoylpropyl, 2-(3-Carbamoyl)propyl, 2-Carbamoylpropyl 3-(1-Carbamoyl)propyl, 2-(2-Carbamoyl)propyl, 2-(Carbamoyl-2-methyl)propyl, 4-Carbamoylbutyl, 1-Carbamoylbutyl, 1-(1-Carbamoyl-2-methyl)butyl, 3-(4-Carbamoyl-2-methyl)butyl.

Carboxyniederalkoxy ist beispielsweise Carboxy-C₁-C₄-alkoxy, wie Carboxymethoxy, 2-Carboxyethoxy, 2- oder 3-Carboxypropyloxy oder 4-Carboxybutyloxy, insbesondere Carboxymethoxy.

Carboxyniederalkyl ist beispielsweise Carboxy-C₁-C₄-alkyl, wie Carboxymethyl, 2-Carboxyethyl, 2- oder 3-Carboxypropyl, 2-Carboxy-2-methyl-propyl, 2-Carboxy-2-ethyl-butyl oder 4-Carboxybutyl, insbesondere Carboxymethyl.

Chinolinylniederalkoxy ist beispielsweise Chinolinyl-C₁-C₄-alkoxy, wie Chinolinylmethoxy, 2-Chinolinylethoxy, 3- Chinolinylpropyloxy oder 4- Chinolinylbutyloxy, insbesondere Chinolinylmethoxy.

Cyanoniederalkoxy ist beispielsweise Cyano-C₁-C₄-alkoxy, wie Cyanomethoxy, 2-Cyano-ethoxy, 2- oder 3-Cyanopropyloxy oder 4-Cyanobutyloxy, insbesondere Cyanomethoxy.

Cyanoniederalkyl ist beispielsweise Cyano-C₁-C₄-alkyl, wie Cyanomethyl, 2-Cyanoethyl, 2- oder 3-Cyanopropyl, 2-Cyano-2-methyl-propyl, 2-Cyano2-ethyl-butyl oder 4-Cyanobutyl, insbesondere Cyanomethyl.

Cycloalkoxy ist beispielsweise 3- bis 8-gliedriges, insbesondere 3- bis 6-gliedriges, Cycloalkoxy, wie Cyclopropyloxy, Cyclobutyloxy, Cyclopentyloxy oder Cyclohexyloxy.

Cycloalkoxyniederalkoxy ist beispielsweise 3- bis 8-gliedriges, insbesondere 3- bis 6-gliedriges, Cycloalkoxy-C₂-C₄-alkoxy, wie 2-Cyclopropyloxyethoxy, 2-Cyclobutyloxyethoxy, 2-Cyclopentyloxyethoxy oder 2-Cyclohexyloxyethoxy.

Cycloalkoxyniederalkyl ist beispielsweise 3- bis 8-gliedriges, insbesondere 3- bis 6-gliedriges, Cycloalkoxy-C₂-C₄-alkyl, wie Cyclopropyloxymethyl, Cyclobutyloxymethyl, Cyclopentyloxymethyl oder Cyclohexyloxymethyl.

3- bis 8-gliedriges Cycloalkyl ist beispielsweise 3- bis 8-gliedriges, insbesondere 3- bis 6-gliedriges, Cycloalkyl, wie Cyclopropyl, Cyclobutyl, Cyclopentyl oder Cyclohexyl.

Cycloalkylniederalkyl ist beispielsweise 3- bis 8-gliedrige, im Falle von R₁ insbesondere 5-bis 7-gliedriges Cycloalkyl-C₁-C₄-alkyl, wie Cyclopentylmethyl, Cyclohexylmethyl oder Cycloheptylmethyl, und im Falle von R₂ insbesondere 3 bis 5-gliedriges Cycloalkyl-C₁-C₄-alkyl, wie Cyclopropylmethyl, Cyclobutylmethyl oder Cyclohexylmethyl.

Diniederalkylamino ist beispielsweise Di-C₁-C₄-alkylamino, wie Dimethylamino, N-Methyl-N-ethylamino, Diethylamino, N-Methyl-N-propylamino oder N-ButylN-methylamino.

Diniederalkylaminoniederalkoxy ist beispielsweise N,N-Di-C₁-C₄-alkylamino-C₁-C₄-alkoxy, wie 2-Dimethylaminoethoxy, 3-Dimethylaminopropyloxy, 4-Dimethylaminobutyloxy, 2-Diethylaminoethoxy, 2-(N-Methyl-N-ethyl-amino)ethoxy oder 2-(N-Butyl-N-methylamino)ethoxy.

Diniederalkylaminoniederalkyl ist beispielsweise N,N-Di-C₁-C₄-alkylamino-C₁-C₄-alkyl, wie 2-Dimethylaminoethyl, 3-Dimethylaminopropyl, 4-Dimethylaminobutyl, 2-Diethylaminoethyl, 2-(N-Methyl-N-ethyl-amino)ethyl oder 2-(N-Butyl-N-methyl-amino)ethyl.

Furylniederalkoxyniederalkoxy ist beispielsweise Furyl-C₁-C₄-alkoxy-C₂-C₄-alkoxy, wie 2-(Furylmethoxy)ethoxy, 2-(2-Furylethoxy)ethoxy, 3-(Furylmethoxy)propyloxy oder 4-(Furylmethoxy)butyloxy.

Furylniederalkyl ist beispielsweise Furyl-C₁-C₄-alkyl, wie Furylmethyl, 1-Furylethyl, 2-Furylethyl, 3-Furylpropyl oder 4-Furylbutyl.

Gegebenenfalls partiell hydriertes oder N-oxidiertes Pyridylniederalkyl ist beispielsweise gegebenenfalls partiell hydriertes Pyridyl oder N-Oxidopyridyl-C₁-C₄-alkyl, wie Pyridyl- oder N-Oxidopyridylmethyl, 2-Pyridylethyl, 2- oder 3-Pyridylpropyl oder 4-Pyridylbutyl, insbesondere 3- oder 4-Pyridylmethyl.

Gegebenenfalls N-oxidiertes Pyridylthioniederalkoxy ist beispielsweise Pyridylthio-C₁-C₄-alkoxy oder N-Oxidopyridylthio-C₁-C₄-alkoxy, wie Pyridylthio- oder N-Oxidopyridylthiomethoxy, 2-Pyridylthioethoxy, 2- oder 3-Pyridylthiopropyloxy oder 4-Pyridylthiobutyloxy, insbesondere 3- oder 4-Pyridylthiomethoxy.

Gegebenenfalls N-oxidiertes Pyridylthioniederalkyl ist beispielsweise Pyridylthio-C₁-C₄-alkyl oder N-Oxidopyridylthio-C₁-C₄-alkyl, wie Pyridylthio- oder N-Oxidopyridylthiomethyl, 2-Pyridylthioethyl, 2- oder 3-Pyridylthiopropyl oder 4-Pyridylthiobutyl, insbesondere 3- oder 4-Pyridylthiometh.

Halogen ist beispielsweise Halogen der Atomnummer bis und mit 35, wie Chlor oder Brom, ferner Fluor.

Halogen(hydroxy)niederalkoxy ist beispielsweise Halogen-C₂-C₇-(hydroxy)alkoxy, insbesondere Halogen-C₂-C₄-(hydroxy)alkoxy, wie 3-Halogen-, wie 3-Chlor-2-hydroxy-propyloxy.

Hydroxyniederalkoxy ist beispielsweise Hydroxy-C₂-C₇-alkoxy, insbesondere Hydroxy-C₂-C₄-alkoxy, wie 2-Hydroxybutyloxy, 3-Hydroxypropyloxy oder 4-Hydroxybutyloxy.

Hydroxyniederalkyl ist beispielsweise Hydroxy-C₂-C₇-alkyl, insbesondere Hydroxy-C₂-C₄-alkyl, wie 2-Hydroxyethyl, 3-Hydroxypropyl oder 4-Hydroxybutyl.

Imidazolylniederalkoxy ist beispielsweise Imidazolyl-C₁-C₄-alkoxy, wie Imidazol-4-ylmethoxy, 2-(Imidazol-4-yl)ethoxy, 3-(Imidazol-4-yl)propyloxy oder 4-(Imidazol-4-yl)butyloxy.

Imidazolylniederalkyl ist beispielsweise Imidazolyl-C₁-C₄-alkyl, wie Imidazol-4-ylmethyl, 2-(Imidazol-4-yl)ethyl, 3-(Imidazol-4-yl)propyl oder 4-(Imidazol-4-yl)butyl.

Imidazolylthioniederalkoxy ist beispielsweise Imidazolylthio-C₁-C₄-alkoxy, wie Imidazolthio-4-ylmethoxy, 2-(Imidazol-4-ylthio)ethoxy, 3-(Imidazol-4-ylthio)propyloxy oder 4-(Imidazolthio-4-yl)butyloxy.

Imidazolylthioniederalkyl ist beispielsweise Imidazolylthio-C₁-C₄-alkyl, wie Imidazolthio-4-ylmethyl, 2-(Imidazolthio-4-yl)ethyl, 3-(Imidazolthio-4-yl)propyl oder 4-(Imidazolthio-4-yl)butyl.

Morpholinoniederalkoxy kann N-oxidiert sein und ist beispielsweise Morpholino-C₁-C₄-alkoxy, wie 1-Morpholinoethoxy, 3-Morpholinopropyloxy, oder 1-(Morpholino-2-methyl)propyloxy.

Morpholinoniederalkyl kann N-oxidiert sein und ist beispielsweise Morpholino-C₁-C₄-alkyl, wie Morpholinomethyl, 2-Morpholinoethyl, 3-Morpholinopropyl oder 1- oder 2-(4-Morpholino)butyl.

N'-Niederalkanoylpiperazinoniederalkoxy ist beispielsweise N'-Niederalkanoylpiperazino-C₁-C₄-alkoxy, wie 4-Acetylpiperazinomethoxy.

N'-Niederalkanoylpiperazinoniederalkyl ist beispielsweise N'-C₂-C₇-noylpiperazino-C₁-C₄-alkyl, wie 4-Acetylpiperazinomethyl.

N'-Niederalkylpiperazinoniederalkyl ist beispielsweise N'-C₁-C₄-Alkylpiperazino-C₁-C₄-alkyl, wie 4-Methylpiperazinomethyl.

Diniederalkylcarbamoylniederalkoxy ist beispielsweise N,N-Di-C₁-C₄-alkylcarbamoyl-C₁-C₄-alkoxy, wie Methyl- oder Dimethylcarbamoyl-C₁-C₄-alkoxy, wie N,N-Dimethylcarbamoyl-methoxy, 2-(N,N-Dimethylcarbamoyl)ethoxy, 3-(N,N-Dimethylcarbamoyl)propyloxy oder 4-(N, N-Dimethylcarbamoyl)butyloxy, insbesondere N, N-Dimethylcarbamoylmethoxy.

Diniederalkylcarbamoylniederalkyl ist beispielsweise N,N-Di-C₁-C₄-alkylcarbamoyl-C₁-C₄-alkyl, wie 2-Dimethylcarbamoylethyl, 3-Dimethylcarbamoylpropyl, 2-Dimethylcarbamoyl-propyl, 2-(Dimethylcarbamoyl-2-methyl)propyl oder 2-(1-Dimethylcarbamoyl-3-methyl)butyl.

Naphthylniederalkoxy ist beispielsweise Naphthyl-C₁-C₄-alkoxy, wie Naphthylmethoxy, 2-Naphthylethoxy, 3- Naphthylpropyloxy oder 4- Naphthylbutyloxy.

Naphthylniederalkyl ist beispielsweise Naphthyl-C₁-C₄-alkyl, wie Naphthylmethyl, 2-Naphthylethyl, 3- Naphthylpropyl oder 4- Naphthylbutyl.

Niederalkoxy ist beispielsweise C₁-C₇-Alkoxy, vorzugsweise C₁-C₅-Alkoxy, wie Methoxy, Ethoxy, Propyloxy, lsopropyloxy, Butyloxy, lsobutyloxy, Sekundärbutyloxy, Tertiärbutyloxy, Pentyloxy oder eine Hexyloxy- oder Heptyloxygruppe.

Niederalkanoylaminoniederalkoxy ist beispielsweise C₁-C₇-Alkanoylamino-C₂-C₄-alkoxy, wie 2-Formylaminoethoxy, 2-Acetylaminoethoxy, 2-Propionylaminoethoxy, 2-Butyrylaminoethoxy, 2-lsobutyrylaminoethoxy, 2-Pivaloylaminoethoxy, 3-Formylaminopropyloxy, 3-Acetylaminopropyloxy, 3-Propionylaminopropyloxy, 3-Butyrylaminopropyloxy, 3-Isobutyrylaminopropyloxy oder 3-Pivaloylaminopropyloxy.

Niederalkanoylaminoniederalkyl ist beispielsweise C₁-C₇-Alkanoylamino-C₂-C₄-alkyl, wie 2-Formylaminoethyl, 2-Acetylaminoethyl, 2-Propionylaminoethyl, 2-Butyrylaminoethyl, 2-lsobutyrylaminoethyl, 2-Pivaloylaminoethyl, 3-Formylaminopropyl, 3-Acetylaminopropyl, 3-Propionylaminopropyl, 3-Butyrylaminopropyl, 3-Isobutyrylaminopropyl oder 3-Pivaloylaminopropyl.

Niederalkanoyl niederalkoxy (Oxoniederalkoxy) trägt die Niederalkanoylgruppe in höherer als der α-Stellung und ist beispielsweise C₁-C₇-Alkanoyl-C₁-C₄-alkoxy, wie 4-Acetylbutoxy.

Niederalkanoyloxy ist beispielsweise C₁-C₇-Alkanoyloxy, wie Formyloxy, Acetyloxy, Propionyloxy, Butyryloxy, Isobutyryloxy oder Pivaloyloxy.

Niederalkanoyloxyniederalkyl trägt die Niederalkanoyloxygruppe in höherer als der α-Stellung und ist beispielsweise C₁-C₇-Alkanoyloxy-C₁-C₄-alkyl, wie 4-Acetoxybutyl.

Niederalkansulfonyl(hydroxy)niederalkoxy ist beispielsweise C₁-C₇-Alkansulfonyl-C₁-C₄-(hydroxy)alkoxy, wie 3-Methansulfonyl-2-hydroxy-propyloxy.

Niederalkansulfonylaminoniederalkoxy ist beispielsweise C₁-C₇-Alkansulfonylamino-C₂-C₄-alkoxy, wie 2-Methansulfonylaminoethoxy, 3-Methansulfonylaminopropyloxy oder 4-Methansulfonylaminobutyloxy.

Niederalkansulfonylaminoniederalkyl ist beispielsweise C₁-C₇-Alkansulfonylamino-C₁-C₄-alkyl, wie Ethansulfonylaminomethyl, 2-Ethansulfonylaminoethyl, 3-Ethansulfonylaminopropyl oder 3-(1,1 -Dimethylethansulfonylamino)propyl.

Niederalkansulfonylaminoniederalkyl ist beispielsweise C₁-C₇-Alkansulfonylamino-C₂-C₄-alkyl, wie 2-Ethansulfonylaminoethyl, 3-Ethansulfonylaminopropyl oder 4-Methansulfonylaminobutyl.

Niederalkansulfonylniederalkoxy ist beispielsweise C₁-C₇-Alkansulfonyl-C₁-C₄-alkoxy, wie Methansulfonylmethoxy oder 3-Methansulfonyl-2-hydroxy-propyloxy.

Niederalkansulfonylniederalkyl ist beispielsweise C₁-C₇-Alkansulfonyl-C₁-C₄-alkyl, wie Ethansulfonylmethyl, 2-Ethansulfonylethyl, 3-Ethansulfonylpropyl oder 3-(1,1-Dimethylethansulfonyl)propyl.

Niederalkenyl ist beispielsweise C₁-C₇-Alkenyl, wie Vinyl oder Allyl.

Niederalkenyloxy ist beispielsweise C₁-C₇-Alkenyloxy, wie Allyloxy.

Niederalkenyloxycarbonyl ist beispielsweise C₁-C₇-Alkenyloxycarbonyl, wie Vinyloxycarbonyl oder Allyloxycarbonyl.

Niederalkenyloxyniederalkoxy ist beispielsweise C₁-C₇-Alkenyloxy-C₁-C₄-alkoxy, wie Allyloxymethoxy.

Niederalkenyloxyniederalkyl ist beispielsweise C₁-C₇-Alkenyloxy-C₁-C₄-alkyl, wie Allyloxymethyl.

Niederalkoxy ist beispielsweise C₁-C₇-Alkoxy, vorzugsweise C₁-C₅-Alkoxy, wie Methoxy, Ethoxy, Propyloxy, Isopropyloxy, Butyloxy, Isobutyloxy, Sekundärbutyloxy, Tertiärbutyloxy, Pentyloxy oder eine Hexyloxy- oder Heptyloxygruppe.

Niederalkoxycarbonyl ist beispielsweise C₁-C₇-Alkoxycarbonyl, vorzugsweise C₁-C₅-Alkoxycarbonyl, wie Methoxycarbonyl, Ethoxycarbonyl, Propyloxycarbonyl, Isopropyloxycarbonyl, Butyloxycarbonyl, Isobutyloxycarbonyl, Sekundärbutyloxycarbonyl, Tertiärbutyloxycarbonyl, Pentyloxycarbonyl oder eine Hexyloxycarbonyl- oder Heptyloxycarbonylgruppe.

Niederalkoxycarbonylaminoniederalkoxy ist beispielsweise C₁-C₇-Alkoxycarbonylamino-C₂-C₇-alkoxy, vorzugsweise C₂-C₅-Alkoxycarbonylamino-C₂-C₇-alkoxy, wie Methoxycarbonylamino-C₂-C₇-alkoxy, Ethoxycarbonylamino-C₂-C₇-alkoxy, Propyloxycarbonylamino-C₂-C₇-alkoxy, Isopropyloxycarbonylamino-C₂-C₇-alkoxy, Butyloxycarbonylamino-C₂-C₇-alkoxy, Isobutyloxycarbonylamino-C₂-C₇-alkoxy, Sekundärbutyloxycarnonylamino-C₂-C₇-alkoxy oder Tertiärbutyloxycarbonylamino-C₂-C₇-alkoxy, worin C₂-C₇-Alkoxy beispielsweise Methoxy, Ethoxy, Propyloxy, Butyloxy, Pentyloxy oder Hexyloxy bedeutet.

Niederalkoxycarbonylaminoniederalkyl ist beispielsweise C₁-C₇-Alkoxycarbonylamino-C₂-C₇-alkyl, vorzugsweise C₂-C₅-Alkoxycarbonylamino-C₂-C₇-alkyl, wie Methoxycarbonylamino-C₂-C₇-alkyl, Ethoxycarbonylamino-C₂-C₇-alkyl, Propyloxycarbonylamino-C₂-C₇-alkyl, Isopropyloxycarbonylamino-C₂-C₇-alkyl, Butyloxycarbonylamino-C₂-C₇-alkyl, lsobutyloxycarbonylamino-C₂-C₇-alkyl, Sekundärbutyloxycarbonylamino-C₂-C₇-alkyl oder Tertiärbutyloxycarbonylamino-C₂-C₇-alkyl, worin C₂-C₇-Alkyl beispielsweise Methyl, Ethyl, Propyl, Butyl, Pentyl oder Hexyl bedeutet.

Niederalkoxycarbonylniederalkoxy ist beispielsweise C₁-C₄-Alkoxycarbonyl-C₁-C₄-alkoxy, wie Methoxycarbonyl- oder Ethoxycarbonylmethoxy, 2-Methoxycarbonyl- oder 2-Ethoxycarbonylethoxy, 2- oder 3-Methoxycarbonyl- oder 2- oder 3-Ethoxycarbonylpropyloxy oder 4-Methoxycarbonyl- oder 4-Ethoxycarbonylbutyloxy, insbesondere Methoxycarbonyl- oder Ethoxycarbonylmethoxy oder 3-Methoxycarbonyl- oder 3-Ethoxycarbonylpropyloxy.

Niederalkoxycarbonylniederalkyl ist beispielsweise C₁-C₄-Alkoxycarbonyl-C₁-C₄-alkyl, wie Methoxycarbonyl- oder Ethoxycarbonylmethyl, 2-Methoxycarbonyl- oder 2-Ethoxycarbonylethyl, 3-Methoxycarbonyl- oder 3-Ethoxycarbonylpropylyl oder 4-Ethoxycarbonylbutyl.

Niederalkoxyiminoniederalkyl ist beispielsweise N-(C₁-C₄-Alkoxy)imino-C₂-C₄-alkyl, wie 3-Methoxyiminopropyl.

Niederalkoxyniederalkenyl ist beispielsweise C₁-C₄-Alkoxy-C₂-C₄-alkenyl, wie 4-Methoxybut-2-enyl.

Niederalkoxyniederalkenyloxy ist beispielsweise C₁-C₄-Alkoxy-C₂-C₄-alkenyloxy, wie 4-Methoxybut-2-enyloxy.

Niederalkoxyniederalkoxy ist beispielsweise C₁-C₄-Alkoxy-C₂-C₄-alkoxy, wie 2-Methoxy-, 2-Ethoxy- oder 2-Propyloxyethoxy, 3-Methoxy- oder 3-Ethoxypropyloxy oder 4-Methoxybutyloxy, insbesondere 3-Methoxypropyloxy oder 4-Methoxybutyloxy.

Niederalkoxyniederalkoxyniederalkyl ist beispielsweise C₁-C₄-Alkoxy-C₁₋C₄-alkoxy-C₁-C₄-alkyl, wie 2-Methoxy-, 2-Ethoxy- oder 2-Propyloxyethoxymethyl, 2-(2-Methoxy-, 2-Ethoxyoder 2-Propyloxyethoxy)ethyl 3-(3-Methoxy- oder 3-Ethoxypropyloxy)propyl oder 4-(2-Methoxybutyloxy)butyl, insbesondere 2-(3-Methoxypropyloxy)ethyl oder 2-(4-Methoxybutyloxy)ethyl.

Niederalkoxyniederalkyl beispielsweise C₁-C₄-Alkoxy-C₁-C₄-alkyl, wie Ethoxymethyl, Propyloxymethyl, Butyloxymethyl, 2-Methoxy-, 2-Ethoxy- oder 2-Propyloxyethyl, 3-Methoxy- oder 3-Ethoxypropyl oder 4-Methoxybutyl, insbesondere 3-Methoxypropyl oder 4-Methoxybutyl.

Niederalkyl kann geradkettig oder verzweigt und/oder überbrückt sein und ist beispielsweise entsprechendes C₁-C₇-Alkyl, wie Methyl, Ethyl, Propyl, Isopropyl, Butyl, Isobutyl, Sekundärbutyl, Tertiärbutyl, oder eine Pentyl-, Hexyl- oder Heptylgruppe. Niederalkyl R₂ bzw. R₃ ist insbesondere C₂-C₇-Alkyl, Niederalkyl R₅ bzw. R₇ ist insbesondere verzweigtes C₃-C₇-Alkyl und Niederalkyl R₈ bzw. R₃ ist beispielsweise geradkettiges, verzweigtes oder überbrücktes C₃-C₇-Alkyl.

Niederalkylamino ist beispielsweise C₁-C₄-Alkylamino, wie Methylamino, Ethylamino, Propylamino, Butylamino, Isobutylamino, Sekundärbutylamino oder Tertiärbutylamino.

Niederalkylaminoniederalkoxy ist beispielsweise C₁-C₄-Alkylamino-C₁-C₄-alkoxy, wie Propylaminomethoxy, 2-Methylamino-, 2-Ethylamino-, 2-Propylamino- oder 2-Butylaminoethoxy, 3-Ethylamino- oder 3-Propylaminopropyloxy oder 4-Methylaminobutoxy.

Niederalkylaminoniederalkyl ist beispielsweise C₁-C₄-Alkylamino-C₁-C₄-alkyl, wie Propylaminomethyl, 2-Methylamino-, 2-Ethylamino-, 2-Propylamino- oder 2-Butylaminoethyl, 3-Ethylamino- oder 3-Propylaminopropyl oder 4-Methylaminobutyl.

Niederalkylcarbamoylniederalkoxy ist beispielsweise N-C₁-C₇-Alkylcarbamoyl-C₁-C₄-alkoxy, wie Methyl- oder Dimethylcarbamoyl-C₁-C₄-alkoxy, z.B. Methylcarbamoylmethoxy, 2-Methylcarbamoylethoxy oder 3-Methylcarbamoylpropyloxy.

Niederalkylcarbamoylniederalkoxy ist beispielsweise N-C₁-C₇-Alkylcarbamoyl-C₁-C₄-alkyl, wie Methyl- oder Dimethylcarbamoyl-C₁-C₄-alkyl, z.B. Methylcarbamoylmethyl, 2-Methylcarbamoylethyl oder 3-Methylcarbamoylpropyl.

Niederalkylen, dessen freie Valenzen von benachbarten oder zueinander 1,3-, 1,4- oder 1,5-ständigen C-Atomen ausgehen ist beispielsweise 1,3-Propylen, 1,2-Ethylen oder 1,3- 2,3- oder 1,4-Butylen oder 1,3-, 2,3-, 2,4- oder 1,5-Butylen.

Niederalkylthio(hydroxy)niederalkoxy ist beispielsweise N-C₁-C₄-Alkylthio-C₁-C₄-(hydroxy)Alkoxy, wie 2-Hydroxy-3-methylthiopropyloxy.

Niederalkylthio ist beispielsweise C₁-C₇-Alkylthio, vorzugsweise C₁-C₅-Alkylthio, wie Methylthio, Ethylthio, Propylthio, Isopropylthio, Butylthio, Isobutylthio, Sekundärbutylthio, Tertiärbutylthio, Pentylthio oder eine Hexylthio- oder Heptylthiogruppe.

Niederalkylthioniederalkoxy ist beispielsweise N-C₁-C₄-Alkylthio-C₁-C₄-alkoxy, wie Methylthio-C₁-C₄-alkoxy, z.B. Methylthiomethoxy, 2-Methylthioethoxy oder 3-Methylthiopropyloxy.

Niederalkylthioniederalkoxy ist beispielsweise N-C₁-C₄-Alkylthio-C₁-C₄-alkoxy, wie Methylthio-C₁-C₄-alkoxy, z.B. Methylthiomethoxy, 2-Methylthioethoxy oder 3-Methylthiopropyloxy.

Niederalkylthioniederalkyl ist beispielsweise N-C₁-C₄-Alkylthio-C₁-C₄-alkyl, wie Methylthio-C₁-C₄-alkyl, z.B. Methylthiomethyl, 2-Methylthioethyl oder 3-Methylthiopropyl.

Oxoniederalkoxy ist beispielsweise Oxo-C₁-C₄-alkoxy, wie 3,3-Dimethyl-2-oxo-butyloxy.

Phenylniederalkoxycarbonyl ist beispielsweise Phenyl-C₁-C₄-alkoxycarbonyl, wie Benzyloxycarbonyl, 1- oder 2-Phenylethoxycarbonyl, 3-Phenylpropyloxycarbonyl oder 4-Phenylbutyloxycarbonyl .

Phenylniederalkoxycarbonyloxy ist beispielsweise Phenyl-C₁-C₄-alkoxycarbonyloxy, wie Benzyloxycarbonyloxy, 1- oder 2-Phenylethoxycarbonyloxy, 3-Phenylpropyloxycarbonyloxy oder 4-Phenylbutyloxycarbonyloxy.

Phenylniederalkoxyniederalkoxy ist beispielsweise Phenyl-C₁-C₄-alkoxy-C₂-C₄-alkoxy, wie 2-Benzyloxyethoxy, 1- oder 2-Phenylethoxyethoxy, 3-Benzyloxypropyloxy, 2-Phenylpropyloxyethoxy, 3-Phenylpropyloxypropyloxy oder 4-Benzyloxybutyloxy.

Phenylniederalkyl ist beispielsweise Phenyl-C₁-C₄-alkyl, wie Benzyl, 1- oder 2-Phenylethyl, 3-Phenylpropyl oder 4-Phenylbutyl.

Naphthylniederalkyl ist beispielsweise Naphthyl-C₁-C₄-alkyl, wie Benzyl, 1- oder 2-Naphthylethyl, 3-Naphthylpropyl oder 4-Naphthylbutyl.

Piperidinoniederalkoxy ist beispielsweise Piperidino-C₁-C₄-alkoxy, wie Piperidinomethoxy, 2-Piperidinoethoxy oder 3-Piperidinopropyloxy.

Piperidinoniederalkyl ist beispielsweise Piperidino-C₁-C₄-alkyl, wie Piperidinomethyl, 2-Piperidinoethyl oder 3-Piperidinopropyl. Polyhalogenniederalkansulfonylaminoniederalkoxy ist beispielsweise Trifluor-C₁-C₇-alkansulfonyl-C₁-C₄-alkoxy, wie Trifluormethansulfonylaminobutyloxy. Polyhalogenniederalkansulfonylaminoniederalkyl ist beispielsweise Trifluor-C₁-C₇-alkansulfonyl-C₁-C₄-alkyl, wie Trifluormethansulfonylaminobutyl.

Polyhalogenniederalkoxy ist beispielsweise Trihalogen-C₁-C₄-alkoxy, wie Trifluormethoxy oder 2,2,2-Trichlorethyl.

Polyhalogenniederalkyl ist beispielsweise Trihalogen-C₁-C₄-alkyl, wie Trifluormethoxy oder 2,2,2-Trichlorethoxy.

Pyridylniederalkoxyniederalkoxy ist beispielsweise Pyridyl-C₁-C₄-alkoxy-C₂-C₄-alkoxy, wie 2-Pyridylmethoxyethoxy, 1- oder 2-Pyridylethoxyethoxy, methoxypropyloxy, 2-Pyridylpropyloxyethoxy, 3-Pyridylpropyloxypropyloxy oder 4-Pyridylmethoxybutyloxy.

Pyridylniederalkoxy, welches auch partiell hydriert oder N-oxidiert sein kann, ist beispielsweise Pyridyl-C₁-C₄-alkoxy, Tertrahydropyridyl-C₁-C₄-alkoxy oder N-Oxidopyridyl-C₁-C₄-alkoxy, wie Pyridylmethoxy, Tetrahydropyridylmethoxy, N-Oxidopyridylmethoxy, 1- oder 2-Pyridylethoxy, 2-Pyridylpropyloxy, 3-Pyridylpropyloxy oder 4-Pyridylbutyloxy.

Pyridylniederalkyl ist beispielsweise Pyridyl-C₁-C₄-alkyl, wie Pyridylmethyl, 1- oder 2-Pyridylethyl, 3-Pyridylpropyl oder 4-Pyridylbutyl.

Pyrimidinylniederalkyl ist beispielsweise Pyrimidinyl-C₁-C₄-alkyl, wie Pyrimidinylmethyl, 1- oder 2-Pyrimidinylethyl, 3-Pyrimidyinlpropyl oder 4-Pyrimidinylbutyl.

Chinolinylniederalkyl ist beispielsweise Chinolinyl-C₁-C₄-alkyl, wie Chinolinylmethyl, 1- oder 2-Chinolinylethyl, 3-Chinolinylpropyl oder 4-Chinolinylbutyl.

Pyrimidinylthioniederalkoxy ist beispielsweise Pyrimidylthio-C₁-C₄-alkoxy, wie Pyrimidinylthiomethoxy, 1- oder 2-Pyrimidinylthioethoxy, 3-Pyrimidinylthiopropyloxy oder 4-Pyrimidinylthiobutyloxy.

Pyrimidinylthioniederalkyl ist beispielsweise Pyrimidyinlthio-C₁-C₄-alkyl, wie Pyrimidinylthiomethyl, 1- oder 2-Pyrimidinylthioethyl, 3-Pyrimidinylthiopropyl oder 4-Pyrimidinylthiobutyl .

Pyrrolidinoniederalkoxy ist beispielsweise Pyrrolidino-C₂-C₄-alkoxy, wie 2-Pyrrolidinoethoxy oder 3-Pyrrolidinopropyloxy.

Pyrrolidinoniederalkyl ist beispielsweise Pyrrolidino-C₁-C₄-alkyl, wie Pyrrolidinomethyl, 2-Pyrrolidinoethyl oder 3-Pyrrolidinopropyl.

S,S-Dioxothiomorpholinoniederalkoxy ist beispielsweise S,S-Dioxothiomorpholino-C₁-C₄-alkoxy, wie S,S-Dioxothiomorpholinomethoxy oder 2-(S,S-Dioxo)Thiomorpholinomethoxy.

S,S-Dioxothiomorpholinoniederalkyl ist beispielsweise S,S-Dioxothiomorpholino-C₁-C₄-alkyl, wie S,S-Dioxothiomorpholinomethyl oder 2-(S,S-Dioxo)Thiomorpholinomethyl.

S-Oxothiomorpholinoniederalkoxy ist beispielsweise S-Oxothiomorpholino-C₁-C₄-alkoxy, wie S-Oxothiomorpholinomethoxy oder 2-(S-Oxo)thiomorpholinomethoxy.

S-Oxothiomorpholinoniederalkyl ist beispielsweise S-Oxothiomorpholino-C₁-C₄-alkyl, wie S-Oxothiomorpholinomethyl oder 2-(S-Oxo)thiomorpholinoethyl.

Thiazolinylthioniederalkoxy ist beispielsweise Thiazolinylthio-C₁-C₄-alkoxy, wie Thiazolinylthiomethoxy, 1- oder 2-Thiazolinylthioethoxy, 3-Thiazolinylthiopropyloxy oder 4-Thiazolinylthiobutyloxy.

Thiazolinylthioniederalkyl ist beispielsweise Thiazolinylthio-C₁-C₄-alkyl, wie Thiazolinylthiomethyl, 1- oder 2-Thiazolinylthioethyl, 3-Thiazolinylthiopropyl oder 4-Thiazolinylthiobutyl.

Thiazolylniederalkoxy ist beispielsweise Thiazolyl-C₁-C₄-alkoxy, wie Thiazolylmethoxy, 1-oder 2-Thiazolylethoxy, 3-Thiazolylpropyloxy oder 4-Thiazolylbutyloxy.

Thienylniederalkoxyniederalkoxy ist beispielsweise Thienyl-C₁-C₄-alkoxy-C₂-C₄-alkoxy, wie 2- Thienylmethoxyethoxy, 1- oder 2-Thienylethoxyethoxy, 3-Thienylmethoxypropyloxy, 2-Thienylpropyloxyethoxy, 3-Thienylpropyloxypropyloxy oder 4-Thienylmethoxybutyloxy.

Thienylniederalkyl ist beispielsweise Thienyl-C₁-C₄-alkyl, wie Thienylmethyl, 1- oder 2-Thienylethyl, 3-Thienylpropyl oder 4-Thienylbutyl.

Trihalogenniederalkanoyloxy ist beispielsweise Trifluoracetoxy.

Triniederalkylsilyloxy ist beispielsweise Trimethylsilyloxy, Dimethyl(butyl)silyloxy oder Tributylsilyloxy.

Salze von Verbindungen mit salzbildenden Gruppen sind insbesondere Säureadditionssalze, Salze mit Basen oder bei Vorliegen mehrerer salzbildender Gruppen gegebenenfalls auch Mischsalze oder innere Salze.

Solche Salze werden beispielsweise von Verbindungen der Formel I mit einer sauren Gruppe, z.B. einer Carboxygruppe, gebildet und sind beispielsweise deren Salze mit geeigneten Basen, wie nicht-toxische, von Metallen der Gruppe Ia, Ib, IIa und IIb des Periodensystems der Elemente abgeleitete Metallsalze, z.B. Alkalimetall-, insbesondere Lithium-, Natrium- oder Kaliumsalze, Erdalkalimetallsalze, beispielsweise Magnesium- oder Calciumsalze, ferner Zinksalze oder Ammoniumsalze, auch solche Salze, welche mit organischen Aminen, wie gegebenenfalls durch Hydroxy substituierten Mono-, Di- oder Trialkylaminen, insbesondere Mono-, Di- oder Triniederalkylaminen, oder mit quaternären Ammoniumbasen gebildet werden, z.B. Methyl-, Ethyl-, Diethyl- oder Triethylamin, Mono-, Bis- oder Tris-(2-hydroxyniederalkyl)-aminen, wie Ethanol-, Diethanol- oder Triethanolamin, Tris(hydroxymethyl)-methylamin oder 2-Hydroxytert-butylamin, N,N-Diniederalkyl-N-(hydroxyniederalkyl)-amin, wie N,N-Dimethyl-N-(2-hydroxyethyl)-amin, oder N-Methyl-D-glucamin, oder quaternären Ammoniumhydroxiden, wie Tetrabutylammoniumhydroxid.

Die Verbindungen der Formel I mit einer basischen Gruppe, z.B. einer Aminogruppe, können Säureadditionssalze bilden, z.B. mit geeigneten anorganischen Säuren, z.B. Halogenwasserstoffsäure, wie Salzsäure, Bromwasserstoffsäure, Schwefelsäure unter Ersatz eines oder beider Protonen, Phosphorsäure unter Ersatz eines oder mehrerer Protonen, z.B. ortho-Phosphorsäure oder Metaphosphosphorsäure, oder Pyrophosporsäure unter Ersatz eines oder mehrerer Protonen, oder mit organischen Carbon-, Sulfon-, Sulfo- oder Phosphonsäuren oder N-substituierter Sulfaminsäuren, z.B. Essigsäure, Propionsäure, Glykolsäure, Bemsteinsäure, Maleinsäure, Hydroxymaleinsäure, Methylmaleinsäure, Fumarsäure, Äpfelsäure, Weinsäure, Gluconsäure, Glucarsäure, Glucuronsäure, Zitronensäure, Benzoesäure, Zimtsäure, Mandelsäure, Salicylsäure, 4-Aminosalicylsäure, 2-Phenoxybenzoesäure, 2-Acetoxybenzoesäure, Embonsäure, Nicotinsäure, Isonicotinsäure, ferner Aminosäuren, wie z.B. den weiter vorn genannten a-Aminosäuren, sowie Methansulfonsäure, Ethansulfonsäure, 2-Hydroxyethansulfonsäure, Ethan-1,2-disulfonsäure, Benzolsulfonsäure, 4-Methylbenzolsulfonsäure, Naphthalin-2-sulfonsäure, 2- oder 3- Phosphoglycerat, Glucose-6-Phosphat, N-Cyclohexylsulfaminsäure (unter Bildung der Cyclamate) oder mit anderen sauren organischen Verbindungen, wie Ascorbinsäure. Verbindungen der Fommel I mit sauren und basischen Gruppen können auch innere Salze bilden.

Zur Isolierung und Reinigung können auch pharmazeutisch ungeeignete Salze Verwendung finden.

Die Verbindungen der Formel I sind wertvolle Zwischenprodukte für die Herstellung von Arzneimittelwirkstoffen, beispielsweise von Verbindungen der Formel II 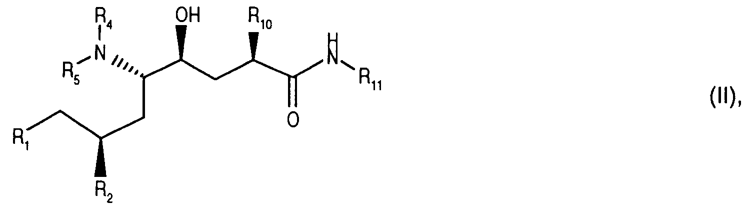
worin
R₁ einen aliphatischen, cycloaliphatischen, cycloaliphatisch-aliphatischen, aromatischen oder heteroaromatischen Rest, eine aliphatisch, araliphatisch oder heteroarylaliphatisch veretherte oder durch eine Hydroxyschutzgruppe geschützte Hydroxygruppe oder eine aliphatisch veretherte Mercaptogruppe und
R₂ einen aliphatischen, cycloaliphatischen, cycloaliphatisch-aliphatischen, araliphatischen oder heteroarylaliphatischen Rest bedeutet oder
R₁ und R₂ gemeinsam einen zweiwertigen aliphatischen Rest darstellen,
R₄ Wasserstoff, Niederalkyl oder Niederalkanoyl steht,
R₅ Wasserstoff oder, sofern R₄ Niederalkyl ist, auch Niederalkyl bedeuten kann, R₁₀ einen aliphatischen, cycloaliphatischen, cycloaliphatisch-aliphatischen oder araliphatischen Rest bedeutet und
R₁₁ einen aliphatisch, cycloaliphatischen oder heteroaromatisch-aliphatischen Rest bedeutet,
und ihrer Salze, die beispielsweise als Antihypertensiva eingesetzt werden können.

Die Verbindungen der Formel I sind insbesondere wertvolle Zwischenprodukte für δ-Amino-γ-hydroxy-ω-aryl-alkansäureamide der Formel II, worin
R₁ einen aliphatischen, cycloaliphatischen, cycloaliphatisch-aliphatischen, aromatischen oder heteroaromatischen Rest, eine aliphatisch, araliphatisch oder heteroarylaliphatisch veretherte oder durch eine Hydroxyschutzgruppe geschützte Hydroxygruppe oder eine aliphatisch veretherte Mercaptogruppe und
R₂ einen aliphatischen, cycloaliphatischen, cycloaliphatisch-aliphatischen, araliphatischen oder heteroarylaliphatischen Rest bedeutet oder
R₁ und R₂ gemeinsam einen zweiwertigen aliphatischen Rest darstellen, R₄ Wasserstoff, einen aliphatischen oder araliphatischen Rest oder eine Aminoschutzgruppe bedeutet und
R₅ Wasserstoff oder einen aliphatischen Rest darstellt,
R₁₀ Niederalkyl, Niederalkenyl, Cycloalkyl oder Arylniederalkyl bedeutet und
R₁₁ Niederalkyl, Cycloalkyl, gegebenenfalls aliphatisch verestertes oder verethertes Hydroxyniederalkyl, gegebenenfalls N-niederalkanoyliertes, N-mono- oder N,N-diniederalkyliertes oder durch Niederalkylen, Hydroxy-, Niederalkoxy- oder Niederalkanoyloxyniederalkylen, gegebenenfalls N'-niederalkanoyliertes bzw. N'-niederalkyliertes Azaniederalkylen, Oxaniederalkylen oder gegebenenfalls S-oxidiertes Thianiederalkylen N,N-disubstituiertes Aminoniederalkyl, gegebenenfalls verestertes oder amidiertes Carboxyniederalkyl, gegebenenfalls verestertes oder amidiertes Dicarboxyniederalkyl, gegebenenfalls verestertes oder amidiertes Carboxy(hydroxy)niederalkyl, gegebenenfalls verestertes oder amidiertes Carboxycyloalkylniederalkyl, Cyanoniederalkyl, Niederalkansulfonylniederalkyl, gegebenenfalls N-mono- oder N,N-diniederalkyliertes Thiocarbamoylniederalkyl, gegebenenfalls N-mono- oder N,N-diniederalkyliertes Sulfamoylniederalkyl oder einen über ein C-Atom gebundenen und gegebenenfalls hydrierten und/oder oxosubstituerten Heteroarylrest oder einen durch einen über ein C-Atom gebundenen und gegebenenfalls hydrierten und/oder oxosubstituerten Heteroarylrest substituiertes Niederalkyl darstellt,
und ihre Salze, die als Reninhemmer wirken und deshalb als Antihypertensiva eingesetzt werden können.

Die Verbindung der Formel I sind vorzugsweise geeignet für die Herstellung von Verbindung der Formel IIa 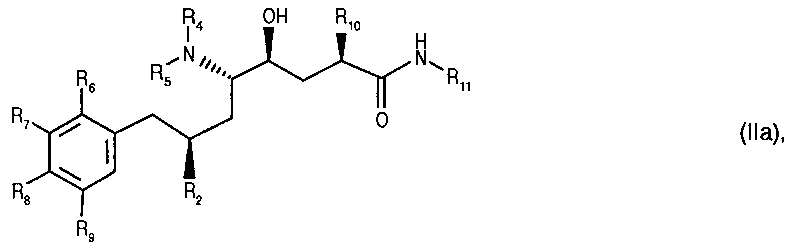
worin
R₂ Niederalkyl, 3- bis 8-gliedriges Cycloalkyl, 3- bis 8-gliedriges Cycloalkylniederalkyl, unsubstituiertes oder im Phenyl- , Naphthyl- bzw. Heteroarylteil durch Niederalkyl, Niederalkoxy, Halogen und/oder Trifluormethyl substituierten Phenylniederalkyl-, Naphthylniederalkyl-, Furylniederalkyl-, Thienylniederalkyl-, gegebenenfalls partiell hydriertes oder N-oxidierten Pyridylniederalkyl-, Pyrimidinylniederalkyl- oder Chinolinylniederalkylrest bedeutet,
R₄ Wasserstoff, Niederalkyl oder Niederalkanoyl, insbesondere Wasserstoff, ist,
R₅ Wasserstoff oder, sofern R₄ für Niederalkyl steht, Wasserstoff oder Niederalkyl ist und
R₆, R₇, R₈ und R₉ die unter der Formel la angegebenen Bedeutungen haben,
R₁₀ Niederalkyl, Niederalkenyl, Cycloalkyl oder Arylniederalkyl bedeutet und
R₁₁ Niederalkyl, Cycloalkyl, gegebenenfalls aliphatisch verestertes oder verethertes Hydroxyniederalkyl, gegebenenfalls N-niederalkanoyliertes, N-mono- oder N,N-diniederalkyliertes oder durch Niederalkylen, Hydroxy-, Niederalkoxy- oder Niederalkanoyloxyniederalkylen, gegebenenfalls N'-niederalkanoyliertes bzw. N'-niederalkyliertes Azaniederalkylen, Oxaniederalkylen oder gegebenenfalls S-oxidiertes Thianiederalkylen N,N-disubstituiertes Aminoniederalkyl, gegebenenfalls verestertes oder amidiertes Carboxyniederalkyl, gegebenenfalls verestertes oder amidiertes Dicarboxyniederalkyl, gegebenenfalls verestertes oder amidiertes Carboxy(hydroxy)niederalkyl, gegebenenfalls verestertes oder amidiertes Carboxycyloalkylniederalkyl, Cyanoniederalkyl, Niederalkansulfonylniederalkyl, gegebenenfalls N-mono- oder N,N-diniederalkyliertes Thiocarbamoylniederalkyl gegebenenfalls N-mono- oder N,N-diniederalkyliertes Sulfamoylniederalkyl oder einen über ein C-Atom gebundenen und gegebenenfalls hydrierten und/oder oxosubstituerten Heteroarylrest oder einen durch einen über ein C-Atom gebundenen und gegebenenfalls hydrierten und/oder oxosubstituerten Heteroarylrest substituiertes Niederalkyl darstellt,
wobei vorzugsweise
R₂ für verzweigtes C₁-C₄-Alkyl, wie Isopropyl, 3- bis 5-gliedriges oder Cycloalkyl-C₁-C₄-alkyl, wie Cyclopropylmethyl, steht,
R₄ Wasserstoff, C₁-C₄-Alkyl, wie Methyl, oder C₁-C₄-Alkanoyl, wie Acetyl, insbesondere Wasserstoff, ist,
R₅ Wasserstoff oder, sofern R₄ für C₁-C₄-Alkyl steht, Wasserstoff oder C₁-C₄-Alkyl, wie Methyl, ist,
R₆ Wasserstoff bedeutet,
R₇ C₁-C₄-Alkyl, wie Methyl oder Tertiärbutyl, C₁-C₄-Alkoxy-C₁-C₄-alkyl, wie 3-Methoxypropyl, C₁-C₄-Alkoxy-C₁-C₄-alkoxy, wie 3-Methoxypropyloxy, C₁-C₄-Alkoxy-C₁-C₄-alkoxy-C₁-C₄-alkyl, wie 3-Methoxybutyl, Phenyl-C₁-C₄-alkoxy, wie Benzyloxy, Pyridyl-C₁-C₄-alkoxy, wie Pyridylmethoxy, C₁-C₄-Alkylthio-C₁-C₄-alkoxy, wie 3-Methylthiopropyloxy, C₁-C₄-Alkansulfonyl-C₁-C₄-alkoxy, wie 3-Methansulfonylpropyloxy, C₁-C₄-Alkanoyloxy-C₁-C₄-alkoxy, wie 3-Acetoxypropyloxy, C₁-C₄-Alkoxycarbonyl-C₁-C₄-alkoxy, wie Methoxy- oder Ethoxycarbonylmethoxy, Carbamoyl-C₁-C₄-alkoxy, wie Carbamoylmethoxy, oder Di-C₁-C₄-alkylcarbamoyl-C₁-C₄-alkoxy, wie Dimethylcarbamoylmethoxy, bedeutet,
R₈ Wasserstoff, C₁-C₄-Alkyl, wie Methyl oder Tertiärbutyl, Hydroxy oder C₁-C₄-Alkoxy, wie Methoxy, bedeutet oder gemeinsam mit R₉ C₁-C₄₋Alkylendioxy, wie Methylendioxy oder Ethylendioxy, darstellt.
R₉ Wasserstoff bedeutet oder gemeinsam mit R₈ C₁-C₄-Alkylendioxy, wie Methylendioxy oder Ethylendioxy, darstellt,
R₁₀ verzweigtes C₁-C₄-Alkyl, wie Isopropyl, bedeutet und
R₁₁ Carbamoyl-C₁-C₄-alkyl, wie 2- oder 3-Carbamoylpropyl, 2-(3-Carbamoyl)propyl oder 1-(2-Carbamoyl-2-methyl)propyl, N-C₁-C₄-Alkylcarbamoyl-C₁-C₄-alkyl, wie 3-(N-Methyl-carbamoyl)propyl, 2-[1-(N-Methylcarbamoyl)]propyl, 1-[2-(N-Methylcarbamoyl)]propyl, vor allem 1-[2(R)-(N-Methylcarbamoyl)]propyl, N,N-Di-C₁-C₄-alkylcamamoyl-C₁-C₄-alkyl, wie N,N-Dimethylcarbamoylmethyl oder 2-(N,N-Dimethylcarbamoyl)ethyl, 3-(N,N-Dimethylcarbamoyl) propyl, Morpholino-C₁-C₄-alkyl, wie 2-Morpholinoethyl, 3-Morpholinopropyl oder 1-(2-Morpholino-2-methyl)propyl, Thiomorpholino-C₁-C₄-alkyl, wie 2-Thiomorpholinoethyl, 4-(1-C₁-C₄-Alkanoylpiperidyl)- C₁-C₄-alkyl, wie 2-[4-(1 -Acetyl)piperidinyl]ethyl, 2-Oxopyrrolidinyl-C₁-C₄-alkyl, wie 2-Oxopyrrolidin-5(S)-ylmethyl oder 2-Oxopyrrolidin-5(R)ylmethyl, bedeutet, und ihre Salze.

Die Verbindungen der Formel I sind in allererster Linie geeignet für die Herstellung von Verbindungen der Formel IIa, worin
R₂ verzweigtes C₁-C₄-Alkyl, wie Isopropyl, bedeutet,
R₄, R₅, R₆ und R₉ Wasserstoff bedeuten,
R₇ für C₁-C₄-Alkoxy-C₁-C₄-alkoxy, wie 3-Methoxypropyloxy, oder C₁-C₄-Alkoxy-C₁-C₄-alkyl, wie 3-Methoxybutyl, steht,
R₈ C₁-C₄-Alkyl, wie Isopropyl oder Tertiärbutyl, oder C₁-C₄-Alkoxy, wie Methoxy, darstellt, R₁₀ verzweigtes C₁-C₄-Alkyl, wie Isopropyl, bedeutet und R₁₁ Carbamoyl-C₁-C₄-alkyl, wie 2- oder 3-Carbamoylpropyl, 2-(3-Carbamoyl)propyl oder 1-(2-Carbamoyl-2-methyl)propyl, N-C₁-C₄-Alkylcarbamoyl-C₁-C₄-alkyl, wie 3-(N-Methylcarbamoyl) propyl, 2-[1-(N-Methylcarbamoyl)]propyl, 1-[2-(N-Methylcarbamoyl)]propyl, vor allem 1-[2(R)-(N-Methylcarbamoyl)]propyl, N,N-Di-C₁-C₄-alkylcarbamoyl-C₁-C₄-alkyl, wie N,N-Dimethylcarbamoylmethyl oder 2-(N,N-Dimethylcarbamoyl)ethyl, 3-(N,N-Dimethylcarbamoyl)propyl, Morpholino-C₁-C₄-alkyl, wie 2-Morpholinoethyl, 3-Morpholinopropyl oder 1-(2-Morpholino-2-methyl)propyl, Thiomorpholino-C₁-C₄-alkyl, wie 2-Thiomorpholinoethyl, 4-(1-C₁-C₄-Alkanoylpiperidyl)- C₁-C₄-alkyl, wie 2-[4-(1-Acetyl)piperidinyl]ethyl, 2-Oxopyrrolidinyl-C₁-C₄-alkyl, wie 2-Oxopyrrolidin-5(S)-ylmethyl oder 2-Oxopyrrolidin-5(R)-ylmethyl, bedeutet, und ihrer Salze.

Die Überführung von Zwischenprodukten der Formel I in Reninhemmer der Formel II erfolgt in üblicher Weise, beispielsweise indem man in einer Verbindung der Formel I, worin R₁ für Formyl, gegebenenfalls verestertes Carboxy oder Hydroxymethyl steht, R₄ eine Aminoschutzgruppe und R₅ Wasserstoff bedeutet, die gegebenenfalls vorhandene freie oder veresterte Carboxygruppe in üblicher Weise, z.B. mittels Dibutylaluminiumhydrid in Toluol, zu Formyl reduziert, bzw. eine gegebenenfalls vorhandene Hydroxymethylgruppe in üblicher Weise, z.B. mittels Pyridin/Schwefeltrioxid in Dimethylsulfoxid/Dichlormethan, zu Formyl oxidiert, den entsprechenden Aldehyd der Formel 1, worin R₃ Formyl bedeutet, in einem etherartigen Lösungsmittel, wie Tetrahydrofuran, erforderlichenfalls in Gegenwart eines Aktivators, wie 1,2-Dibrombutan, mit Magnesium und einer Verbindung der Formel 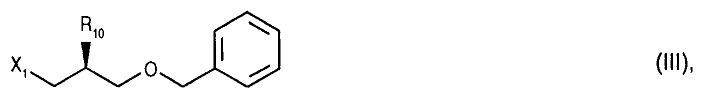
worin X₁ Halogen, wie Brom, bedeutet, umsetzt, die erhaltene Verbindung der Formel IV 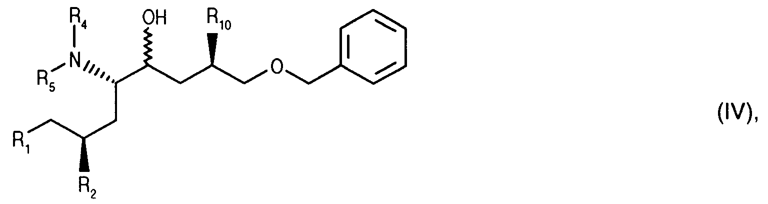
an der Amino- und Hydroxygruppe durch eine zweiwertige Schutzgruppe X₂, wie Isopropyliden, intermediär schützt, indem man beispielsweise unter sauren Bedingungen, wie in Gegenwart von p-Toluolsulfonsäure-Monohydrat, mit einem Acetonketal, wie einem Acetondiniederalkylketal, z.B. mit 2,2-Dimethoxypropan, umsetzt, das erhaltene Diatereomerengemisch in üblicher Weise, beispielsweise durch Flash-Säulenchromatographie an Kieselgel unter Verwendung von Essigsäureethylester/Hexan (1:2) als Laufmittel in die Komponenten auftrennt und aus dem Diastereomeren der Formel V 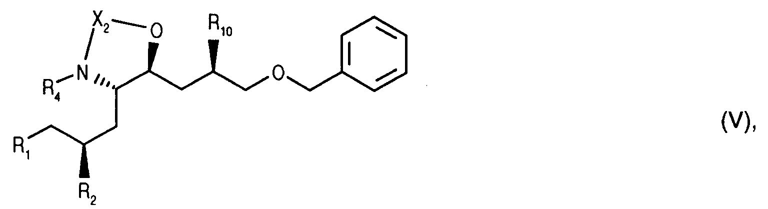
die Benzylgruppe in üblicher Weise, beispielsweise durch katalytische Hydrierung in Gegenwart von Palladium auf Kohle, abspaltet, die so freigesetzte terminale Hydroxymethylgruppe in üblicher Weise, beispielsweise durch Umsetzung mit einem Perruthenat, z.B. mit Tetrapropylammoniumperruthenat in Gegenwart von N-Methylmorphoin-N-oxid, zu Formyl oxidiert, die gebildete Formylgruppe in üblicher Weise, beispielsweise mittels eines Permanganates, wie Kaliumpermanganat, zu Carboxy oxidiert, die erhaltene Säure der Formel VI 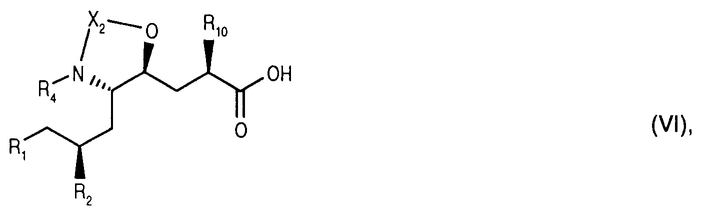
in üblicher Weise, beispielsweise in Gegenwart eines Cyanphosphonsäureesters, wie Cyanphosphonsäurediethylester, und eines tertiären organischen Amins, wie Triethylamin, vorzugsweise in N,N-Dimethylformamid, mit einem Amin der Formel VII

H₂N-R₁₁ (VII)

kondensiert und aus der erhaltenen Verbindung der Formel VIII 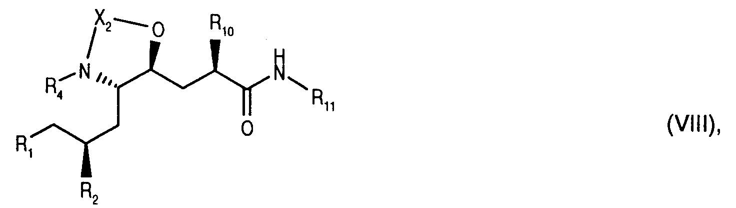
die Schutzgruppen in üblicher Weise, beispielsweise durch Säurebehandlung, z.B. mit Chlorwasserstoffsäure in einem cyclischen Ether, wie Dioxan, abspaltet und gewünschtenfalls eine verfahrensgemäß erhältliche Verbindung in eine andere Verbindung der Formel II umwandelt, gegebenenfalls erhältliche Isomerengemische auftrennt und/oder eine verfahrensgemäß erhältliche Verbindung der Formel II mit mindestens einer salzbildenden Gruppe in ein Salz oder ein verfahrensgemäß erhältliches Salz in die freie Verbindung oder in ein anderes Salz überführt.

Die Erfindung betrifft in erster Linie Verbindungen der Formel 1, worin
R₁ Niederalkyl, Niederalkenyl, 3- bis 8-gliedriges Cycloalkyl, 3- bis 8-gliedriges Cycloalkylniederalkyl, einen unsubstituierten oder durch Aminoniederalkoxy, Aminoniederalkyl, Arylniederalkoxy, Carbamoylniederalkoxy, Carbamoylniederalkyl, Carboxyniederalkoxy, Carboxyniederalkyl, Cyanoniederalkoxy, Cyanoniederalkyl, 3- bis 8-gliedriges Cycloalkoxy, 3- bis 8-gliedriges Cycloalkoxyniederalkoxy, 3- bis 8-gliedriges Cycloalkoxyniederalkyl, 3- bis 8-gliedriges Cycloalkyl, Diniederalkylamino, Diniederalkylaminoniederalkoxy, Diniederalkylaminoniederalkyl, Pyridylniederalkoxy, Tetrahydropyridylniederalkoxy, N-Oxidopyridylniederalkoxy, Thiazolylniederalkoxy, Morpholinoniederalkoxy, Pyridylthioniederalkoxy, N-Oxidopyridylthioniederalkoxy, Pyridylthioniederalkyl, N-Oxidopyridylthioniederalkyl, Halogen(hydroxy)niederalkoxy, Halogen, Hydroxy, Hydroxyniederalkoxy, Hydroxyniederalkyl, Imidazolylthioniederalkoxy, Imidazolylthioniederalkyl, Morpholinoniederalkoxy, Morpholinoniederalkyl, N-Mono- oder N,N-Diniederalkylcarbamoylniederalkoxy, N-Mono- oder N,N-Diniederalkylcarbamoylniederalkyl, gegebenenfalls N-oxidierte Pyridylniederalkyl, N'-Niederalkylpiperazino- bzw. N'-Niederalkanoylpiperazinoniederalkoxy, N'-Niederalkylpiperazino- bzw. N'-Niederalkanoylpiperazinoniederalkyl, Naphthyl, Naphthylniederalkoxy, Niederalkanoylaminoniederalkoxy, Niederalkanoylaminoniederalkyl, Niederalkanoylniederalkoxy, Niederalkanoyloxyniederalkyl, Niederalkansulfonyl(hydroxy)niederalkoxy, Niederalkansulfonylaminoniederalkoxy, Niederalkansulfonylaminoniederalkyl, Niederalkansulfonylniederalkoxy, Niederalkansulfonylniederalkyl, Niederalkenyloxy, Niederalkenyloxyniederalkoxy, Niederalkenyloxyniederalkyl, Niederalkoxy, Niederalkoxycarbonylaminoniederalkoxy, Niederalkoxycarbonylaminoniederalkyl, Niederalkoxycarbonylniederalkoxy, Niederalkoxycarbonylniederalkyl, Niederalkoxyiminoniederalkyl, Niederalkoxyniederalkenyl, Niederalkoxyniederalkenyloxy, Niederalkoxyniederalkoxy, Niederalkoxyniederalkoxyniederalkyl, Niederalkoxyniederalkyl, Niederalkyl, Niederalkylamino, Niederalkylaminoniederalkoxy, Niederalkylaminoniederalkyl, Niederalkylthio(hydroxy)niederalkoxy, Niederalkylthioniederalkoxy, Niederalkylthioniederalkoxy, Niederalkylthioniederalkyl, Oxoniederalkoxy, Piperazinoniederalkoxy, Piperazinoniederalkyl, Piperidinoniederalkoxy, Piperidinoniederalkyl, Polyhalogenniederalkansulfonylaminoniederalkoxy, Polyhalogenniederalkansulfonylaminoniederalkyl, Polyhalogenniederalkoxy, Polyhalogenniederalkyl, Pyrimidinylthioniederalkoxy, Pyrimidinylthioniederalkoxy, Pyrimidinylthioniederalkyl, Pyrrolidinoniederalkoxy, Pyrrolidinoniederalkyl, S,S-Dioxothiomorpholinoniederalkoxy, S,S-Dioxothiomorpholinoniederalkyl, S-Oxothiomorpholinoniederalkyoxy, S-Oxothiomorpholinoniederalkyl, Thiazolylthioniederalkoxy, Thiazolinylthioniederalkoxy, Thiazolylthioniederalkyl, Thiazolinylthioniederalkyl und/oder Thiomorpholino mono-, di- oder trisubstituierten Phenyl- oder Naphthyl- oder durch einen ankondensierten Benzo- oder Cyclohexenoring disubstituierten Phenylrest, einen unsubstituierten oder durch Niederalkyl, Niederalkoxy, Halogen und/oder Trifluormethyl substituierten Furyl-, Thienyl-, Pyridyl-, Pyrimidinyl-, Indolyl- oder Chinolinylrest, Niederalkoxy, Niederalkenyloxy, unsubstituierte oder im Phenylteil durch Niederalkyl, Niederalkoxy, Halogen und/oder Trifluormethyl substituiertes Phenylniederalkoxy, unsubstituiertes oder im Ring durch Niederalkyl, Niederalkoxy, Halogen und/oder Trifluormethyl substituierte Furylniederalkoxy-, Thienylniederalkoxy-, Pyridylniederalkoxy- oder Chinolinylniederalkoxygruppen, Niederalkanoyloxy, Trihalogenniederalkanoyloxy, eine unsubstituierte oder durch Niederalkyl, Niederalkoxy, Halogen, Trifluormethyl und/oder Nitro substituierte Benzoyloxy- oder Phenylniederalkoxycarbonyloxygruppe, Triniederalkylsilyloxy, Benzyl(diniederalkyl)silyloxy oder Niederalkylthio bedeutet und
R₂ Niederalkyl, 3- bis 8-gliedriges Cycloalkyl, 3- bis 8-gliedriges Cycloalkylniederalkyl, unsubstituiertes oder im Phenyl- Naphthyl- bzw. Heteroarylteil durch Niederalkyl, Niederalkoxy, Halogen und/oder Trifluormethyl substituierten Phenylniederalkyl-, Naphthylniederalkyl-, Furylniederalkyl-, Thienylniederalkyl-, gegebenenfalls partiell hydriertes oder N-oxidierten Pyridylniederalkyl-, Pyrimidinylniederalkyl- oder Chinolinylniederalkylrest bedeutet oder
R₁ und R₂ gemeinsam Niederalkylen, dessen freie Valenzen von benachbarten oder zueinander 1,3-, 1,4- oder 1,5-ständigen C-Atomen ausgehen, darstellen,
R₃ für Carboxy, Niederalkoxycarbonyl, Niederalkenyloxycarbonyl oder einen unsubstituierten oder durch Niederalkyl, Niederalkoxy, Halogen und/oder Trifluormethyl substituierten Phenylniederalkoxycarbonyl- oder Phenyloxycarbonylrest steht,
R₄ Wasserstoff, Niederalkyl, einen unsubstituierten oder durch Niederalkyl, Niederalkoxy, Halogen und/oder Trifluormethyl substituierten Phenyl- oder Naphthylniederalkylrest, Niederalkanoyl, Trihalogenniederalkanoyl, eine unsubstituierte oder durch Niederalkyl, Niederalkoxy, Halogen, Trifluormethyl und/oder Nitro substituierte Benzoyl- oder Phenylniederalkoxycarbonylgruppe, Triniederalkylsilyl oder Benzyl(diniederalkyl)silyl bedeutet und
R₅ Wasserstoff oder Niederalkyl darstellt,
und ihrer Salze.

Die Erfindung betrifft vor allem Verbindungen der Formel Ib 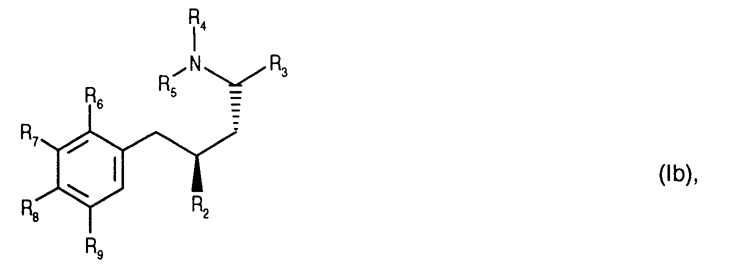
worin
R₂ Niederalkyl, 3- bis 8-gliedriges Cycloalkyl, 3- bis 8-gliedriges Cycloalkylniederalkyl, unsubstituiertes oder im Phenyl-, Naphthyl- bzw. Heteroarylteil durch Niederalkyl, Niederalkoxy, Halogen und/oder Trifluormethyl substituierten Phenylniederalkyl-, Naphthylniederalkyl-, Furylniederalkyl-, Thienylniederalkyl-, gegebenenfalls partiell hydriertes oder N-oxidierten Pyridylniederalkyl-, Pyrimidinylniederalkyl- oder Chinolinylniederalkylrest bedeutet,
R₃ für Carboxy, Niederalkoxycarbonyl, Niederalkenyloxycarbonyl oder einen unsubstituierten oder durch Niederalkyl, Niederalkoxy, Halogen und/oder Trifluormethyl substituierten Phenylniederalkoxycarbonyl- oder Phenyloxycarbonylrest steht,
R₄ Wasserstoff, Niederalkyl, einen unsubstituierten oder durch Niederalkyl, Niederalkoxy, Halogen und/oder Trifluormethyl substituierten Phenyl- oder Naphthylniederalkylrest, Niederalkanoyl, Trihalogenniederalkanoyl, eine unsubstituierte oder durch Niederalkyl, Niederalkoxy, Halogen, Trifluormethyl und/oder Nitro substituierte Benzoyl- oder Phenylniederalkoxycarbonylgruppe, Triniederalkylsilyl oder Benzyl (diniederalkyl)silyl bedeutet,
R₅ Wasserstoff oder Niederalkyl darstellt,
R₆ Wasserstoff, Hydroxy, Niederalkoxy, Cycloalkoxy, Niederalkoxyniederalkoxy, Carboxyniederalkoxy, Niederalkoxycarbonylniederalkoxy, Carbamoylniederalkoxy oder N-Mono- oder N,N- Diniederalkylcarbamoylniederalkoxy bedeutet,
R₇ Wasserstoff, Niederalkyl, Cycloalkyl, Niederalkoxyniederalkyl, Niederalkoxyniederalkoxyniederalkyl, Cycloalkoxyniederalkyl, Hydroxy, Niederalkanoyloxyniederalkyl, Hydroxyniederalkoxy, Halogen(hydroxy)niederalkoxy, Niederalkansulfonyl(hydroxy)niederalkoxy, Aminoniederalkyl, Niederalkylaminoniederalkyl, Diniederalkylaminoniederalkyl, Niederalkanoylaminoniederalkyl, Niederalkoxycarbonylaminoniederalkyl, Niederalkoxyiminoniederalkyl, Aminoniederalkoxy, Niederalkylaminoniederalkoxy, Diniederalkylaminoniederalkoxy, Niederalkanoylaminoniederalkoxy, Niederalkoxycarbonylaminoniederalkoxy, Oxoniederalkoxy, Niederalkoxy, Cycloalkoxy, Niederalkenyloxy, Cycloalkoxyniederalkoxy, Niederalkoxyniederalkoxy, Niederalkoxyniederalkenyl, Niederalkenyloxyniederalkoxy, Niederalkoxyniederalkenyloxy, Niederalkenyloxyniederalkyl, Niederalkanoylniederalkoxy, Niederalkylthioniederalkoxy, Niederalkansulfonylniederalkoxy, Niederalkylthio(hydroxy)niederalkoxy, Arylniederalkoxy, Thiazolylthioniederalkoxy bzw. Thiazolinylthioniederalkoxy, Imidazolylthioniederalkoxy, gegebenenfalls N-oxidierte Pyridylthioniederalkoxy, Pyrimidinylthioniederalkoxy, Cyanoniederalkoxy, Niederalkoxycarbonylniederalkoxy, Carbamoylniederalkoxy, N-Mono- oder N,N-Diniederalkylcarbamoylniederalkoxy, Carboxyniederalkyl, Niederalkoxycarbonylniederalkyl, Carbamoylniederalkyl oder N-Mono- oder N,N-Diniederalkylcarbamoylniederalkyl bedeutet,
R₈ Niederalkyl, Polyhalogenniederalkyl, Niederalkoxyniederalkyl, Cycloalkoxyniederalkyl, Hydroxyniederalkyl, Niederalkylthioniederalkyl, Niederalkansulfonylniederalkyl, gegebenenfalls partiell hydriertes oder N-oxidierte Pyridylniederalkyl, Thiazolylthioniederalkyl bzw. Thiazolinylthioniederalkyl, Imidazolylthioniederalkyl, gegebenenfalls N-oxidiertes Pyridylthioniederalkyl, Pyrimidinylthioniederalkyl, Aminoniederalkyl, Niederalkylaminoniederalkyl, Diniederalkylaminoniederalkyl, Niederalkanoylaminoniederalkyl, Niederalkansulfonylaminoniederalkyl, Polyhalogenniederalkansulfonylaminoniederalkyl, Pyrrolidinoniederalkyl, Piperidinoniederalkyl, Piperazino-, N'-Niederalkylpiperazino- bzw. N'-Niederalkanoylpiperazinoniederalkyl, Morpholinoniederalkyl, Thiomorpholino-, S-Oxothiomorpholino- oder S,S-Dioxothiomorpholinoniederalkyl, Cyanoniederalkyl, Carboxyniederalkyl, Niederalkoxycarbonylniederalkyl, Carbamoylniederalkyl, N-Mono- oder N,N-Diniederalkylcarbamoylniederalkyl, Cycloalkyl, unsubstituiertes oder durch Niederalkyl, Niederalkoxy, Hydroxy, Niederalkylamino, Diniederalkylamino, Halogen und/oder Trifluormethyl mono-, di- oder trisubstituiertes Phenyl oder Naphthyl, Hydroxy, Niederalkoxy, Cycloalkoxy, Niederalkoxyniederalkoxy, Cycloalkoxyniederalkoxy, Hydroxyniederalkoxy, unsubstituiertes oder durch Niederalkyl, Niederalkoxy, Hydroxy, Niederalkylamino, Diniederalkylamino, Halogen und/oder Trifluormethyl mono-, di- oder trisubstituiertes Phenylniederalkoxy oder Naphthylniederalkoxy, Niederalkoxy, Polyhalogenniederalkoxy, Niederalkylthioniederalkoxy, Niederalkansulfonylniederalkoxy, gegebenenfalls hydriertes Heteroarylniederalkoxy, gegebenenfalls partiell oder vollständig hydriertes Heteroarylthioniederalkoxy, wie Thiazolylthioniederalkoxy bzw. Thiazolinylthioniederalkoxy, Imidazolylthioniederalkoxy, gegebenenfalls N-oxidierte Pyridylthioniederalkoxy, Pyrimidinylthioniederalkoxy, Aminoniederalkoxy, Niederalkylaminoniederalkoxy, Diniederalkylaminoniederalkoxy, Niederalkanoylaminoniederalkoxy, Niederalkansulfonylaminoniederalkoxy, Polyhalogenniederalkansulfonylaminoniederalkoxy, Pyrrolidinoniederalkoxy, Piperidinoniederalkoxy, Piperazino-, N'-Niederalkylpiperazino- bzw. N'-Niederalkanoylpiperazinoniederalkoxy, Morpholinoniederalkoxy, Thiomorpholino-, S-Oxothiomorpholino- oder S,S-Dioxothiomorpholinoniederalkoxy, Cyanoniederalkoxy, Carboxyniederalkoxy, Niederalkoxy carbonylniederalkoxy, Carbamoylniederalkoxy oder N-Mono- oder N,N-Diniederalkylcarbamoylniederalkoxy bedeutet oder gemeinsam mit R₉ Niederalkylendioxy oder einen ankondensierten Benzo- oder Cyclohexenoring darstellt, R₉ gemeinsam mit R₈ Niederalkylendioxy oder einen ankondensierten Benzo- oder Cyclohexenoring darstellt oder für Wasserstoff, Niederalkyl, Hydroxy, Niederalkoxy oder Cycloalkoxy steht,
und ihrer Salze.

Die Erfindung betrifft insbesondere Verbindungen der Formel Ib, worin
R₂ für Niederalkyl , 3- bis 5-gliedriges Cycloalkylniederalkyl oder Cycloalkyl steht,
R₃ für Carboxy, Niederalkoxycarbonyl, Formyl oder Hydroxymethyl steht,
R₄ Niederalkoxycarbonyl oder unsubstituiertes oder durch Niederalkyl, Niederalkoxy, Nitro und/oder Halogen substituiertes α-Phenylniederalkoxycarbonyl bedeutet,
R₅ und R₆ Wasserstoff bedeuten,
R₇ Niederalkyl, Niederalkoxyniederalkyl, Niederalkoxyniederalkoxy, Niederalkoxyniederalkoxyniederalkyl, unsubstituiertes oder durch Niederalkyl, Niederalkoxy, Hydroxy, Halogen, Nitro und/oder Amino substituiertes Phenylniederalkoxy, gegebenenfalls N-oxidiertes Pyridylniederalkoxy, Niederalkylthioniederalkoxy, Niederalkansulfonylniederalkoxy, Niederalkanoylniederalkoxy, gegebenenfalls N-oxidiertes Pyridylniederalkoxy, Cyanoniederalkoxy, Carboxyniederalkoxy, Niederalkoxycarbonylniederalkoxy, Carbamoylniederalkoxy Niederalkylcarbamoylniederalkoxy oder Diniederalkylcarbamoylniederalkoxy bedeutet,
R₈ Wasserstoff, Niederalkyl, Hydroxy, Niederalkoxy, oder Polyhalogenniederalkoxy bedeutet oder gemeinsam mit R₉ Niederalkylendioxy darstellt und
R₉ Wasserstoff bedeutet oder gemeinsam mit R₈ Niederalkylendioxy darstellt, und ihre Salze.

Die Erfindung betrifft vorzugsweise Verbindungen der Formel Ib, worin
R₂ für verzweigtes C₁-C₄-Alkyl, wie Isopropyl, 3- bis 5-gliedriges oder Cycloalkyl-C₁-C₄-alkyl, wie Cyclopropylmethyl, steht,
R₃ für Carboxy, C₁-C₄-Alkoxycarbonyl, Formyl oder Hydroxymethyl steht,
R₄ C₁-C₄-Alkoxycarbonyl, wie Terti ärbutyloxycarbonyl, oder α-Phenyl-C₁-C₄-alkoxycarbonyl, wie Benzyloxycarbonyl, bedeutet,
R₅ und R₆ Wasserstoff bedeuten,
R₇ C₁-C₄-Alkyl, wie Methyl oder Tertiärbutyl, C₁-C₄-Alkoxy-C₁-C₄-alkyl, C₁-C₄-Alkoxy-C₁-C₄-alkoxy, wie 3-Methoxypropyloxy, C₁-C₄-Alkoxy-C₁-C₄-alkoxy-C₁-C₄-alkyl, wie 3-Methoxybutyl, Phenyl-C₁-C₄-alkoxy, wie Benzyloxy, Pyridyl-C₁-C₄-alkoxy, wie Pyridylmethoxy, C₁-C₄-Alkyl-thio-C₁-C₄-alkoxy, wie 3-Methylthiopropyloxy, C₁-C₄-Alkansulfonyl-C₁-C₄-alkoxy, wie 3-Methansulfonylpropyloxy, C₁-C₄-Alkanoyl-C₁-C₄-alkoxy, C₁-C₄-Alkoxycarbonyl-C₁-C₄-alkoxy, wie Methoxy- oder Ethoxycarbonylmethoxy, Carbamoyl-C₁-C₄-alkoxy, wie Carbamoylmethoxy, oder Di-C₁-C₄-alkylcarbamoyl-C₁-C₄-alkoxy, wie Dimethylcarbamoylmethoxy, bedeutet,
R₈ Wasserstoff, C₁-C₄-Alkyl, wie Methyl oder Tertiärbutyl, Hydroxy oder C₁-C₄-Alkoxy, wie Methoxy, bedeutet oder gemeinsam mit R₉ C₁-C₄-Alkylendioxy darstellt und
R₉ Wasserstoff bedeutet oder gemeinsam mit R₈ C₁-C₄-Alkylendioxy darstellt,
und ihre Salze.

Die Erfindung betrifft in allererster Linie Verbindungen der Formel Ib, worin
R₂ verzweigtes C₁-C₄-Alkyl, wie Isopropyl, bedeutet,
R₃ Carboxy, Formyl oder Hydroxymethyl ist,
R₄ für C₁-C₄-Alkoxycarbonyl steht,
R₅, R₆ und R₉ Wasserstoff darstellen,
R₇ für C₁-C₄-Alkoxy-C₁-C₄-alkoxy, wie 3-Methoxypropyloxy, oder C₁-C₄-Alkoxy-C₁-C₄-alkyl, wie 3-Methoxybutyl, steht und
R₈ Wasserstoff, C₁-C₄-Alkyl, wie Isopropyl oder Tertiärbutyl, oder C₁-C₄-Alkoxy, wie Methoxy, darstellt,
und ihrer Salze.

Die Erfindung betrifft namentlich die in den Beispielen genannten Verbindungen der Formel I und ihre Salze, insbesondere ihre pharmazeutisch verwendbaren Salze.

Das Verfahren zur Herstellung von Verbindung der Formel I ist dadurch gekennzeichnet, daß man eine Verbindung der Formel IX 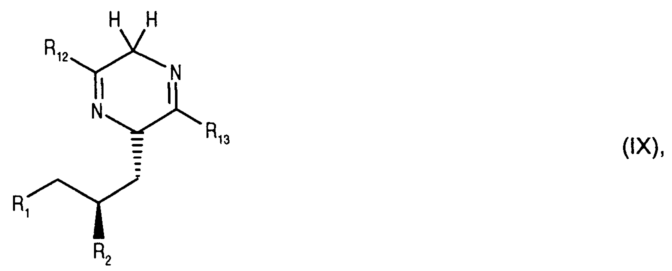
worin R₁₂ und R₁₃ unabhängig voneinander für aliphatisch, araliphatisch oder aromatisch verethertes Hydroxy, insbesondere für Niederalkoxy, stehen, R₁ und R₂ die vorstehend unter der Formel I angegebenen Bedeutungen haben, unter Aufspaltung des Pyrazinringes zur entsprechenden Verbindung der Formel I hydrolysiert, worin R₃ für aliphatisch, araliphatisch oder aromatisch verestertes Carboxy der Formel -C(=O)-R₁₃ steht, und gewünschtenfalls eine verfahrensgemäß erhältliche Verbindung in eine andere Verbindung der Formel I umwandelt, gegebenenfalls erhältliche Isomerengemische auftrennt und/oder eine verfahrensgemäß erhältliche Verbindung der Formel I mit mindestens einer salzbildenden Gruppe in ein Salz oder ein verfahrensgemäß erhältliches Salz in die freie Verbindung oder in ein anderes Salz überführt.

Die Ausgangsstoffe der Formel IX können beispielsweise hergestellt werden, indem man eine Verbindung der Formel X 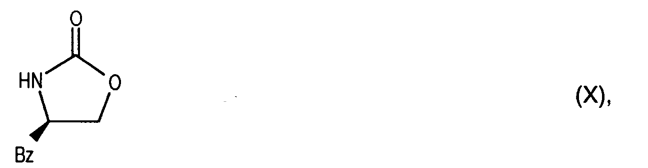
mit einer Verbindung der Formel 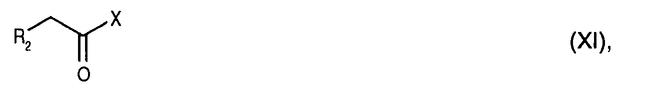
umsetzt, worin X reaktionsfähiges verestertes Hydroxy, beispielsweise Halogen, wie Chlor, oder eine Gruppe der Formel -O-C=O-CH₂-R₂ bedeutet, die erhaltene Verbindung der Formel XII 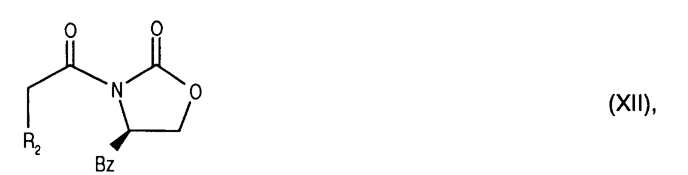
durch Umsetzung mit einer Metallbase, wie Lithiumhexamethyldisilazid in einem cyclischen Ether, wie Tetrahydrofuran, vorzugsweise unter Kühlung auf etwa -80°C bis -60°C, vorzugsweise bei etwa -70°C, metalliert und anschließend mit einer Verbindung der Formel XIII 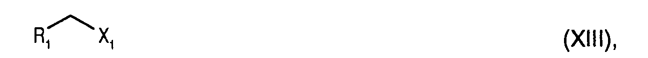
worin X₁ reaktionsfähiges verestertes Hydroxy, insbesondere Halogen, wie Brom, bedeutet, kondensiert, in der erhaltenen Verbindung der Formel XIV 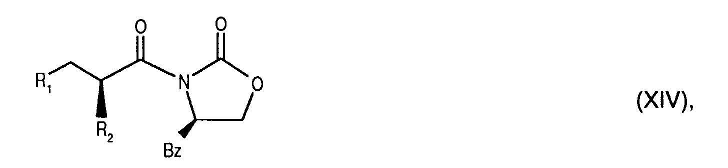
die 4-Benzyl-2-oxo-oxazolidin-1-ylcarbonylgruppe, beispielsweise mittels Lithiumhydroxid/Wasserstoffperoxid, selektiv zu Carboxy hydrolysiert, die Carboxygruppe, beispielsweise mittels Natriumborhydrid/Jod in Tetrahydrofuran, zu Hydroxymethyl reduziert, die Hydroxymethylgruppe, z.B. mit N-Bromsuccinimid/Triphenylphosphin in Dichlormethan, halogeniert und das Reaktionsprodukt der Formel XV 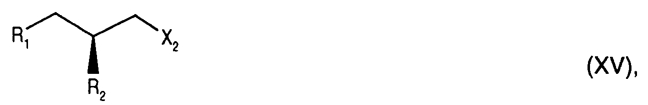
worin X₂ reaktionsfähiges verestertes Hydroxy, insbesondere Halogen, beispielsweise Brom, ist, mit einem Metallsalz einer Verbindung der Formel XVI 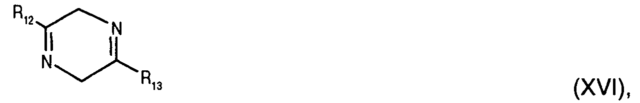
erhältlich aus derselben durch Behandlung mit einer Metallbase, beispielsweise mit Butyllithium in Hexan unter Kühlung auf etwa -80° C bis -60° C, vorzugsweise bei etwa -70° C, kondensiert.

In analoger Weise erhält man auch Verbindungen der Formel 1, worin R₁ und R₂ gemeinsam einen zweiwertigen aliphatischen Rest, insbesondere Niederalkylen, darstellen, indem man den Ausgangsstoff der Formel X statt mit einer Verbindung der Formel XI mit einer Verbindung der Formel XVII 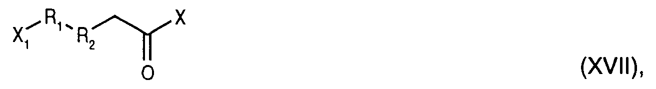
worin X reaktionsfähiges verestertes Hydroxy, beispielsweise Halogen, wie Chlor, oder eine Gruppe der Formel O-(C=O)-CH₂-R₂-R₁-X₁, bedeutet, kondensiert, in dem Reaktionsprodukt der Formel XVIII 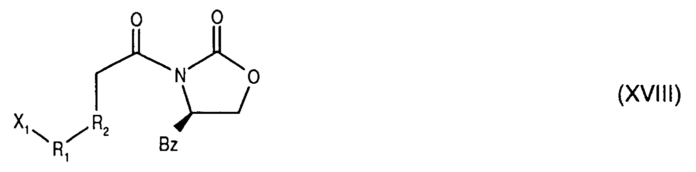
die 4-Benzyl-2-oxo-oxazolidin-1-ylcarbonylgruppe selektiv zu Carboxy hydrolysiert, die Carboxygruppe zu Hydroxymethyl reduziert, die Hydroxymethylgruppe halogeniert und das Raktionsprodukt der Formel XIX 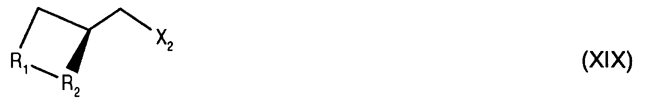
mit einem Metallsalz einer Verbindung der Formel XVI umsetzt.

Erfindungsgemäß erhältliche Verbindungen der Formel I kann man, wie erwähnt, in andere Verbindungen der Formel I überführen.

So kann man in Verbindungen der Formel 1, worin R₃ verestertes Carboxy ist, die veresterte Carboxygruppe in üblicher Weise, beispielsweise durch basische oder saure Hydrolyse, in Carboxy überführen. Umgekehrt kann man Carboxy R₃ in üblicher Weise, beispielsweise durch Umsetzung mit einem Alkohol in Gegenwart eines sauren Mittels, oder durch Überführung in Chlorcarbonyl und Weiterumsetzung mit einem Alkohol in Gegenwart eines basischen Kondensationsmittels verestern.

Auch kann man Verbindungen der Formel 1, worin R₃ gegebenenfalls verestertes Carboxy ist, in üblicher Weise, beispielsweise mittels Dibutylaluminiumhydrid in Toluol oder durch Überführung in das entsprechende Säurechlorid, beispielsweise durch Umsetzung mit Oxalylchlorid, und anschließende Reduktion z.B. mittels Natriumtritertiärbutyloxyaluminiumhydrid in Tetrahydrofuran zum entsprechenden Aldehyd, worin R₁ Formyl ist, reduzieren.

Weiterhin kann man in erhaltenen Verbindungen der Formel 1, worin R₄ und R₅ Wasserstoff bedeuten, in üblicher Weise eine Aminoschutzgruppe oder einen aliphatischen oder araliphatischen Rest R₄ und gewünschtenfalls einen aliphatischen Rest R₅ einführen. So kann man ein primäres Amin durch Umsetzung mit einem Alkylierungsmittel, Aryalkylierungsmittel oder Alkanoylierungsmittel in Gegenwart eines basischen Kondensationsmittels N-mono- oder N,N-diniederalkylieren, N-aralkylieren bzw. N-niederalkanoylieren. In analoger Weise kann man auch eine Aminoschutzgruppe einführen, Tertiärbutyloxycarbonyl beispielsweise durch Umsetzung mit Ditertiärbutyldicarbonat in Gegenwart eines Triniederalkylamins, wie von N,N-Diisopropyl-N-ethyl-amin.

In Verbindungen der Formeln I, die als Rest R₁ eine Gruppe der Formel la aufweisen, kann man ferner Hydroxy R₆, R₂, R₈ und/oder R₉ durch eine der unter den Formeln I bzw. II genannten veretherten Hydroxygruppen ersetzen, indem man die entsprechende Verbindung der Formeln I, II, VI, VIa und VII, worin R₆, R₂, R₈ und/oder R₉ Hydroxy ist, in üblicher Weise, beispielsweise in Gegenwart eines basischen Kondensationsmittels, mit einer Verbindung der Formeln R'₆-Y, R'₇-Y, R'₈-Y und/oder R'₉-Y umsetzt, worin R'₆ Niederalkyl oder gegebenenfalls verestertes oder amidiertes Carboxyniederalkyl bedeutet, R'7 Niederalkyl, Niederalkoxyniederalkyl, Niederalkoxyniederalkoxyniederalkyl, Cycloalkoxyniederalkyl, gegebenenfalls niederalkanoyliertes, halogeniertes oder sulfonyliertes Hydroxyniederalkyl, Oxoniederalkyl, Niederalkyl, Niederalkenyl, Cycloalkoxyniederalkyl, Niederalkoxyniederalkyl, Niederalkoxyniederalkenyl, Niederalkenyloxyniederalkyl, Niederalkenyloxyniederalkyl, Niederalkanoylniederalkyl, gegebenenfalls S-oxidiertes Niederalkylthioniederalkyl, Niederalkylthio(hydroxy)niederalkyl, Arylniederalkyl, gegebenenfalls hydriertes Heteroarylniederalkyl, gegebenenfalls hydriertes Heteroarylthioniederalkyl, Cyanoniederalkyl oder gegebenenfalls verestertes oder amidiertes Carboxyniederalkyl bedeutet, R'8 Niederalkyl, Niederalkoxyniederalkyl, Hydroxyniederalkyl, Arylniederalkyl, halogeniertes Niederalkyl, Cyanoniederalkyl oder gegebenenfalls verestertes oder amidiertes Carboxyniederalkyl bedeutet, und R'₉ Niederalkyl steht, und Y reaktionsfähiges verestertes Hydroxy, insbesondere mit einer Mineralsäure, mit Schwefelsäure oder mit einer organischen Sulfonsäure veresterte Hydroxygruppen, wie Halogen, vorzugsweise Chlor, Brom oder Jod, Gruppen der Formel O-SO₂-O-R'_{A}, oder Niederalkan- oder gegebenenfalls substituiertes Benzolsulfonyloxy, insbesondere Methan-, Ethan-, Benzol-, p-Toluol- oder p-Brombenzolsulfonyloxy, bedeutet. Die Umsetzung erfolgt, wie erwähnt, vorzugsweise in Gegenwart eines basischen Kondensationsmittels, wie einem Alkalimetallcarbonat, beispielsweise von Kaliumcarbonat, in einem inerten Lösungsmittel, wie einem Niederalkanol, wie Methanol, Ethanol, Butanol, Tertiärbutanol oder insbesondere Amylalkohol, vorteilhaft bei erhöhter Temperatur, beispielsweise im Temperaturbereich von etwa 40° bis 140° C, erforderlichenfalls unter, beispielsweise azeotroper, Abdestillation des gebildeten Reaktionswassers.

Ferner kann man verfahrensgemäß erhältliche Salze von Verbindungen der Formeln I in an sich bekannter Weise in die freien Verbindungen umwandeln, z.B. durch Behandeln mit einer Base, wie einem Alkalimetallhydroxid, einem Metallcarbonat oder -hydrogencarbonat, oder Ammoniak, oder einer anderen eingangs genannten salzbildenden Base bzw. mit einer Säure, wie einer Mineralsäure, z.B. mit Chlorwasserstoff, oder einer anderen eingangs genannten salzbildenden Säure.

Erhaltene Salze können in an sich bekannter Weise in andere Salze überführt werden, Säureadditionssalze z.B. durch Behandeln mit einem geeigneten Metallsalz, wie einem Natrium-, Barium- oder Silbersalz, einer anderen Säure in einem geeigneten Lösungsmittel, in welchem ein sich bildendes anorganisches Salz unlöslich ist und damit aus dem Reaktionsgleichgewicht ausscheidet, und Basesalze durch Freisetzung der freien Säure und erneute Versalzung.

Die Verbindungen der Formeln 1, einschließlich ihrer Salze, können auch in Form von Hydraten erhalten werden oder das zur Kristallisation verwendete Lösungsmittel einschließen.

Infolge der engen Beziehung zwischen den neuen Verbindungen in freier Form und in Form ihrer Salze sind vorstehend und nachfolgend unter den freien Verbindungen und ihren Salzen sinn- und zweckgemäß gegebenenfalls auch die entsprechenden Salze bzw. freien Verbindungen zu verstehen.

Die Erfindung betrifft auch diejenigen Ausführungsformen des Verfahrens, bei denen man von einer auf irgend einer Stufe als Zwischenprodukt erhältlichen Verbindung ausgeht und die fehlenden Schritte durchführt oder das Verfahren auf irgendeiner Stufe abbricht oder einen Ausgangsstoff unter den Reaktionsbedingungen bildet oder in Form eines reaktionsfähigen Derivates oder Salzes verwendet oder eine nach dem erfindungsgemäßen Verfahren erhältliche Verbindung unter den Verfahrensbedingungen erzeugt und in situ weiterverarbeitet. Dabei geht man vorzugsweise von solchen Ausgangsstoffen aus, die zu den oben als sehr bevorzugt oder ganz besonders bevorzugt beschriebenen Verbindungen führen.

Die neuen Ausgangsstoffe, die speziell für die Herstellung der erfindungsgemäßen Verbindungen entwickelt wurden, insbesondere die zu den eingangs als bevorzugt gekennzeichneten Verbindungen der Formel I führende Ausgangsstoffauswahl, die Verfahren zu ihrer Herstellung und ihre Verwendung als Zwischenprodukte bilden ebenfalls einen Gegenstand der Erfindung.

Dies betrifft insbesondere Verbindungen der Formel IX, die sich, wie erwähnt, als Zwischenprodukte zur Herstellung von Verbindungen der Formel I eignen.

Die Erfindung betrifft dementsprechend auch Verbindungen der Formel IX 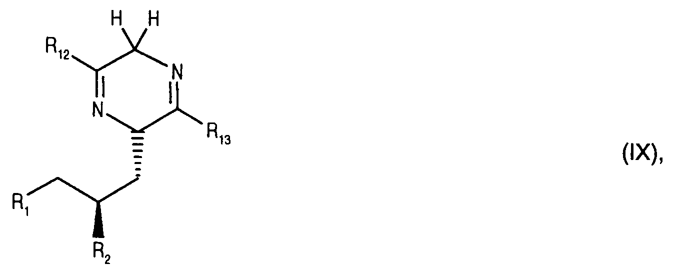
worin R₁ einen aliphatischen, cycloaliphatischen, cycloaliphatisch-aliphatischen, aromatischen oder heteroaromatischen Rest, eine aliphatisch, araliphatisch oder heteroarylaliphatisch veretherte oder durch eine Hydroxyschutzgruppe geschützte Hydroxygruppe oder eine aliphatisch veretherte Mercaptogruppe und
R₂ einen aliphatischen, cycloaliphatischen, cycloaliphatisch-aliphatischen, araliphatischen oder heteroarylaliphatischen Rest bedeutet oder
R₁ und R₂ gemeinsam einen zweiwertigen aliphatischen Rest darstellen und R₁₂ und R₁₃ unabhängig voneinander für aliphatisch, araliphatisch oder aromatisch verethertes Hydroxy stehen, und ihre Salze sowie Verfahren zu ihrer Herstellung.

Die Erfindung betrifft in erster Linie Verbindungen der Formel IX, worin
R₁ Niederalkyl, Niederalkenyl, 3- bis 8-gliedriges Cycloalkyl, 3- bis 8-gliedriges Cycloalkylniederalkyl, einen unsubstituierten oder durch Aminoniederalkoxy, Aminoniederalkyl, Arylniederalkoxy, Carbamoylniederalkoxy, Carbamoylniederalkyl, Carboxyniederalkoxy, Carboxyniederalkyl, Cyanoniederalkoxy, Cyanoniederalkyl, 3- bis 8-gliedriges Cycloalkoxy, 3- bis 8-gliedriges Cycloalkoxyniederalkoxy, 3- bis 8-gliedriges Cycloalkoxyniederalkyl, 3- bis 8-gliedriges Cycloalkyl, Diniederalkylamino, Diniederalkylaminoniederalkoxy, Diniederalkylaminoniederalkyl, Pyridylniederalkoxy, Tertrahydropyridylniederalkoxy, N-Oxidopyridylniederalkoxy, Thiazolylniederalkoxy, Morpholinoniederalkoxy, Pyridylthioniederalkoxy, N-Oxidopyridylthioniederalkoxy, Pyridylthioniederalkyl, N-Oxidopyridylthioniederalkyl, Halogen(hydroxy)niederalkoxy, Halogen, Hydroxy, Hydroxyniederalkoxy, Hydroxyniederalkyl, Imidazolylthioniederalkoxy, Imidazolylthioniederalkyl, Morpholinoniederalkoxy, Morpholinoniederalkyl, N-Mono- oder N,N-Diniederalkylcarbamoylniederalkoxy, N-Mono- oder N,N-Diniederalkylcarbamoylniederalkyl, gegebenenfalls N-oxidierte Pyridylniederalkyl, N'-Niederalkylpiperazino- bzw. N'-Niederalkanoylpiperazinoniederalkoxy, N'-Niederalkylpiperazino- bzw. N'-Niederalkanoylpiperazinoniederalkyl, Naphthyl, Naphthylniederalkoxy, Niederalkanoylaminoniederalkoxy, Niederalkanoylamino-niederalkyl, Niederalkanoylniederalkoxy, Niederalkanoyloxyniederalkyl, Niederalkansulfonyl(hydroxy)niederalkoxy, Niederalkansulfonylaminoniederalkoxy, Niederalkansulfonylaminoniederalkyl, Niederalkansulfonylniederalkoxy, Niederalkansulfonylniederalkyl, Niederalkenyloxy, Niederalkenyloxyniederalkoxy, Niederalkenyloxyniederalkyl, Niederalkoxy, Niederalkoxycarbonylaminoniederalkoxy, Niederalkoxycarbonylaminoniederalkyl, Niederalkoxycarbonylniederalkoxy, Niederalkoxycarbonylniederalkyl, Niederalkoxyiminoniederalkyl, Niederalkoxyniederalkenyl, Niederalkoxyniederalkenyloxy, Niederalkoxyniederalkoxy, Niederalkoxyniederalkoxyniederalkyl, Niederalkoxyniederalkyl, Niederalkyl, Niederalkylamino, Niederalkylaminoniederalkoxy, Niederalkylaminoniederalkyl, Niederalkylthio(hydroxy)niederalkoxy, Niederalkylthioniederalkoxy, Niederalkylthioniederalkoxy, Niederalkylthioniederalkyl, Oxoniederalkoxy, Piperazinoniederalkoxy, Piperazinoniederalkyl, Piperidinoniederalkoxy, Piperidinoniederalkyl, Polyhalogenniederalkansulfonylaminoniederalkoxy, Polyhalogenniederalkansulfonylaminoniederalkyl, Polyhalogenniederalkoxy, Polyhalogenniederalkyl, Pyrimidinylthioniederalkoxy, Pyrimidinylthioniederalkoxy, Pyrimidinylthioniederalkyl, Pyrrolidinoniederalkoxy, Pyrrolidinoniederalkyl, S,S-Dioxothiomorpholinoniederalkoxy, S,S-Dioxothiomorpholinoniederalkyl, S-Oxothiomorpholinoniederalkoxy-, S-Oxothiomorpholinoniederalkyl, Thiazolylthioniederalkoxy, Thiazolinylthioniederalkoxy, Thiazolylthioniederalkyl, Thiazolinylthioniederalkyl und/oder Thiomorpholino mono-, di- oder trisubstituierten Phenyl- oder Naphthyl- oder durch einen ankondensierten Benzo- oder Cyclohexenoring disubstituierten Phenylrest, einen unsubstituierten oder durch Niederalkyl, Niederalkoxy, Halogen und/oder Trifluormethyl substituierten Furyl-, Thienyl-, Pyridyl-, Pyrimidinyl-, Indolyl- oder Chinolinylrest, Niederalkoxy, Niederalkenyloxy, unsubstituierte oder im Phenylteil durch Niederalkyl, Niederalkoxy, Halogen und/oder Trifluormethyl substituiertes Phenylniederalkoxy, unsubstituiertes oder im Ring durch Niederalkyl, Niederalkoxy, Halogen und/oder Trifluormethyl substituierte Furylniederalkoxy-, Thienylniederalkoxy-, Pyridylniederalkoxyoder Chinolinylniederalkoxygruppen, Niederalkanoyloxy, Trihalogenniederalkanoyloxy, eine unsubstituierte oder durch Niederalkyl, Niederalkoxy, Halogen, Trifluormethyl und/oder Nitro substituierte Benzoyloxy- oder Phenylniederalkoxycarbonyloxygruppe, Triniederalkylsilyloxy, Benzyl(diniederalkyl)silyloxy oder Niederalkylthio bedeutet,
R₂ Niederalkyl, 3- bis 8-gliedriges Cycloalkyl, 3- bis 8-gliedriges Cycloalkylniederalkyl, unsubstituiertes oder im Phenyl- , Naphthyl- bzw. Heteroarylteil durch Niederalkyl, Niederalkoxy, Halogen und/oder Trifluormethyl substituierten Phenylniederalkyl-, Naphthylniederalkyl-, Furylniederalkyl-, Thienylniederalkyl-, gegebenenfalls partiell hydriertes oder N-oxidierten Pyridylniederalkyl-, Pyrimidinylniederalkyl- oder Chinolinylniederalkylrest bedeutet oder R₁ und R₂ gemeinsam Niederalkylen, dessen freie Valenzen von benachbarten oder zueinander 1,3-, 1,4- oder 1,5-ständigen C-Atomen ausgehen, darstellen und R₁₂ und R₁₃ unabhängig voneinander für Niederalkoxy, Niederalkenyloxy, unsubstituiertes oder durch Niederalkyl, Niederalkoxy, Halogen und/oder Trifluormethyl substituiertes Phenyloxy oder unsubstituiertes oder im Phenyl- bzw. Naphthylteil durch Niederalkyl, Niederalkoxy, Halogen und/oder Trifluormethyl substituiertes Phenylniederalkoxy oder Naphthylniederalkoxy stehen, und ihre Salze.

Die Erfindung betrifft vor allem Verbindungen der Formel IXa 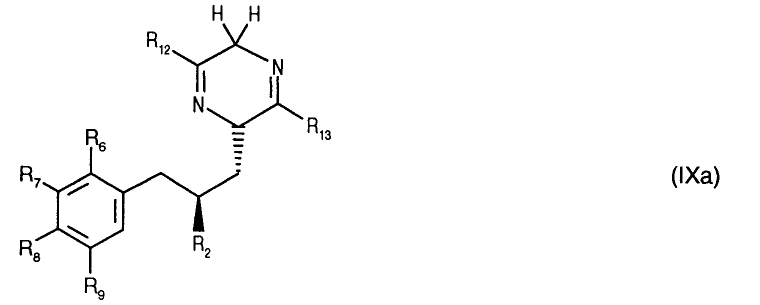
bedeutet, worin
R₂ Niederalkyl, 3- bis 8-gliedriges Cycloalkyl, 3- bis 8-gliedriges Cycloalkylniederalkyl, unsubstituiertes oder im Phenyl-, Naphthyl- bzw. Heteroarylteil durch Niederalkyl, Niederalkoxy, Halogen und/oder Trifluormethyl substituierten Phenylniederalkyl-, Naphthylniederalkyl-, Furylniederalkyl-, Thienylniederalkyl-, gegebenenfalls partiell hydriertes oder N-oxidierten Pyridylniederalkyl-, Pyrimidinylniederalkyl- oder Chinolinylniederalkylrest bedeutet,
R₆ Wasserstoff, Hydroxy, Niederalkoxy, Cycloalkoxy, Niederalkoxyniederalkoxy, Carboxyniederalkoxy, Niederalkoxycarbonylniederalkoxy, Carbamoylniederalkoxy oder N-Mono- oder N,N- Diniederalkylcarbamoylniederalkoxy bedeutet,
R₇ Wasserstoff, Niederalkyl, Cycloalkyl, Niederalkoxyniederalkyl, Niederalkoxyniederalkoxyniederalkyl, Cycloalkoxyniederalkyl, Hydroxy, Niederalkanoyloxyniederalkyl, Hydroxyniederalkoxy, Halogen(hydroxy)niederalkoxy, Niederalkansulfonyl(hydroxy)niederalkoxy, Aminoniederalkyl, Niederalkylaminoniederalkyl, Diniederalkylaminoniederalkyl, Niederalkanoylaminoniederalkyl, Niederalkoxycarbonylaminoniederalkyl, Niederalkoxyiminoniederalkyl, Aminoniederalkoxy, Niederalkylaminoniederalkoxy, Diniederalkylaminoniederalkoxy, Niederalkanoylaminoniederalkoxy, Niederalkoxycarbonylaminoniederalkoxy, Oxoniederalkoxy, Niederalkoxy, Cycloalkoxy, Niederalkenyloxy, Cycloalkoxyniederalkoxy, Niederalkoxyniederalkoxy, Niederalkoxyniederalkenyl, Niederalkenyloxyniederalkoxy, Niederalkoxyniederalkenyloxy, Niederalkenyloxyniederalkyl, Niederalkanoylniederalkoxy, Niederalkylthioniederalkoxy, Niederalkansulfonylniederalkoxy, Niederalkylthio(hydroxy)niederalkoxy, Arylniederalkoxy, Thiazolylthioniederalkoxy bzw. Thiazolinylthioniederalkoxy, Imidazolylthioniederalkoxy, gegebenenfalls N-oxidierte Pyridylthioniederalkoxy, Pyrimidinylthioniederalkoxy, Cyanoniederalkoxy, Niederalkoxycarbonylniederalkoxy, Carbamoylniederalkoxy, N-Mono- oder N,N-Diniederalkylcarbamoylniederalkoxy, Carboxyniederalkyl, Niederalkoxycarbonylniederalkyl, Carbamoylniederalkyl oder N-Mono- oder N,N-Diniederalkylcarbamoylniederalkyl bedeutet,
R₈ Niederalkyl, Polyhalogenniederalkyl, Niederalkoxyniederalkyl, Cycloalkoxyniederalkyl, Hydroxyniederalkyl, Niederalkylthioniederalkyl, Niederalkansulfonylniederalkyl, gegebenenfalls partiell hydriertes oder N-oxidierte Pyridylniederalkyl, Thiazolylthioniederalkyl bzw. Thiazolinylthioniederalkyl, Imidazolylthioniederalkyl, gegebenenfalls N-oxidiertes Pyridylthioniederalkyl, Pyrimidinylthioniederalkyl, Aminoniederalkyl, Niederalkylaminoniederalkyl, Diniederalkylaminoniederalkyl, Niederalkanoylaminoniederalkyl, Niederalkansulfonylaminoniederalkyl, Polyhalogenniederalkansulfonylaminoniederalkyl, Pyrrolidinoniederalkyl, Piperidinoniederalkyl, Piperazino-, N'-Niederalkylpiperazino- bzw. N'-Niederalkanoylpiperazinoniederalkyl, Morpholinoniederalkyl, Thiomorpholino-, S-Oxothiomorpholino- oder S,S-Dioxothiomorpholinoniederalkyl, Cyanoniederalkyl, Carboxyniederalkyl, Niederalkoxycarbonylniederalkyl, Carbamoylniederalkyl, N-Mono- oder N,N-Diniederalkylcarbamoylniederalkyl, Cycloalkyl, unsubstituiertes oder durch Niederalkyl, Niederalkoxy, Hydroxy, Niederalkylamino, Diniederalkylamino, Halogen und/oder Trifluormethyl mono-, di- oder trisubstituiertes Phenyl oder Naphthyl, Hydroxy, Niederalkoxy, Cycloalkoxy, Niederalkoxyniederalkoxy, Cycloalkoxyniederalkoxy, Hydroxyniederalkoxy, unsubstituiertes oder durch Niederalkyl, Niederalkoxy, Hydroxy, Niederalkylamino, Diniederalkylamino, Halogen und/oder Trifluormethyl mono-, di- oder trisubstituiertes Phenylniederalkoxy oder Naphthylniederalkoxy, Niederalkoxy, Polyhalogenniederalkoxy, Niederalkylthioniederalkoxy, Niederalkansulfonylniederalkoxy, gegebenenfalls hydriertes Heteroarylniederalkoxy, gegebenenfalls partiell oder vollständig hydriertes Heteroarylthioniederalkoxy, wie Thiazolylthioniederalkoxy bzw. Thiazolinylthioniederalkoxy, Imidazolylthioniederalkoxy, gegebenenfalls N-oxidierte Pyridylthioniederalkoxy, Pyrimidinylthioniederalkoxy, Aminoniederalkoxy, Niederalkylaminoniederalkoxy, Diniederalkylaminoniederalkoxy, Niederalkanoylaminoniederalkoxy, Niederalkansulfonylaminoniederalkoxy, Polyhalogenniederalkansulfonylaminoniederalkoxy, Pyrrolidinoniederalkoxy, Piperidinoniederalkoxy, Piperazino-, N'-Niederalkylpiperazino- bzw. N'-Niederalkanoylpiperazinoniederalkoxy, Morpholinoniederalkoxy, Thiomorpholino-, S-Oxothiomorpholino- oder S,S-Dioxothiomorpholinoniederalkoxy, Cyanoniederalkoxy, Carboxyniederalkoxy, Niederalkoxycarbonylniederalkoxy, Carbamoylniederalkoxy oder N-Mono- oder N,N-Diniederalkylcarbamoylniederalkoxy bedeutet oder gemeinsam mit R₉ Niederalkylendioxy oder einen ankondensierten Benzo- oder Cyclohexenoring darstellt,
R₉ gemeinsam mit R₈ Niederalkylendioxy oder einen ankondensierten Benzo- oder Cyclohexenoring darstellt oder für Wasserstoff, Niederalkyl, Hydroxy, Niederalkoxy oder Cycloalkoxy steht und
R₁₂ und R₁₃ unabhängig voneinander für Niederalkoxy, Niederalkenyloxy, unsubstituiertes oder durch Niederalkyl, Niederalkoxy, Halogen und/oder Trifluormethyl substituiertes Phenyloxy oder unsubstituiertes oder im Phenyl- bzw. Naphthylteil durch Niederalkyl, Niederalkoxy, Halogen und/oder Trifluormethyl substituiertes Phenylniederalkoxy oder Naphthylniederalkoxy stehen,
und ihre Salze.

Die Erfindung betrifft insbesondere Verbindungen der Formel IXa, worin
R₂ für Niederalkyl, 3- bis 5-gliedriges Cycloalkylniederalkyl oder Cycloalkyl steht,
R₆ Wasserstoff bedeutet,
R₇ Niederalkyl, Niederalkoxyniederalkyl, Niederalkoxyniederalkoxy, Niederalkoxyniederalkoxyniederalkyl, unsubstituiertes oder durch Niederalkyl, Niederalkoxy, Hydroxy, Halogen, Nitro und/oder Amino substituiertes Phenylniederalkoxy, gegebenenfalls N-oxidiertes Pyridylniederalkoxy, Niederalkylthioniederalkoxy, Niederalkansulfonylniederalkoxy, Niederalkanoylniederalkoxy, gegebenenfalls N-oxidiertes Pyridylniederalkoxy, Cyanoniederalkoxy, Carboxyniederalkoxy, Niederalkoxycarbonylniederalkoxy, Carbamoylniederalkoxy Niederalkylcarbamoylniederalkoxy oder Diniederalkylcarbamoylniederalkoxy bedeutet,
R₈ Wasserstoff, Niederalkyl, Hydroxy, Niederalkoxy, oder Polyhalogenniederalkoxy bedeutet oder gemeinsam mit R₉ Niederalkylendioxy darstellt,
R₉ Wasserstoff bedeutet oder gemeinsam mit R₈ Niederalkylendioxy darstellt und
R₁₂ und R₁₃ unabhängig voneinander für Niederalkoxy, Niederalkenyloxy, unsubstituiertes oder durch Niederalkyl, Niederalkoxy, Halogen und/oder Trifluormethyl substituiertes Phenyloxy oder unsubstituiertes oder im Phenylteil durch Niederalkyl, Niederalkoxy, Halogen und/oder Trifluormethyl substituiertes Phenylniederalkoxy stehen, und ihre Salze.

Die Erfindung betrifft in allererster Linie Verbindungen der Formel IXa, worin
R₂ für verzweigtes C₁-C₄-Alkyl, wie Isopropyl, 3- bis 5-gliedriges oder Cycloalkyl-C₁-C₄-alkyl, wie Cyclopropylmethyl, steht,
R₆ Wasserstoff bedeutet,
R₇ C₁-C₄-Alkyl, wie Methyl oder Tertiärbutyl, C₁-C₄-Alkoxy-C₁-C₄-alkyl, wie 3-Methoxypropyl, C₁-C₄-Alkoxy-C₁-C₄-alkoxy, wie 3-Methoxypropyloxy, C₁-C₄-Alkoxy-C₁-C₄-alkoxy-C₁-C₄-alkyl, wie 3-Methoxybutyl, Phenyl-C₁-C₄-alkoxy, wie Benzyloxy, Pyridyl-C₁-C₄-alkoxy, wie Pyridylmethoxy, C₁-C₄-Alkylthio-C₁-C₄-alkoxy, wie 3-Methylthiopropyloxy, C₁-C₄-Alkansulfonyl-C₁-C₄-alkoxy, wie 3-Methansulfonylpropyloxy, C₁-C₄-Alkanoyloxy-C₁-C₄-alkoxy, wie 3-Acetoxypropyloxy, C₁-C₄-Alkoxycarbonyl-C₁-C₄-alkoxy, wie Methoxy- oder Ethoxycarbonylmethoxy, Carbamoyl-C₁-C₄-alkoxy, wie Carbamoylmethoxy, oder Di-C₁-C₄-alkylcarbamoyl-C₁-C₄-alkoxy, wie Dimethylcarbamoylmethoxy, bedeutet,
R₈ Wasserstoff, C₁-C₄-Alkyl, wie Methyl oder Tertiärbutyl, Hydroxy oder C₁-C₄-Alkoxy, wie Methoxy, bedeutet oder gemeinsam mit R₉ C₁-C₄-Alkylendioxy, wie Methylendioxy oder Ethylendioxy, darstellt,
R₉ Wasserstoff bedeutet oder gemeinsam mit R₈ C₁-C₄-Alkylendioxy, wie Methylendioxy oder Ethylendioxy, darstellt und
R₁₂ und R₁₃ gleiche oder verschiedene C₁-C₄-Alkoxy- oder C₂-C₄-Alkenyloxygruppen, wie Methoxy, Ethoxy, Propyloxy oder Allyloxy, bedeuten,
und ihrer Salze.

Die Erfindung betrifft namentlich die in den Beispielen genannten Verbindungen der Formel IX und ihre Salze.

Das erfindungsgemäße Verfahren zur Herstellung von Verbindungen der IX ist dadurch gekennzeichnet, daß man eine Verbindung der Formel 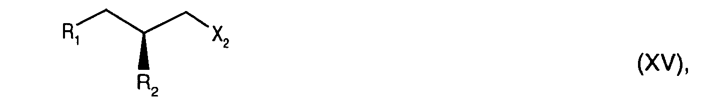
worin X₂ reaktionsfähiges verestertes Hydroxy, insbesondere Halogen, beispielsweise Brom, ist, mit einem Metallsalz einer Verbindung der Formel XVI 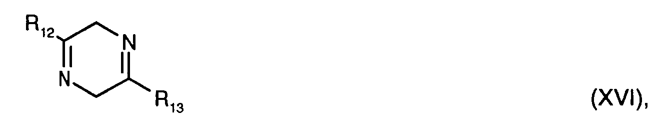
erhältlich aus derselben durch Behandlung mit einer Metallbase kondensiert und gewünschtenfalls eine erfindungsgemäße Verbindung der Formel IX in eine andere erfindungsgemäße Verbindung der Formel IX umwandelt, oder gegebenenfalls erhältliche Isomerengemische auftrennt und/oder die verfahrensgemäß erhältliche Verbindung der Formel IX in ein Salz oder ein verfahrensgemäß erhältliches Salz in die freie Verbindung oder in ein anderes Salz überführt.

Die selektive Hydrolyse der 4-Benzyl-2-oxo-oxazolidin-1-ylcarbonylgruppe zu Carboxy erfolgt in üblicher Weise, beispielsweise mittels Lithiumhydroxid/Wasserstoffperoxid.

Die Reduktion der Hydroxymethylgruppe erfolgt nach üblichen Methoden, beispielsweise mittels Natriumborhydrid/Jod in Tetrahydrofuran Die Halogenierung der Hydroxymethylgruppe erfolgt nach üblichen Methoden, beispielsweise mittels N-Bromsuccinimid/Triphenylphosphin in Dichlormethan.

Metallsalze von Verbindungen der Formel XVI werden in üblicher Weise durch Umsetzung mit einer Alkali- oder Erdalkalimetallverbindung eines aliphatischen Kohlenwasserstoffes gebildet, vorzugsweise mit Butyllithium in Hexan unter Kühlung auf etwa -80° C bis -60° C, vorzugsweise bei etwa -70° C.

Die nachfolgenden Beispiele dienen zur Illustration der Erfindung; Temperaturen sind in Celsiusgraden, Drucke in mbar angegeben.

Massenspektroskopische Meßwerte werden entweder durch konventionelle MS oder nach der "Fast-Atom-Bombardment" (FAB-MS)-Methode erhalten. Die Massenangaben beziehen sich im ersten Fall auf das unprotonierte Molekülion (M)⁺ oder das protonierte Molekülion (M+H)⁺.

Die verwendeten Kurzbezeichnungen und Abkürzungen haben die folgenden Bedeutungen:
- C₁₈-Nucleosil®: Handelsname für mit Octadecylresten belegtes "Reversed Phase"- Säulenmaterial für HPLC (Nucleosil® 5C₁₈, Macherey & Nagel, BRD)
- FAB-MS: Fast-Atom-Bombardment Mass-Spectroscopy
- FC: Flash Säulenchromatographie
- HPLC: Hochleistungs-Flüssigchromatographie (verwendete Säulendimension: 250 x 4.6 mm; Stationäre Phase: Nucleosil® 5C₁₈; Mobile Phase: A) Wasser + 0.1 Vol.-% Trifluoressigsäure, B) Aceonitril + 0.1 Vol.-% Trifluoressigsäure; sofern nichts anderes angegeben ist, wird der folgende Fließmittelgradient verwendet:
20-100% B in 20min + 8min 100% B.
Fließmittelgradiet (I): linear in 60 min von 30 Vol.-% B + 70 Vol.-% A nach 90 Vol.-% B + 10 Vol.-% A.
- Hyflo®: Handelsname für Filterhilfsmittel (Fluka, Buchs, Schweiz)
- IR: Infrarotspektroskopie
- Kp: beim in Torr angegebenen Druck
- min: Minute(n)
- h: Stunde(n)
- L: Liter
- mL: Milliliter
- MS: Massenspektroskopie
- R_{f}: Verhältnis von Laufstrecke einer Substanz zu Entfernung der Laufmittelfront vom Startpunkt bei DC
- Rₜ: Retentionszeit einer Substanz bei HPLC (in Min)
- Schmp.: Schmelzpunkt (Temperatur).

### Herstellungsbeispiel 1: 2(S)-Amino-4(S)-[4-methoxy-3-(3-methoxypropoxy)-benzyl]-5-methyl-hexansäureethylester

Die Lösung des Rohproduktes 2(S)-{2(S)-[4-Methoxy-3-(3-methoxypropoxy)-benzyl]-3-methylbutyl}-3,6-diethoxy-2,5-dihydro-pyrazin (472 g) in Acetonitril (3.6 L) wird bei Raumtemperatur unter Rühren mit 1 N Salzsäure (3.6 L, 3.60 Mol) versetzt. Nach 30 min wird der Reaktionsansatz vorsichtig in eine eiskalte gesättigte wässerige Natriumhydrogencarbonatlösung gegossen. Man extrahiert mit Dichlormethan (3 x 4 L), wäscht die vereinten organischen Phasen mehrmals mit Wasser und engt ein. Nach dem Trocknen im Vakuum erhält man die rohe Titelverbindung als Öl (351 g):
R_{f}(Dichlormethan/Methanol/Ammoniak konz. 850:50:1) = 0.34.

Das Ausgangsmaterial kann beispielsweise folgendermaßen hergestellt werden:

### a) 3,6-Diethoxy-2,5-dihydro-pyrazin

Zu einer Lösung von Glycinanhydrid (256.7 g, 2.25 Mol) in Dichlormethan (2.5 L) wird unter Rühren bei Raumtemperatur eine Lösung von Triethyloxonium-tetrafluoroborat (1.07 kg, 5.36 Mol) in Dichlormethan (2.5 L) zugegeben. Die Suspension wird über 64 h bei Raumtemperatur gerührt, anschließend auf 0 °C gekühlt und zur Aufarbeitung mit einer Phosphat-Pufferlösung pH 7.36, hergestellt durch Lösen von Dikaliumhydrogenphosphat (3.29 kg, 4.19 Mol) und Kaliumdihydrogenphosphat (0.945 1.54 Mol) in Wasser (11.2 L), über 5 min versetzt. Die Mischung wird noch 30 min nachgerührt und dann über Hyflo® klarfiltriert. Der Filtrierrückstand wird mit Dichlormethan (3 L) nachgewaschen, die organische Phase der vereinten Filtrate wird abgetrennt und die Wasserphase mit Dichlormethan (2 x 2 L) extrahiert. Die vereinten organischen Phasen werden mit gesättigter Natriumchloridlösung (1 x 2 L) gewaschen, über Watte filtriert und eingeengt. Der Rückstand wird in Dichlormethan (400 mL) gelöst, unter Rühren mit Hexan (4 L) versetzt, der nach weiteren 10 min Rühren entstandene Niederschlag abgenutscht und mit Hexan gewaschen. Das Filtrat wird am Rotationsverdampfer bei 40 °C unter vermindertem Druck soweit eingeengt bis Kristallisation einsetzt. Die erhaltene Suspension läßt man unter Kühlen im Eisbad ausrühren, filtriert den Niederschlag ab und wäscht mit wenig kaltem Hexan nach. Nach dem Trocknen im Hochvakuum bei 30 °C erhält man die reine Titelverbindung als weißen kristallinen Festkörper (230 g). Aus der Mutterlauge erhält man durch erneutes Umkristallisieren aus Dichlormethan/Hexan weitere 61 g (76 % Gesamtausbeute) der Titelverbindung. Schmp. 82-83 °C. Anal. C₈H₁₄N₂O₂ (170.19): ber. C, 56.45; H, 8.29; N, 16.45; gef. C, 56.42; H, 8.47; N, 16.50.

### b) 2(S)-{2(S)-[4-Methoxy-3-(3-methoxypropoxy)-benzyl]-3-methylbutyl}-3,6-diethoxy-2,5-dihydro-pyrazin

Die Lösung von 3,6-Diethoxy-2,5-dihydro-pyrazin (229.8 g, 1.35 Mol) in Tetrahydrofuran (3.0 L) wird unter inerter Atmosphäre bei -40 °C tropfenweise mit 1.6 M n-Butyllithium in Hexan (788 mL, 1.26 Mol) während 20 min versetzt. Der Ansatz wird weitere 15 min bei -40 °C gerührt und anschließend eine Lösung von 2(R)-[4-Methoxy-3-(3-methoxypropoxy)-benzyl]-3-methyl-butylbromid (323.4 g, 0.90 Mol) in Tetrahydrofuran (1.0 L) während 20 min zugetropft. Nach beendeter Addition läßt man auf -20 °C aufwärmen und rührt über 18 h bei dieser Temperatur nach. Der Reaktionsansatz wird eingeengt und der Rückstand zwischen Essigsäureethylester (1 L) und Wasser (1 L) verteilt. Die organische Phase wird noch mit 2 x 1 L Wasser gewaschen, die wässerigen Phasen mit jeweils 1 L Essigsäureethylester rückextrahiert und die vereinten organischen Phasen mit gesättigter natriumchloridlösung (1 L) gewaschen und über Magnesiumsulfat getrocknet. Nach dem Einengen wird der Rückstand im Hochvakuum über 1 h bei 35 °C getrocknet. Man erhält die rohe Titelverbindung im Gemisch mit einem geringen Anteil an epimerem 2(R)-{2(S)-[4-Methoxy-3-(3-methoxypropoxy)-benzyl]-3-methylbutyl}-3,6-diethoxy-2,5-dihydro-pyrazin (ca. 95:5 Diastereomerengemisch) sowie überschüssigem 3,6-Diethoxy-2,5-dihydro-pyrazin als farbloses Öl (472 g): R_{f} (Essigsäureethylester/Hexan 1:2) = 0.20. Das als Ausgangsmaterial verwendete 2(R)-[4-Methoxy-3-(3-methoxypropoxy)-benzyl]-3-methyl-butylbromid kann beispielsweise wie nachfolgend beschrieben hergestellt werden:

### a1) 2(R)-[4-Methoxy-3-(3-methoxypropoxy)-benzyl]-3-methyl-butylbromid

Zu einer Lösung von 2(R)-[4-Methoxy-3-(3-methoxypropoxy)-benzyl]-3-methyl-butanol (102.2 g) in Dichlormethan (2L) werden bei 0 °C nacheinander unter Rühren Triphenylphosphin (108.6 g) und portionsweise N-Bromsuccinimid (73.7 g) addiert. Der Ansatz wird über weitere 18 h bei Raumtemperatur gerührt und anschließend unter vermindertem Druck eingeengt. Der Rückstand wird mittels FC an Kieselgel (Essigsäureethylester/Hexan 1:4) gereinigt. Man erhält die Titelverbindung (102.2 g) als weißen Festkörper: Schmp. 52-53 °C (umkristallisiert aus Diethylether/Hexan). R_{f} (Essigsäureethylester/Hexan 1:1) = 0.56. Anal. C₁₇H₂₇BrO₃ (359.30): ber. C, 56.83; H, 7.57; Br, 22.24; gef. C, 56.81; H, 7.34; Br, 22.06.

### b1) 2(R)-[4-Methoxy-3-(3-methoxypropoxy)-benzyl]-3-methyl-butanol

Eine Lösung von 2(R)-[4-Methoxy-3-(3-methoxypropoxy)-benzyl]-3-methyl-buttersäure (186 g) in Tetrahydrofuran (500 mL) wird bei Raumtemperatur langsam zu einer Suspension von Natriumborhydrid (27.2 g) in Tetrahydrofuran (1.5 L) addiert. Die Mischung wird bis zur Beendigung der Gasentwicklung gerührt (ca. 45 min), anschließend wird eine Lösung von Jod (76.2 g) in Tetrahydrofuran (1.0 L) langsam addiert und der Reaktionsansatz während weiterer 4 Tage bei Raumtemperatur gerührt. Methanol (1.0 L) wird vorsichtig über 20 min zugetropft und die Mischung nach weiteren 30 min Rühren im Vakuum eingeengt. Der Rückstand wird mit Essigsäureethylester (3 x 2 L) extrahiert und die vereinten organischen Phasen nacheinander mit jeweils 2 L 2N Salzsäure, Wasser, gesättigter Natriumthiosulfat-Lösung, Wasser, 0.1 N Natronlauge (1 L) und gesättigter Kochsalzlösung gewaschen, über Magnesiumsulfat getrocknet und eingeengt. Der Rückstand wird durch FC an Kieselgel (Essigsäureethylester/Hexan 1:1) gereinigt, und man erhält die Titelverbindung als Öl (160 g): R_{f} (Essigsäureethylester/Hexan 1:1) = 0.28. Anal. C₁₇H₂₈O₄ (296.41): ber. C, 68.89; H, 9.52; gef. C, 68.34; H, 9.70.

### c1) 2(R)-[4-Methoxy-3-(3-methoxypropoxy)-benzyl]-3-methyl-buttersäure

Zu einer Lösung von 4(R)-Benzyl-3-{2(R)-[4-methoxy-3-(3-methoxypropoxy)-benzyl]-3-methyl-butyryl}-oxazolidin-2-on (300 g) in Tetrahydrofuran/Wasser 3:1 (4.8 L) werden unter Eiskühlung eine 30 %-ige Wasserstoffperoxidlösung (434 mL) und Lithiumhydroxid 98 % (31.2 g) addiert. Nach beendeter Zugabe läßt man den Reaktionsansatz über 3 h auf Raumtemperatur aufwärmen, kühlt erneut auf 0 °C ab und addiert tropfenweise über 30 min eine 1.5 M wässerige Natriumsulfitlösung (2.55 L) und anschließend eine gesättigte wässerige Natriumhydrogencarbonatlösung (1 L). Die Mischung wird unter vermindertem Druck aufkonzentriert und die so erhaltene wässerige Lösung mit Dichlormethan gewaschen (3 x 3 L). Die wässerige Phase wird durch Zugabe von 2 N Salzsäure auf pH 3 gebracht, mit Dichlormethan (3 x 3 L) extrahiert, die vereinten organischen Phasen über Magnesiumsulfat getrocknet und eingeengt. Man erhält die Titelverbindung als Öl (186.8 g), das in Hexan/Diethylether bei -20 °C zur Kristallisation gebracht werden kann: Schmp. 43.5-44 °C. R_{f} (Essigsäureethylester/Hexan 2:1) = 0.30. Anal. C₁₇H₂₆O₅ (310.39): ber. C, 65.78; H, 8.44; gef. C, 65.96; H, 8.70.

### d1) 4(R)-Benzyl-3-{2(R)-[4-methoxy-3-(3-methoxypropoxy)-benzyl]-3-methyl-butyryl}-oxazolidin-2-on

Zu einer 1 M Lithiumhexamethyldisilazid-Lösung in Tetrahydrofuran (600 mL, 0.60 Mol), verdünnt mit wasserfreiem Tetrahydrofuran (600 mL), wird unter inerter Atmosphäre bei -70 °C eine Lösung von 4(R)-Benzyl-3-isovaleroyl-oxazolidin-2-on (156.6 g) in Tetrahydrofuran (500 mL) addiert und der Ansatz für weitere 75 min bei -70 °C gerührt. Anschließend wird eine Lösung von 4-Methoxy-3-(3-methoxypropoxy)-benzylbromid (145 g) in Tetrahydrofuran (500 mL) addiert. Man läßt die Reaktionstemperatur über einen Zeitraum von 2 h von -70 °C auf 0 °C aufwärmen und rührt weitere 18 h bei 0 °C. Die Reaktion wird durch Zugabe einer 10 %-igen wässerigen Ammoniumchloridlösung (250 mL) gequencht, die Mischung unter vermindertem Druck eingeengt und die wässerige Phase mit Essigsäureethylester (3 x 1.2 L) extrahiert. Die organischen Phasen werden mit gesättigter Natriumchloridlösung gewaschen, über Magnesiumsulfat getrocknet und eingeengt. Reinigung des Rückstandes mittels FC an Kieselgel (Hexan/Essigsäureethylester 1:1) ergibt die Titelverbindung als weißen Festkörper (202 g): Schmp. 55-56 °C (umkristallisiert aus Diethylether/Hexan). R_{f} (Essigsäureethylester/Hexan 1:2) = 0.30. Anal. C₂₇H₃₅NO₆ (469.58): ber. C, 69.06; H, 7.51; N, 2.98; gef. C, 68.64; H, 7.49; N, 3.12.

### e1) 4-Methoxy-3-(3-methoxypropoxy)-benzylbromid

Zu einer Lösung von 4-Methoxy-3-(3-methoxypropoxy)-benzylalkohol (111.1 g) in Chloroform (1.31 L) wird tropfenweise Trimethylsilylbromid (97 mL) addiert, wobei die Reaktionstemperatur durch Eiskühlung auf 10-25 °C gehalten wird. Nach beendeter Zugabe rührt man weitere 10 min bei Raumtemperatur und engt die Mischung unter vermindertem Druck ein. Der Rückstand wird mittels FC an Kieselgel (Essigsäureethylester/Hexan 1:3) gereinigt, und man erhält die Titelverbindung (144.5 g) nach Umkristallisation aus Hexan als weißen kristallinen Festkörper: Schmp. 50-51 °C. R_{f} (Essigsäureethylester/Hexan 1:2) = 0.34

### f1) 4-Methoxy-3-(3-methoxypropoxy)-benzylalkohol

Zu einer Lösung von 4-Methoxy-3-(3-methoxypropoxy)-benzaldehyd (336 g) in Methanol (3.36 L) wird Natriumborhydrid (39.7 g) in Portionen addiert, wobei die Reaktionstemperatur bei 0-5 °C gehalten wird. Nach beendeter Zugabe rührt man den Ansatz weitere 60 min bei Raumtemperatur, engt unter vermindertem Druck ein und verteilt den Rückstand zwischen eisgekühlter 2 N Salzsäure und Essigsäureethylester (3 x 2 L). Die vereinten organischen Phasen werden mit Wasser und gesättigter natriumhydrogencarbonatlösung gewaschen, über Magnesiumsulfat getrocknet und eingeengt. Nach Reinigung des Rückstandes durch FC an Kieselgel (Dichlormethan/Methanol 96:4) erhält man die Titelverbindung als Öl (326 g): R_{f} (Essigsäureethylester/Hexan 2:1) = 0.31. Anal. C₁₂H₁₈O₄ (226.27): ber. C, 63.70; H, 8.02; gef. C, 63.70; H, 8.24.

### g1) 4-Methoxy-3-(3-methoxypropoxy)-benzaldehyd

Zu einer Lösung von 4-Methoxy-3-(3-brompropoxy)-benzaldehyd (844 g) in Methanol (4.15 L) wird bei 61-64 °C eine 30 %-ige Lösung von Natriummethanolat in Methanol (0.86 L, 4.64 Mol) tropfenweise addiert. Der Ansatz wird weitere 1.5 h unter Rückfluß gerührt, auf Raumtemperatur abkühlen gelassen und mit Wasser (150 mL) versetzt. Man entfernt das Methanol am Rotationsverdampfer (30 °C Badtemperatur) und verteilt anschließend den Rückstand zwischen Diethylether (3 x 3 L) und eiskalter 4 N Salzsäure. Die vereinten organischen Phasen werden mit Wasser und gesättigter Natriumhydrogencarbonatlösung gewaschen, über Magnesiumsulfat getrocknet und eingeengt. Den Rückstand reinigt man durch FC an Kieselgel (Essigsäureethylester/Hexan 1:3) und erhält die Titelverbindung als Öl (498 g): R_{f} (Essigsäureethylester/Hexan 1:2) = 0.18.

### h1) 4-Methoxy-3-(3-brompropoxy)-benzaldehyd

Zu einer Lösung von 3-Hydroxy-4-methoxy-benzaldehyd (200 g) in Acetonitril werden Kaliumcarbonat (272.3 g) und 1,3-Dibrompropan (1.34 L) addiert und die weiße Suspension über 19 h unter Rückfluß gerührt. Nach dem Abkühlen filtriert man, entfernt das Lösungsmittel und überschüssiges 1,3-Dibrompropan unter vermindertem Druck und reinigt den Rückstand mittels FC an Kieselgel (Essigsäureethylester/Hexan 1:3). Man erhält die Titelverbindung als weißen Festkörper (334 g): R_{f} (Essigsäureethylester/Hexan 1:1) = 0.50.

Anal. C₁₁H₁₃BrO₃ (273.13): ber. C, 48.37; H, 4.80; Br, 29.26; gef. C, 48.61; H, 4.84; Br, 29.19.

### Herstellungsbeispiel 2: 2(S)-(tert-Butoxycarbonyl)amino-4(S)-[4-methoxy-3-(3-methoxy-propoxy)-benzyl]-5-methyl-hexansäureethylester

Die Lösung des Rohproduktes aus Beispiel 1) (351 g) in Dichlormethan (2.7 L) wird unter Rühren bei 0 °C mit Ethyldiisopropylamin (200 mL, 1.17 Mol) und einer Lösung von Di-*tert*-Butyldicarbonat (216 g, 0.99 Mol) in Dichlormethan (0.3 L) versetzt. Die Mischung wird während 18 h bei Raumtemperatur nachgerührt, anschließend eingeengt und der Rückstand an Kieselgel (Fließmittelgradient Essigsäureethylester/Hexan von 1:4 nach 1:2) chromatographiert. Das so erhaltene Rohprodukt (428 g; ca. 95:5 Verhältnis der Titelverbindung zum epimeren 2(R)-(*tert*-Butoxycamonyl)amino-4(S)-[4-methoxy-3-(3-methoxypropoxy)-benzyl]-5-methyl-hexansäureethylester) wird aus Hexan umkristallisiert. Man erhält die Titelverbindung als weißen kristallinen Festkörper (353 g, 81 % Ausbeute über 3 Stufen): R_{f} (1:1 Essigsäureethylester/Hexan) = 0.47. Schmp. 59-60 °C. IR (CH₂Cl₂) 3435, 2960,1710,1590,1515 1515 cm⁻¹. [α]²⁵D = +8.1 ± 1.0 (*c* 1, CH₂Cl₂).¹ H NMR (DMSO-d₆ bei 80 °C) δ 0.75-0.9 (m, 6H), 1.18 (t, *J* = 7 Hz, 3H), 1.39 (s, 9H), 1.5-1.7 (m, 4H),1.93 (m, 2H), 2.45 (d, *J* = 6 Hz, 2H), 3.27 (s, 3H), 3.49 (t, *J* = 6.4 Hz, 2H), 3.74 (s, 3H), 4.00 (t, *J* = 6.4 Hz, 2H), 4.0-4.1 (m, 3H), 6.66 (bs, 1H), 6.68 (dd, *J* = 2, 8 Hz,1H), 6.75 (d, *J* = 2 Hz, 1 H), 6.84 (d, *J* = 8 Hz,1H) ppm. Anal. C₂₆H₄₃NO₇ (481.63): ber. C, 64.84; H, 9.00; N, 2.91; gef. C, 65.00; H, 9.16; N, 3.04. Die Diastereomerenreinheit (>99 % de) wurde durch Hochdruckflüssigkeitschromatographie (HPLC) an einer Nudeosil® 5 C18 Reversed Phase Säule mit einem Fließmittelgradienten 20-100 % B in 35 min (A = Wasser/0.1 %
Trifluoressigsäure; B = Acetonitril/0.1 % Trifluoressigsäure) bestimmt.

### Herstellungsbeispiel 3:

In analoger Weise wie in Herstellungsbeispiel 2) beschrieben, können die nachfolgenden Verbindungen hergestellt werden:
2(S)-(*tert*-Butoxycarbonyl)amino-4(S)-(p-*tert*-butylphenyl)-5-methyl-hexansäureethylester;
2(S)-(*tert*-Butoxycarbonyl)amino-4(R)-(p-*tert*-butylbenzyl)-hexansäureethylester;
2(S)-(*tert*-Butoxycarbonyl)amino-4(S)-[4-*tert*-butyl-3-(3-methoxypropoxy)-benzyl]-5-methylhexansäureethylester;
2(S)-(*tert*-Butoxycarbonyl)amino-4(S)-[3-benzyloxy-4,5-ethylendioxy-benzyl]-5-methylhexansäureethylester;
2(S)-(*tert*-Butoxycarbonyl)amino-4(S)-[4-ethyl-3-(3-methoxypropoxy)-benzyl]-5-methylhexansäureethylester;
2(S)-(*tert*-Butoxycarbonyl)amino-4(S)-[3-benzyloxy-4-methoxy-benzyl]-5-methyl-hexansäureethylester;
2(S)-(*tert*-Butoxycarbonyl)amino-4(S)-[4-benzyloxy-3-(3-methoxy-propoxy)-benzyl]-5-methylhexansäureethylester.

Die Ausgangsmaterialien können beispielsweise analog wie in den Herstellungsbeispielen 1) und 1b) beschrieben, ausgehend von den im Herstellungsbeispiel 5) beschriebenen Zwischenprodukten, hergestellt werden.

### Herstellungsbeispiel 4: 2(S)-(tert-Butoxycarbonyl)amino-4(S)-benzyloxymethyl-5-methyl-hexansäureethylester

Die Mischung aus 2(S)-[2(S)-Benzyloxymethyl-3-methylbutyl]-3,6-diethoxy-2,5-dihydropyrazin (1.20 g, 3.33 mmol), Acetonitril (12 mL) und 1 N Salzsäure (12 mL) wird bei Raumtemperatur während 20 min gerührt und anschliessend in eine gesättigte Natriumhydrogencarbonat-Lösung (50 mL) gegossen. Man extrahiert mit Dichlormethan (3 x 60 mL), wäscht die vereinten organischen Phasen mit Wasser (3 x 100 mL) und engt auf ein Volumen von 15 mL ein. Die so erhaltene Lösung wird auf 0 °C gekühlt und mit Ethyldiisopropylamin (0.73 mL, 4.29 mmol) und einer Lösung von Di-*tert*-Butyldicarbonat (0.79 g, 3.63 mmol) in Dichlormethan (2 mL) versetzt. Der Ansatz wird während 16 h bei Raumtemperatur gerührt und nach weitgehendem Abdampfen des Lösungsmittels der Rückstand mittels Flash-Chromatographie (100 g Kieselgel, 1:6 Essigsäureethylester/Hexan) gereinigt. Man erhält die Titelverbindung im Gemisch mit dem epimeren 2(R)-(*tert*-Butoxycarbonyl)amino-4(S)-benzyloxymethyl-5-methylhexansäureethylester (1.23 g, 94 %; ca. 2:1 Diastereomeren-Verhältnis) als farbloses Oel: R_{f} (Essigsäureethylester/Hexan 1:4) = 0.34. ¹H-NMR (CDCl₃):δ 0.85-0.9 (m, 6H), 1.25-1.3 (m, 3H), 1.42 (s, 9H), 1.55-1.9 (m, 4H), 3.25-3.5 (m, 2H), 4.1-4.35 (m, 3H), 4.45-4.55 (m, 2H), 5.35 (d, 0.33H), 5.43 (d, 0.67H), 7.25-7.4 (m, 5H) ppm. Anal. C₂₂H₃₅NO₅ (393.52): ber. C, 67.15; H, 8.96; N, 3.56; gef. C, 66.88; H, 8.67; N, 3.50.

Das Ausgangsmaterial kann beispielsweise folgendermaßen hergestellt werden:

### a) 2(S)-[2(S)-Benzyloxymethyl-3-methylbutyl]-3,6-diethoxy-2,5-dihydro-pyrazin

Zur Lösung von 3,6-Diethoxy-2,5-dihydro-pyrazin (1.02 g, 6.0 mmol) in Tetrahydrofuran wird unter inerter Atmosphäre bei -40 °C 1.6 M n-Butyllithium in Hexan (3.5 mL, 5.6 mmol) zugetropft. Nach 15 min Rühren wird eine Lösung von 2(S)-Benzyloxymethyl-3-methylbenzylbromid (1.09 g, 4.0 mmol) in Tetrahydrofuran (5 mL) bei -40 °C zugetropft und anschliessend das Reaktionsgemisch während 16 h bei -18 °C stehen gelassen. Der Ansatz wird eingeengt und der Rückstand zwischen Essigsäureethylester (30 mL) und Wasser (30 mL) verteilt. Die wässerige Phase wird mit Essigsäureethylester (2 x 30 mL) extrahiert und die vereinten organischen Phasen mit gesättigter Natriumchlorid-Lösung gewaschen, über Magnesiumsulfat getrocknet und eingeengt. Das Rohprodukt wird durch Flash-Chromatographie (100 g Kieselgel, 1:6 Essigsäureethylester/Hexan) gereinigt. Man erhält die Titelverbindung (1.28 g, 89 %) im Gemisch mit dem Diastereomeren 2(R)-[2(S)-Benzyloxymethyl-3-methylbutyl]-3,6-diethoxy-2,5-dihydro-pyrazin (ca. 2:1 Diastereomeren-Verhältnis aufgrund ¹H-NMR-Spektrum) als blassgelbes Oel: R_{f} (Essigsäureethylester/Hexan 1:4) = 0.32. ¹H-NMR (CDCl₃): δ 0.75-0.9 (m, 6H), 1.2-1.35 (m, 6H), 1.4-2.05 (m, 4H), 3.35-3.55 (m, 2H), 3.95-4.15 (m, 7H), 4.47 (s, 0.34H), 4.50 (s, 0.66H), 7.25-7.4 (m, 5H) ppm.

### Herstellungsbeispiel 5: 1-Brom-2(R)-isopropyl-3-(p-tert-butyl-phenyl)-propan

Die Lösung von 2(R)-(p-*tert*-Butyl-benzyl)-3-methyl-butanol (2.3 g) in Dichlormethan (50 mL) wird unter Rühren bei 0° C mit Triphenylphosphin (3.15 g) und anschliessend portionen-weise mit N-Bromsuccinimid (2.14 g) versetzt. Danach wird das Reaktionsgemisch 16 h bei Raumtemperatur gerührt und eingeengt. Aus dem Rückstand erhält man durch FC-Reinigung (100 g Kieselgel, Laufmittel Dichlormethan-/Hexan 1:1) die Titelverbindung: R_{f} (Hexan) = 0.49.

Die Ausgangsmaterialien können beispielsweise wie folgt hergestellt werden:

### a) 2(R)-(p-tert-Butyl-benzyl)-3-methyl-butanol

Die Suspension von Lithiumaluminiumhydrid (2.41 g) in Tetrahydrofuran (160 mL) wird unter Rühren bei 0° C mit einer Lösung von 4(R)-Benzyl-3-[2(R)-(p-*tert*-butyl-benzyl)-3-methyl-butyryl]-oxazolidin-2-on (8.63 g) in Tetrahydrofuran (40 mL) tropfenweise versetzt. Das Reaktionsgemisch wird 4 h bei 0° C gerührt und dann bei 0° C nacheinander mit Essigsäureethylester (5 mL), 30 ml eines (1:1 )-Gemisches aus Tetrahydrofuran/Wasser und 2 N Schwefelsäure (80 mL) versetzt. Die Suspen-sion wird mit Essigsäureethylester extrahiert und das Rohprodukt mittels FC (700 g Kieselgel, Laufmittel Dichlormethan) gereinigt. Man erhält die Titelverbindung: R_{f}(Dichlormethan) = 0.34; Schmp. 49-51 °C.

### b) 4(R)-Benzyl-3-[2(R)-(p-tert-butyl-benzyl)-3-methyl-butyryl]-oxazolidin-2-on

Unter inerter Atmosphäre wird zu einer 1 M Lithiumhexamethyldisilazid-Lösung in Tetrahydrofuran (31 mL), verdünnt mit Tetrahydrofuran (30 mL), unter Rühren bei -70 °C eine Lösung von 4(R)-Benzyl-3-isovaleroyl-oxazolidin-2-on (13.2 g) in Tetrahydrofuran (20 mL) tropfenweise addiert und das Reaktionsgemisch 1 h bei -70 °C gerührt. Danach wird eine Lösung von p-*tert*-Butyl-benzylbromid (9.6 g) in Tetrahydrofuran (20 mL) zugetropft und eine weitere Stunde bei -25 °C und 4 h bei 0 °C gerührt. Das Reaktionsgemisch wird mit 6 mL einer gesät-tigten Ammoniumchlorid-Lösung versetzt, durch Einengen unter reduziertem Druck vom Tetrahydrofuran befreit und mit Diethyl-ether extrahiert. Aus dem Rückstand des Extraktes erhält man durch Reinigen mittels FC (700 g Kieselgel, Laufmittel Dichlormethan/-Hexan 1:1) die Titelverbindung: R_{f}(Dichlormethan/-Hexan 1:1) = 0.30; Schmp. 123.5-124 °C.

In analoger Weise kann man auch die folgenden Verbindungen der Formel XV herstellen:
1-Brom-2(S)-ethyl-3-(p-*tert*-butylphenyl)-propan;
1-Brom-2(R)-isopropyl-3-(p-*tert*-butylphenyl)-propan;
1-Brom-2(R)-isopropyl-3-[3-benzyloxy-4,5-ethylendioxy-phenyl]-propan;
1-Brom-2(R)-isopropyl-3-[4-benzyloxy-3-(3-methoxy-propoxy)-phenyl]-propan;
1-Brom-2(R)-isopropyl-3-[4-*tert*-butyl-3-(3-methoxy-propoxy)-phenyl]-propan;
1-Brom-2(R)-isopropyl-3-[4-ethyl-3-(3-methoxy-propoxy)-phenyl]-propan;
1-Brom-2(R)-isopropyl-3-[4-methoxy-3-benzyloxy-phenyl]-propan.

### Herstellungsbeispiel 6: 2(S)-(tert-Butoxycarbonyl)amino-4(S)-[4-methoxy-3-(3-methoxypropoxy)-benzyl]-5-methyl-hexan-1-ol

Zur Lösung des Produktes aus Herstellungsbeispiel 2) (28.9 g, 60 mmol) in Tetrahydrofuran (430 mL) wird bei Raumtemperatur Lithiumborhydrid (3.0 g, 138 mmol) in einer Portion addiert und der Ansatz über weitere 16 h gerührt. Anschliessend versetzt man tropfenweise mit Methanol, engt die Mischung am Rotavap (40 °C Badtemperatur) ein und versetzt den Rückstand mit 400 mL einer Mischung aus 1 N Salzsäure und Eis. Man extrahiert mit Dichlormethan (3 x 400 mL), trocknet die vereinten organischen Phasen über Magnesiumsulfat und engt ein. Umkristallisation des Rohproduktes aus Diethylether/Hexan ergibt die Titelverbindung als weissen kristallinen Festkörper (25.2 g, 95%): Schmp. 66-67 °C. R_{f} (Essigsäureethylester/Hexan 1:1) = 0.19. IR (CH₂Cl₂) 3435, 2960, 1710, 1590, 1515,1465 cm⁻¹ [α]²⁵D = +1.8 ± 1.0 (*c* 1, CH₂Cl₂).¹H NMR (DMSO-d₆ bei 80°C): δ 0.65-0.8 (m, 6H), 1.25-1.4 (m, 2H), 1.41 (s, 9H), 1.6-1.65 (m, 2H), 1.93 (m, 2H), 2.33 (dd, J = 9,14 Hz,1H), 2.60 (dd, J = 6,14 Hz, 1H), 3.26 (s, 3H), 3.25-3.35 (m, 2H), 3.49 (t, *J* = 6.4 Hz, 2H), 3.35-3.55 (m, 1H). 3.74 (s, 3H), 4,01 (t, *J* = 6.4 Hz, 2H), 4.19 (t, *J* = 6 Hz, 1H), 5.98 (br d, 1H), 6.70 (dd, *J* = 2, 8 Hz, 1H), 6.80 (d, *J* = 2 Hz, 1H), 6.82 (d, *J* = 8 Hz, 1H) ppm. Anal. C₂₄H₄₁NO₆ (439.59): ber. C, 65.58; H, 9.40; N, 3.19; gef. C, 65.52; H, 9.19; N, 3.21.

### Herstellungsbeispiel 7: 2(S)-(tert-Butoxycarbonyl)amino-4(S)-[4-methoxy-3-(3-methoxy-propoxy)-benzyl]-5-methyl-hexanal

Zu einer Lösung von 2(S)-(*tert*-Butoxycarbonyl)amino-4(S)-[4-methoxy-3-(3-methoxypropoxy)-benzyl]-5-methyl-hexanol (184.8 g) in Dichlormethan (1.34 L) wird bei 0 °C zunächst Triethylamin (175.6 mL) und dann eine Lösung von Pyridin-Schwefeltrioxid-Komplex (221 g) in Dimethylsulfat (0.84 L) tropfenweise während 45 min unter Rühren addiert. Nach beendeter Zugabe wird noch 30 min bei 0 °C gerührt, und dann Eiswasser (1.6 L) addiert. Man extrahiert mit Dichlormethan (3 x 1.6 L), wäscht die vereinten organischen Phasen mit gesättigter Natriumhydrogencarbonat-Lösung (2 x 1.6 L), Wasser (1.6 L) und gesättigter Natriumchlorid-Lösung, filtriert durch Baumwolle und entfernt das Lösungsmittel im Vakuum. Nach dem Trocknen des Rückstandes im HV erhält man die rohe Titelverbindung als Oel (190 g): R_{f} (Essigsäureethylester/Hexan 1:1) = 0.44. IR (CH₂Cl₂) 3430, 2960, 1710, 1589,1515 cm⁻¹. ¹H-NMR (CDCl₃): δ 0.86 (m, 6H), 1.44 (s, 9H), 1.55-1.8 (m, 4H), 2.09 (m, 2H), 2.4-2.7 (m, 2H), 3.35 (s, 3H), 3.57 (t, J = 6.4 Hz, 2H), 3.83 (s, 3H), 4.10 (t, J = 6.4 Hz, 2H), 4.05-4.20 (m, 1H), 4.89 (d, br., 1H), 6.7-6.8 (m, 3H), 9.50 (s, 1H).

### Herstellungsbeispiel 8: 2(S)-(tert-Butoxycarbonyl)amino-4(S)-(p-tert-butyl-benzyl)-5-methylhexanal

Zu einer Lösung von 2(S)-(*tert*-Butoxycarbonyl)amino-4(S)-(p-*tert*-butyl-benzyl)-5-methylhexansäureethylester (1 g) in Toluol (20 ml) wird bei -75° C eine 1.2 M Diiso-butylaluminium-hydrid-Lösung in Toluol (4.2 mL) langsam zugetropft. Das Reaktionsgemisch wird 30 min bei -70° C gerührt, dann mit 10 mL Methanol versetzt, auf ein Gemisch von Eis und 1 N Salzsäure (10 mL) gegossen und mit Essigsäureethylester extrahiert. Man erhält die Titelverbindung: R_{f}(Dichlormethan) = 0.35.

Ebenso können die nachfolgenden Verbindungen hergestellt werden:
2(S)-(*tert*-Butoxycarbonyl)amino-4(R)-(p-*tert*-butyl-benzyl)-hexanal;
2(S)-(*tert*-Butoxycarbonyl)amino-4(S)-[4-*tert*-butyl-3-(3-methoxypropoxy)-benzyl]-5-methyl-hexanal;
2(S)-(*tert*-Butoxycarbonyl)amino-4(S)-[3-benzyloxy-4,5-ethylendioxy-benzyl]-5-methyl-hexanal;
2(S)-(*tert*-Butoxycarbonyl)amino-4(S)-[4-ethyl-3-(3-methoxypropoxy)-benzyl]-5-methyl-hexanal;
2(S)-(*tert*-Butoxycarbonyl)amino-4(S)-[4-methoxy-3-benzyloxy-benzyl]-5-methyl-hexanal;
2(S)-(*tert*-Butoxycarbonyl)amino-4(S)-[4-benzyloxy-3-(3-methoxy-propoxy)-benzyl]-5-methylhexanal;

### Verwendungsbeispiel 1: 5(S)-Amino-4(S)-hydroxy-2(S)-isopropyl-7(S)-[4-methoxy-3-(3-methoxy-propoxy)-benzyl]-8-methyl-n-onansäure-N-(2-morpholin-4-yl-ethyl)amiddihydrochlorid

3.09 g 5(S)-(*tert*-Butoxycarbonyl)amino-4(S)-hydroxy-2(S)-isopropyl-7(S)-[4-methoxy-3-(3- methoxy-propoxy)-benzyl]-8-methyl-n-onansäure-N-(2-morpholin-4-yl-ethyl)amid werden in 4 N Salzsäure in Dioxan (40 mL) bei 0° C gelöst und für 2 h bei dieser Temperatur gerührt. Das Reaktionsgemisch wird lyophilisiert und man erhält die Titelverbindung: R_{f} (Dichlormethan/-Methanol 8:2) = 0.27; HPLC Rₜ = 9.52 min; FAB-MS (M+H)⁺ = 566.

Die Ausgangsmaterialien werden wie folgt hergestellt:

### a) 5(S)-(tert-Butoxycarbonyl)amino-4(S)-hydroxy-2(S)-isopropyl-7(S)-[4-methoxy-3-(3-methoxy-propoxy)-benzyl]-8-methyl-n-onansäure-N-(2-morpholin-4-yl-ethyl) amid

Die Mischung aus N-(*tert*-Butoxycarbonyl)amino-2(S)-{4(S)-[2(S)-[4-methoxy-3-(3-methoxy-propoxy)-benzyl]-3-methyl-butyl]-2,2-dimethyl- oxazolidin-5(S)-ylmethyl}-3-methyl-N-(2-morpholin-4-yl-ethyl)-butyramid (4.18 g) und p-Toluolsulfonsäure-Monohydrat (1.30 g) in Methanol (160 mL) wird 1 h bei 0° C und weitere 18 h bei Raumtemperatur gerührt. Nach Entfernen des Lösungsmittels wird der Rückstand mit 0.1 N Natriumhydroxid (200 mL) versetzt und mit Dichlormethan extrahiert. Nach Einengen der organischen Phasen wird das Rohprodukt durch FC (230 g Kieselgel, Dichlormethan/-Methanol 95:5) gereinigt. Man erhält die Titelverbindung: R_{f} (Dichlormethan-/Methanol 9:1) = 0.55.

### b) N-(tert-Butoxycarbonyl)amino-2(S)-{4(S)-[2(S)-[4-methoxy-3-(3-methoxypropoxy)-benzyl]-3-methyl-butyl]-2,2-dimethyl-oxazolidin-5(S)- ylmethyl}-3-methyl-N-(2-morpholin-4-yl-ethyl)-butyramid

Eine Lösung von N-(*tert*-Butoxycarbonyl)amino-2(S)-{4(S)-[2(S)-[4-methoxy-3-(3-methoxy-propoxy)-benzyl]-3-methyl-butyl]-2,2-dimethyl-oxazolidin-5(S)-ylmethyl}-3-methyl-buttersäure (3.88 g) in N,N-Dimethylformamid (190 mL) wird unter Rühren bei 0° C nacheinander mit Triethylamin (1.09 mL), 4-(2-Aminoethyl)-morpholin (1.02 mL) und Cyanphosphon-säure-diethylester (1.19 mL) versetzt. Der Ansatz wird 18 h bei Raumtemperatur gerührt, anschliessend unter vermindertem Druck eingeengt und der Rückstand zwischen Diethylether und gesättigter Natriumhydrogencarbonat-Lösung verteilt. Die organischen Phasen werden mit gesättigter Kochsalzlösung gewaschen und eingedampft. Der Eindampfrückstand wird durch FC (230 g Kieselgel, Dichlormethan/Methanol 95:5) gereinigt und man erhält die Titelverbindung: R_{f} (Dichlormethan-/Methanol 95:5) = 0.25.

### c) N-(tert-Butoxycarbonyl)amino-2(S)-{4(S)-[2(S)-[4-methoxy-3-(3-methoxypropoxy)-benzyl]-3-methyl-butyl]-2-2-dimethyl-oxazolidin-5(S)-ylmethyl}-3-methyl-buttersäure

Zu einer Lösung von N-(*tert*-Butoxycarbonyl)amino-2(S)-{4(S)-[2(S)-[4-methoxy-3-(3-methoxypropoxy)-benzyl]-3-methyl-butyl]-2,2-dimethyl-oxazolidin-5(S)-ylmethyl}-3-methylbutyraldehyd (53.0 g) in Toluol (470 mL) werden bei 0° C unter Rühren nacheinander Wasser (470 mL), Kaliumpermanganat (79.1 g) und Tetrabutylammoniumbromid (9.7 g) addiert. Das Reaktionsgemisch wird 48 h bei 0-5° C gerührt und anschliessend bei 10° C mit einer Natriumsulfit-Lösung 10% (1.2 L) und nach weiteren 30 min mit CitronensäureLösung 10% (1.95 L) und Wasser (1.2 L) versetzt. Man extrahiert mit Essigsäureethylester (3 x 2.5 L), wäscht die organischen Phasen mit Wasser und gesättigter Natriumchlorid-Lösung, trocknet über Magnesiumsulfat und engt ein. Reinigung des Rohproduktes durch FC (2.3 kg Kieselgel, Essigsäureethylester/-Hexan 3:7) ergibt die reine Titelverbindung als Oel (31.7 g): R_{f} (Essigsäureethylester-/Hexan 1:2) = 0.21; Anal. C₃₃H₅₅NO₈ 593.80): ber. C, 66.75; H, 9.34; N, 2.36; gef. C, 66.69; H, 9.39; N, 2.39.

### d) N-(tert-Butoxycarbonyl)amino-2(S)-{4(S)-[2(S)-[4-methoxy-3-(3-methoxypropoxy)-benzyl]-3-methyl-butyl]-2,2-dimethyl-oxazolidin-5(S)-ylmethyl}-3-methyl-butyraldehyd

Zur Mischung aus N-(*tert*-Butoxycarbonyl)amino-2(S)-{4(S)-[2(S)-[4-methoxy-3-(3-methoxy-propoxy)-benzyl]-3-methyl-butyl]-2,2-dimethyl-oxazolidin-5(S)-ylmethyl}-3-methyl-butan-1-ol (53.0 g), N-Methylmorpholin-N-oxid (16.6 g) und 100 g Molekularsieb (0.3 nm) in Dichlormethan (1.8 L) wird bei Raumtemperatur Tetrapropylammonium-perruthenat (1.60 g) addiert und der Ansatz über 30 min gerührt. Man filtriert ab, verdünnt mit Dichlormethan und wäscht das Filtrat nacheinander mit 2 M Natriumsulfit-Lösung , gesättigter Kochsalzlösung und 1 M Kupfer-(-ll)-sulfat-Lösung. Nach Trocknen über Magnesiumsulfat wird die organische Phase eingeengt und man erhält die Titelverbindung als Rohprodukt: R_{f} (Essigsäureethylester/-Hexan 1:2) = 0.43.

### e) N-(tert-Butoxycarbonyl)amino-2(S)-{4(S)-[2(S)-[4-methoxy-3-(3-methoxypropoxy)-benzyl]-3-methyl-butul]-2,2-dimethyl-oxazolidin-5(S)-ylmethyl}-3-methyl-butan-1-ol

Eine Lösung von N-(*tert*-Butoxycarbonyl)amino-4(S)-{2(S)-[4-methoxy-3-(3-methoxy-propoxy)-benzyl]-3-methyl-butyl}-5(S)-[2(S)-benzyloxymethyl-3-methyl-butyl]-2,2-dimethyloxazolidin (3.7 g) in Tetrahydrofuran (50 mL) wird in Gegenwart von Pd/C 5% (1.0 g) über 15 min bei Raumtemperatur und Normaldruck hydriert. Das Reaktionsgemisch wird filtriert und das Filtrat eingeengt. Der Rückstand wird mittels FC (140 g Kieselgel, Essigsäureethylester/Hexan 1:2) gereinigt und man erhält die Titelverbindung als farbloses Oel (2.92 g): R_{f} (Essigsäureethylester/-Hexan 1:2) = 0.28; Anal. C₃₃H₅₇NO₇ (579.82): ber. C, 68.36; H, 9.91; N, 2.42; gef. C, 67.78; H, 9.82; N, 2.30.

### f) N-(tert-Butoxycarbonyl)amino-4(S)-{2(S)-[4-methoxy-3-(3-methoxypropoxy)-benzyl]-3-methyl-butyl}-5(S)-[2(S)-benzyloxymethyl-3-methyl-butyl]-2,2-dimethyl-oxazolidin (Diastereomer A) und N-(tert-Butoxycarbonyl)amino-4(S)-{2(S)-[4-methoxy-3-(3-methoxypropoxy)-benzyl]-3-methyl-butyl}-5(R)-[2(S)-benzyloxymethyl-3-methyl-butyl]-2,2-dimethyloxazolidin Diastereomer B)

Zu einer Lösung von 3(S)-Benzyloxymethyl-6(S)-(*tert*-butoxycarbonyl)amino-8(S)-[4-methoxy-3-(3-methoxypropoxy)-benzyl]-2,9-dimethyl-decan-5(R,S)-ol (7.0 g) in Dichlormethan (1.86 L) werden bei Raumtemperatur 2,2-Dimethoxypropan (10.9 mL) und p-Toluolsulfonsäure-Monohydrat (10 mg) addiert. Nach Rühren über 24 h bei Raumtemperatur wird der Ansatz eingeengt und der Rückstand durch FC (1 kg Kieselgel, Dichlormethan/Diethylether 96:4) gereinigt. Man erhält die beiden Titelverbindungen jeweils als farbloses Oel. Diastereomer A (3.72 g): R_{f} (Dichlormethan/*tert*-Butylmethylether 95:5) = 0.36. Diastereomer B (2.68 g): R_{f} (Dichlormethan/*tert*.Butylmethylether 95:5) = 0.44

### g) 3(S)-Benzyloxymethyl-6(S)-(tert-butoxycarbonyl)amino-8(S)-[4-methoxy-3-(3-methoxypropoxy)-benzyl]-2,9-dimethyl-decan-5(R,S)-ol

51.1 g Magnesiumspäne werden in Tetrahydrofuran (1.4 L) bei 55° C vorgelegt.

Anschliessend wird während 30 min wird eine Lösung aus 2(S)-Brommethyl-3-methyl-butylbenzylether (380 g) und 1,2-Dibrom-ethan (30.2 ml) in Tetrahydrofuran (0.8 L) bei 55° C zugetropft. Das Reaktionsgemisch wird noch 20 min bei 55° C weitergerührt und anschliessend auf 5° C abgekühlt. Danach wird eine Lösung aus 2(S)-(*tert*-Butoxycarbonyl)amino-4(S)-[4-methoxy-3-(3-methoxypropoxy)-benzyl]-5-methyl-hexanal (190 g) in Tetrahydrofuran (700 mL) zugetropft. Das Reaktionsgemisch wird noch 3 h bei Raumtemperatur gerührt, dann bei 5° C mit gesättigter Ammoniumchlorid-Lösung versetzt und mit Diethylether extrahiert. Die eingeengten organischen Phasen werden durch FC (4 kg Kieselgel, Essigsäureethylester/-Hexan 1:3) gereinigt. Man erhält die Titelverbindung als farbloses Oel (139 g): R_{f} (Essigsäureethylester/-Hexan 1:2) = 0.26; HPLC Rₜ = 22.7 und 22.8 min ((4:6)- Diastereomerengemisch).

### Verwendungsbeispiel 2: 5(S)-Amino-7(S)-(p-tert-butyl--benzyl)-4(S)-hydroxy-2(R,S),8-dimethyl-nonansäure-N(butyl)amid-hydrochlorid

111 mg 5(S)-(tert-Butoxycarbonyl)amino-7(S)-(p-*tert*-butyl--benzyl)-4(S)-hydroxy-2(R,S),8- dimethyl-nonansäure-N(butyl)amid werden in 4 N Salzsäure in Dioxan (2 mL) bei 0°C gelöst und für 60 min bei 20 °C gerührt. Das Reaktionsgemisch wird unter vermindertem Druck eingedampft und der Rückstand mittels FC (50 g Kieselgel, Dichlormethan/-Methanol 9:1) gereinigt. Man erhält die Titelverbindung als Diastereomerengemisch: R_{f} (Dichlormethan/Methanol 9:1) = 0.20; Rₜ(I) = 36.6 und 37.5 min; FAB-MS (M+H)⁺ = 419.

Die Ausgangsmaterialien können beispielsweise folgendermassen hergestellt werden:

### a) 5(S)-(tert-Butoxycarbonyl)amino-7(S)-(p-tert-butyl--benzyl)-4(S)-hydroxy-2(R,S),8-dimethyl-nonansäure-N(butyl)amid

150 mg 2-[3(S)-(*tert*-Butoxycarbonyl)amino-5(S)-(p-*tert*-butyl--benzyl)-2(S)-hydroxy-6-methyl-heptyl]-N-butyl-acrylamid (Diastereomer 1, Verwendungsbeispiel 2b) werden in Gegenwart von 150 mg Pd/C 10% in Tetrahydrofuran (20 mL) für 2 h bei Raumtemperatur und Normaldruck hydriert. Das Reaktions-ge-misch wird filtriert und eingedampft. Der Rückstand wird mittels FC (50 g Kieselgel, Dichlormethan/-Diethyl-ether 8:2) gereinigt. Man erhält die Titelverbindung als Diastereomeren-ge-misch: R_{f}(Dichlormethan/-Diethyl-ether 8:2) = 0.18.

### b) 2-[3(S)-(tert-Butoxycarbonyl)amino-5(S)-(p-tert-butyl--benzyl)-2(S)-hydroxy-6-methyl-heptyl]-N-butyl-acrylamid

695 mg Methacrylsäurebutylamid werden in Tetrahydrofuran (30 mL) gelöst und bei -75°C mit einer1.6 M n-Butyllithium-Lösung in Hexan (6.2 mL) versetzt. Das Reaktionsgemisch wird 30 Min bei 0°C ge-rührt und anschliessend bei -75° mit einer 1 M Chlortitantriisopropoxid-Lösung in Hexan (9.8 L) ver-setzt. Es wird 15 min bei -75 °C weitergerührt und danach bei der gleichen Tempe-ratur die Lösung von 2(S)-(*tert*-Butoxycarbonyl)amino-4(S)-(p-*tert*-butyl-benzyl)-5-methyl-hexanal (924 mg) in Tetrahydrofuran (10 mL) zugetropft. Das Reaktionsgemisch wird 15 min bei -75 °C und 70 min bei 0°C weitergerührt und dann hintereinander mit 10%iger wässriger Citronensäure-lösung (15 mL), Wasser und Diethylether versetzt. Man extrahiert mehrmals mit Diethylether und trennt anschliessend das rohe Diastereo-meren-ge-misch durch FC (700 g Kieselgel, Dichlormethan-/Diethyl-ether 9:1) auf. Man erhält die Titelverbindung (Diastereomer I): R_{f} (Dichlormethan/-Diethyl-ether 9:1) -=0.21; sowie das C-2 epimere Dia-stereo-mer II: R_{f} (Dichlormethan-/Diethyl-ether 9:1) = 0.14.

### Verwendungsbeispiel 3: 5(S)-Amino-7(S)-[4-methoxy-3-(3-methoxypropoxy)--benzyl]-4(S)-hydroxy-2(R) 8-dimethyl-nonansäure-N(butyl)amid-hydrochlorid

Analog wie im Verwendungsbeispiel 2) beschrieben wird die Titelverbindung ausgehend von 27 mg 5(S)-(*tert*-Butoxycarbonyl)amino-7(S)-[4-methoxy-3-(3-methoxypropoxy)benzyl]-4(S)-hydroxy-2(R),8-dimethyl-nonansäure-N(butyl)amid hergestellt und durch FC (2 g Kiesel-gel, Dichlormethan-/Methanol 95:5) gereinigt: R_{f}(Dichlomethan/Methanol 9:1) = 0.15; Rₜ(I) = 21.9 min; Hochauflösendes FAB-MS(M+H)⁺: ber. 481.3641; gef. 481.3636.

Das Ausgangsmaterial wird ausgehend von 5(S)-(*tert*-Butoxycarbonyl)amino-4(S)-hydroxy7(S)-(3-hydroxy-4-methoxy--benzyl)-2(R),8-dimethyl-nonansäure-N(butyl)amid durch Umsetzung zunächst mit Natriumhydrid in N,N-Dimethylformamid (30 min bei Raumtemperatur) und anschliessender Alkylierung mit 3-Methoxy-propyljodid (24 h Rühren bei Raumtemperatur nach üblichen Verfahren hergestellt.

Das dazu als Ausgangsmaterial verwendete 5(S)-(*tert*-Butoxycarbonyl)amino-4(S)-hydroxy7(S)-(3-hydroxy-4-methoxy--benzyl)-2(R),8-dimethyl-nonansäure-N(butyl)amid kann beispielsweise folgendermassen hergestellt werden:

### a) 5(S)-(tert-Butoxycarbonyl)amino-4(S)-hydroxy-7(S)-(3-hydroxy-4-methoxy--benzyl)-2(R),8-dimethyl-nonansäure-N(butyl)amid

Die Lösung von 5(S)-(*tert*-Butoxycarbonyl)amino-4(S)-hydroxy-7(S)-(3-benzyloxy-4-methoxy--benzyl)-2(R),8-dimethyl-nonansäure-N(butyl)amid (4.7 g) in Methanol (60 mL) wird in Gegen-wart von Pd/C 10% (2.35 g) bei Raumtemperatur und Normaldruck während 1 h hydriert. Nach Filtration, Einengen des Filtrats und Trocknen unter Hochvakuum erhält man die Titel-ver-bindung: R_{f} (Hexan/Essigsäureethylester 1:1) = 0.15; FAB-MS (M+H)⁺ = 509.

### b) 5(S)-(tert-Butoxycarbonyl)amino-4(S)-hydroxy-7(S)-(3-benzyloxy-4-methoxy-benzyl)-2(R) 8-dimethyl-nonansäure-N(butyl)amid

Eine Lösung von 2-[3(S)-(*tert*-Butoxycamonyl)amino-5(S)-(3-benzyloxy-4-methoxy--benzyl)-2(S)-hydroxy-6-methyl-heptyl]-N-butyl-acrylamid (3.5 g) in absolutem Methanol (30 mL) wird unter inerter Atmosphäre in Gegenwart von 20 mg [Ru₂Cl₄((S)-BINAP)₂]·NEt₃ bei Raumtemperatur und 25 bar während 5 h hydriert. Das Reaktionsgemisch wird filtriert und nach Einengen des Filtrats der Rückstand mittels FC (200 g Kiesel-gel,
Hexan/Essigsäureethylester 1:1) ge-reinigt. Man erhält die Titelverbindung: R_{f} (Hexan/Essigsäureethylester 1:1) = 0.16; FAB-MS (M+H)⁺ = 599.

Das als Ausgangsmaterial verwendete 2-[3(S)-(*tert*-Butoxycarbonyl)amino-5(S)-(3-benzyloxy-4-methoxy--benzyl)-2(S)-hydroxy-6-methyl-heptyl]-N-butyl-acrylamid wird analog wie im Verwendungsbeispiel 2b) beschrieben ausgehend von 2(S)-(*tert*-Butoxycarbonyl)amino-4(S)-[4-methoxy-3-(3-methoxypropoxy)-benzyl]-5-methyl-hexanal hergestellt.

## Patentansprüche

1. Verbindungen der Formel I 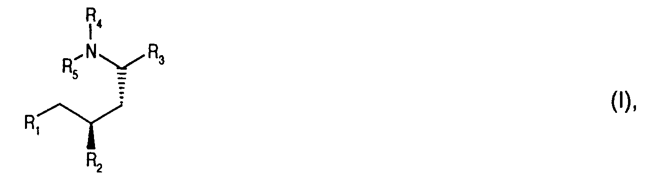 worin
R₁ einen aliphatischen, cycloaliphatischen, cycloaliphatisch-aliphatischen, aromatischen oder heteroaromatischen Rest, eine aliphatisch, araliphatisch oder heteroarylaliphatisch veretherte oder durch eine Hydroxyschutzgruppe geschützte Hydroxygruppe oder eine aliphatisch veretherte Mercaptogruppe und
R₂ einen aliphatischen, cycloaliphatischen, cycloaliphatisch-aliphatischen, araliphatischen oder heteroarylaliphatischen Rest bedeutet oder
R₁ und R₂ gemeinsam einen zweiwertigen aliphatischen Rest darstellen,
R₃ für gegebenenfalls aliphatisch, araliphatisch oder aromatisch verestertes Carboxy, Formyl oder Hydroxymethyl steht,
R₄ Wasserstoff, einen aliphatischen oder araliphatischen Rest oder eine Aminoschutzgruppe bedeutet und
R₅ Wasserstoff oder einen aliphatischen Rest darstellt,
und ihre Salze.

2. Verbindungen gemäß Anspruch 1 der Formel Ib, 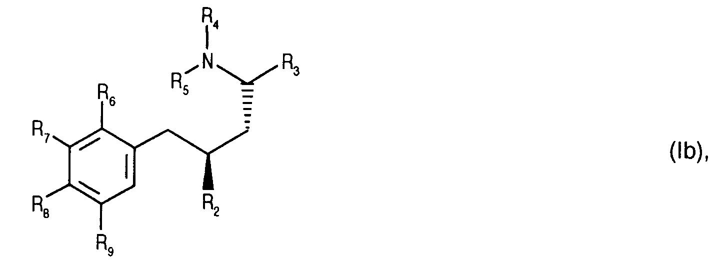 worin
R₂ Niederalkyl, 3- bis 8-gliedriges Cycloalkyl, 3- bis 8-gliedriges Cycloalkylniederalkyl, unsubstituiertes oder im Phenyl-, Naphthyl- bzw. Heteroarylteil durch Niederalkyl, Niederalkoxy, Halogen und/oder Trifluormethyl substituierten Phenylniederalkyl-, Naphthylniederalkyl-, Furylniederalkyl-, Thienylniederalkyl-, gegebenenfalls partiell hydriertes oder N-oxidierten Pyridylniederalkyl-, Pyrimidinylniederalkyl- oder Chinolinylniederalkylrest bedeutet,
R₃ für Carboxy, Niederalkoxycarbonyl, Niederalkenyloxycarbonyl oder einen unsubstituierten oder durch Niederalkyl, Niederalkoxy, Halogen und/oder Trifluormethyl substituierten Phenylniederalkoxycarbonyl- oder Phenyloxycarbonylrest steht, R₄ Wasserstoff, Niederalkyl, einen unsubstituierten oder durch Niederalkyl, Niederalkoxy, Halogen und/oder Trifluormethyl substituierten Phenyl- oder Naphthylniederalkylrest, Niederalkanoyl, Trihalogenniederalkanoyl, eine unsubstituierte oder durch Niederalkyl, Niederalkoxy, Halogen, Trifluormethyl und/oder Nitro substituierte Benzoyl- oder Phenylniederalkoxycarbonylgruppe, Triniederalkylsilyl oder Benzyl(diniederalkyl)silyl bedeutet, R₅ Wasserstoff oder Niederalkyl darstellt,
R₆ Wasserstoff, Hydroxy, Niederalkoxy, Cycloalkoxy, Niederalkoxyniederalkoxy, Carboxyniederalkoxy, Niederalkoxycarbonylniederalkoxy, Carbamoylniederalkoxy oder N-Mono- oder N,N- Diniederalkylcarbamoylniederalkoxy bedeutet,
R₇ Wasserstoff, Niederalkyl, Cycloalkyl, Niederalkoxyniederalkyl, Niederalkoxyniederalkoxyniederalkyl, Cycloalkoxyniederalkyl, Hydroxy, Niederalkanoyloxyniederalkyl, Hydroxyniederalkoxy, Halogen(hydroxy)niederalkoxy, Niederalkansulfonyl(hydroxy)niederalkoxy, Aminoniederalkyl, Niederalkylaminoniederalkyl, Diniederalkylaminoniederalkyl, Niederalkanoylaminoniederalkyl, Niederalkoxycarbonylaminoniederalkyl, Niederalkoxyiminoniederalkyl, Aminoniederalkoxy, Niederalkylaminoniederalkoxy, Diniederalkylaminoniederalkoxy, Niederalkanoylaminoniederalkoxy, Niederalkoxycarbonylaminoniederalkoxy, Oxoniederalkoxy, Niederalkoxy, Cycloalkoxy, Niederalkenyloxy, Cycloalkoxyniederalkoxy, Niederalkoxyniederalkoxy, Niederalkoxyniederalkenyl, Niederalkenyloxyniederalkoxy, Niederalkoxyniederalkenyloxy, Niederalkenyloxyniederalkyl, Niederalkanoylniederalkoxy, Niederalkylthioniederalkoxy, Niederalkansulfonylniederalkoxy, Niederalkylthio(hydroxy)niederalkoxy, Arylniederalkoxy, Thiazolylthioniederalkoxy bzw. Thiazolinylthioniederalkoxy, Imidazolylthioniederalkoxy, gegebenenfalls N-oxidierte Pyridylthioniederalkoxy, Pyrimidinylthioniederalkoxy, Cyanoniederalkoxy, Niederalkoxycarbonylniederalkoxy, Carbamoylniederalkoxy, N-Mono- oder N, N-Diniederalkylcarbamoylniederalkoxy, Carboxyniederalkyl, Niederalkoxycarbonylniederalkyl, Carbamoylniederalkyl oder N-Mono- oder N,N-Diniederalkylcarbamoylniederalkyl bedeutet,
R₈ Niederalkyl, Polyhalogenniederalkyl, Niederalkoxyniederalkyl, Cycloalkoxyniederalkyl, Hydroxyniederalkyl, Niederalkylthioniederalkyl, Niederalkansulfonylniederalkyl, gegebenenfalls partiell hydriertes oder N-oxidierte Pyridylniederalkyl, Thiazolylthioniederalkyl bzw. Thiazolinylthioniederalkyl, Imidazolylthioniederalkyl, gegebenenfalls N-oxidiertes Pyridylthioniederalkyl, Pyrimidinylthioniederalkyl, Aminoniederalkyl, Niederalkylaminoniederalkyl, Diniederalkylaminoniederalkyl, Niederalkanoylaminoniederalkyl, Niederalkansulfonylaminoniederalkyl, Polyhalogenniederalkansulfonylaminoniederalkyl, Pyrrolidinoniederalkyl, Piperidinoniederalkyl, Piperazino-, N'-Niederalkylpiperazino- bzw. N'-Niederalkanoylpiperazinoniederalkyl, Morpholinoniederalkyl, Thiomorpholino-, S-Oxothiomorpholino- oder S,S-Dioxothiomorpholinoniederalkyl, Cyanoniederalkyl, Carboxyniederalkyl, Niederalkoxycarbonylniederalkyl, Carbamoylniederalkyl, N-Mono- oder N,N-Diniederalkylcarbamoylniederalkyl, Cycloalkyl, unsubstituiertes oder durch Niederalkyl, Niederalkoxy, Hydroxy, Niederalkylamino, Diniederalkylamino, Halogen und/oder Trifluormethyl mono-, di- oder trisubstituiertes Phenyl oder Naphthyl, Hydroxy, Niederalkoxy, Cycloalkoxy, Niederalkoxyniederalkoxy, Cycloalkoxyniederalkoxy, Hydroxyniederalkoxy, unsubstituiertes oder durch Niederalkyl, Niederalkoxy, Hydroxy, Niederalkylamino, Diniederalkylamino, Halogen und/oder Trifluormethyl mono-, di- oder trisubstituiertes Phenylniederalkoxy oder Naphthylniederalkoxy, Niederalkoxy, Polyhalogenniederalkoxy, Niederalkylthioniederalkoxy, Niederalkansulfonylniederalkoxy, gegebenenfalls hydriertes Heteroarylniederalkoxy, gegebenenfalls partiell oder vollständig hydriertes Heteroarylthioniederalkoxy, wie Thiazolylthioniederalkoxy bzw. Thiazolinylthioniederalkoxy, Imidazolylthioniederalkoxy, gegebenenfalls N-oxidierte Pyridylthioniederalkoxy, Pyrimidinylthioniederalkoxy, Aminoniederalkoxy, Niederalkylaminoniederalkoxy, Diniederalkylaminoniederalkoxy, Niederalkanoylaminoniederalkoxy, Niederalkansulfonylaminoniederalkoxy, Polyhalogenniederalkansulfonylaminoniederalkoxy, Pyrrolidinoniederalkoxy, Piperidinoniederalkoxy, Piperazino-, N'-Niederalkylpiperazino- bzw. N'-Niederalkanoylpiperazinoniederalkoxy, Morpholinoniederalkoxy, Thiomorpholino-, S-Oxothiomorpholino- oder S,S-Dioxothiomorpholinoniederalkoxy, Cyanoniederalkoxy, Carboxyniederalkoxy, Niederalkoxycarbonylniederalkoxy, Carbamoylniederalkoxy oder N-Mono- oder N,N-Diniederalkylcarbamoylniederalkoxy bedeutet oder gemeinsam mit R₉ Niederalkylendioxy oder einen ankondensierten Benzo- oder Cyclohexenoring darstellt,
R₉ gemeinsam mit R₈ Niederalkylendioxy oder einen ankondensierten Benzo- oder Cyclohexenoring darstellt oder für Wasserstoff, Niederalkyl, Hydroxy, Niederalkoxy oder Cycloalkoxy steht,
und ihrer Salze.

3. Verbindungen gemäß Anspruch 2 der Formel Ib, worin
R₂ für Niederalkyl , 3- bis 5-gliedriges Cycloalkylniederalkyl oder Cycloalkyl steht,
R₃ für Carboxy, Niederalkoxycarbonyl, Formyl oder Hydroxymethyl steht,
R₄ Niederalkoxycarbonyl oder unsubstituiertes oder durch Niederalkyl, Niederalkoxy, Nitro und/oder Halogen substituiertes α-Phenylniederalkoxycarbonyl bedeutet,
R₅ und R₆ Wasserstoff bedeuten,
R₇ Niederalkyl, Niederalkoxyniederalkyl, Niederalkoxyniederalkoxy, Niederalkoxyniederalkoxyniederalkyl, unsubstituiertes oder durch Niederalkyl, Niederalkoxy, Hydroxy, Halogen, Nitro und/oder Amino substituiertes Phenylniederalkoxy, gegebenenfalls N-oxidiertes Pyridylniederalkoxy, Niederalkylthioniederalkoxy, Niederalkansulfonylniederalkoxy, Niederalkanoylniederalkoxy, gegebenenfalls N-oxidiertes Pyridylniederalkoxy, Cyanoniederalkoxy, Carboxyniederalkoxy, Niederalkoxycarbonylniederalkoxy, Carbamoylniederalkoxy Niederalkylcarbamoylniederalkoxy oder Diniederalkylcarbamoylniederalkoxy bedeutet
R₈ Wasserstoff, Niederalkyl, Hydroxy, Niederalkoxy, oder Polyhalogenniederalkoxy bedeutet oder gemeinsam mit R₉ Niederalkylendioxy darstellt und
R₉ Wasserstoff bedeutet oder gemeinsam mit R₈ Niederalkylendioxy darstellt, und ihre Salze.

4. Verbindungen gemäß Anspruch 2 der Formel Ib, worin
R₂ für verzweigtes C₁-C₄-Alkyl oder 3- bis 5-gliedriges Cycloalkyl-C₁-C₄-alkyl steht,
R₃ für Carboxy, C₁-C₄-Alkoxycarbonyl, Formyl oder Hydroxymethyl steht,
R₄ C₁-C₄-Alkoxycarbonyl oder α-Phenyl-C₁-C₄-alkoxycarbonyl bedeutet,
R₅ und R₆ Wasserstoff bedeuten,
R₇ C₁-C₄-Alkyl, C₁-C₄-Alkoxy-C₁-C₄-alkyl, C₁-C₄-Alkoxy-C₁-C₄-alkoxy, C₁-C₄-Alkoxy-C₁-C₄-alkoxy-C₁-C₄-alkyl, Phenyl-C₁-C₄-alkoxy, Pyridyl-C₁-C₄-alkoxy, C₁-C₄-Alkylthio-C₁-C₄-alkoxy, C₁-C₄-Alkansulfonyl-C₁-C₄-alkoxy, C₁-C₄-Alkanoyl-C₁-C₄-alkoxy, C₁-C₄-Alkoxycarbonyl-C₁-C₄ alkoxy, Carbamoyl-C₁-C₄-alkoxy oder Di-C₁-C₄-alkylcarbamoyl-C₁-C₄-alkoxy bedeutet,
R₈ Wasserstoff, C₁-C₄-Alkyl, Hydroxy oder C₁-C₄-Alkoxy bedeutet oder gemeinsam mit R₉ C₁-C₄-Alkylendioxy darstellt und
R₉ Wasserstoff bedeutet oder gemeinsam mit R₈ C₁-C₄-Alkylendioxy darstellt,
und ihre Salze.

5. Verbindungen gemäß Anspruch 2 der Formel Ib, worin
R₂ verzweigtes C₁-C₄-Alkyl bedeutet,
R₃ Carboxy, Formyl oder Hydroxymethyl ist,
R₄ für C₁-C₄-Alkoxycarbonyl steht,
R₅, R₆ und R₉ Wasserstoff darstellen,
R₇ für C₁-C₄-Alkoxy-C₁-C₄-alkoxy oder C₁-C₄-Alkoxy-C₁-C₄-alkyl steht und
R₈ Wasserstoff, C₁-C₄-Alkyl oder C₁-C₄-Alkoxy darstellt,
und ihrer Salze.

6. 2(S)-Amino-4(S)-[4-methoxy-3-(3-methoxypropoxy)-benzyl]-5-methyl-hexansäureethylester oder ein Salz davon.

7. 2(S)-(*tert*-Butoxycarbonyl)amino-4(S)-[4-methoxy-3-(3-methoxypropoxy)-benzyl]-5-methyl-hexansäureethylester oder ein Salz davon.

8. 2(S)-(*tert*-Butoxycarbonyl)amino-4(S)-(p-*tert*-butylphenyl)-5-methyl-hexansäureethylester; oder ein Salz davon.

9. 2(S)-(*tert*-Butoxycarbonyl)amino-4(R)-(p-tert-butylbenzyl)-hexansäureethylester; oder ein Salz davon.

10. 2(S)-(*tert*-Butoxycarbonyl)amino-4(S)-[4-tert-butyl-3-(3-methoxypropoxy)-benzyll-5-methyl-hexansäureethylester oder ein Salz davon.

11. 2(S)-(*tert*-Butoxycarbonyl)amino-4(S)-[3-benzyloxy-4,5-ethylendioxy-benzyl]-5-methyl-hexansäureethylester oder ein Salz davon.

12. 2(S)-(*tert*-Butoxycarbonyl)amino-4(S)-[4-ethyl-3-(3-methoxypropoxy)-benzyl]-5-methyl-hexansäureethylester oder ein Salz davon.

13. 2(S)-(*tert*-Butoxycarbonyl)amino-4(S)-[3-benzyloxy-4-methoxy-benzyl]-5-methyl-hexansäureethylester oder ein Salz davon.

14. 2(S)-(*tert*-Butoxycarbonyl)amino-4(S)-[4-benzyloxy-3-(3-methoxy-propoxy)-benzyl] -5-methyl-hexansäureethylester oder ein Salz davon.

15. 2(S)-(*tert*-Butoxycarbonyl)amino-4(S)-benzyloxymethyl-5-methyl-hexansäureethylester oder ein Salz davon.

16. 2(S)-(*tert*-Butoxycarbonyl)amino-4(S)-[4-methoxy-3-(3-methoxypropoxy)-benzyl]-5-methyl-hexan-1-ol oder ein Salz davon.

17. 2(S)-(*tert*-Butoxycarbonyl)amino-4(S)-[4-methoxy-3-(3-methoxypropoxy)-benzyl]-5-methyl-hexanal oder ein Salz davon.

18. 2(S)-(*tert*-Butoxycarbonyl)amino-4(S)-(p-*tert*-butyl-benzyl)-5-methyl-hexanal oder ein Salz davon.

19. 2(S)-(*tert*-Butoxycarbonyl)amino-4(R)-(p-*tert*-butyl-benzyl)-hexanal oder ein Salz davon.

20. 2(S)-(*tert*-Butoxycarbonyl)amino-4(S)-[4-*tert*-butyl-3-(3-methoxypropoxy)-benzyl]-5-methyl-hexanal oder ein Salz davon.

21. 2(S)-(*tert*-Butoxycarbonyl)amino-4(S)-[3-benzyloxy-4,5-ethylendioxy-benzyl]-5-methyl-hexanal oder ein Salz davon.

22. 2(S)-(*tert*-Butoxycarbonyl)amino-4(S)-[4-ethyl-3-(3-methoxypropoxy)-benzyl]-5-methyl-hexanal oder ein Salz davon.

23. 2(S)-(*tert*-Butoxycarbonyl)amino-4(S)-[4-methoxy-3-benzyloxy-benzyl]-5-methyl-hexanal oder ein Salz davon.

24. 2(S)-(*tert*-Butoxycarbonyl)amino-4(S)-[4-benzyloxy-3-(3-methoxy-propoxy)-benzyl]-5-methyl-hexanal oder ein Salz davon.

25. Verfahren zur Herstellung von Verbindungen der Formel I 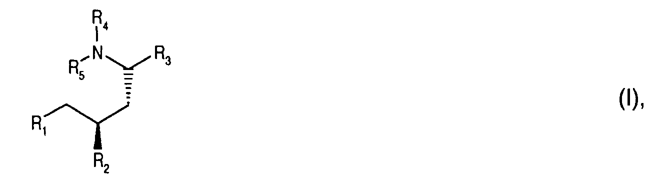 worin
R₁ einen aliphatischen, cycloaliphatischen, cycloaliphatisch-aliphatischen, aromatischen oder heteroaromatischen Rest, eine aliphatisch, araliphatisch oder heteroarylaliphatisch veretherte oder durch eine Hydroxyschutzgruppe geschützte Hydroxygruppe oder eine aliphatisch veretherte Mercaptogruppe und
R₂ einen aliphatischen, cycloaliphatischen, cycloaliphatisch-aliphatischen, araliphatischen oder heteroarylaliphatischen Rest bedeutet oder
R₁ und R₂ gemeinsam einen zweiwertigen aliphatischen Rest darstellen,
R₃ für gegebenenfalls aliphatisch, araliphatisch oder aromatisch verestertes Carboxy, Formyl oder Hydroxymethyl steht,
R₄ Wasserstoff, einen aliphatischen oder araliphatischen Rest oder eine Aminoschutzgruppe bedeutet und
R₅ Wasserstoff oder einen aliphatischen Rest darstellt,
und ihrer Salze, dadurch gekennzeichnet, daß man eine Verbindung der Formel IX 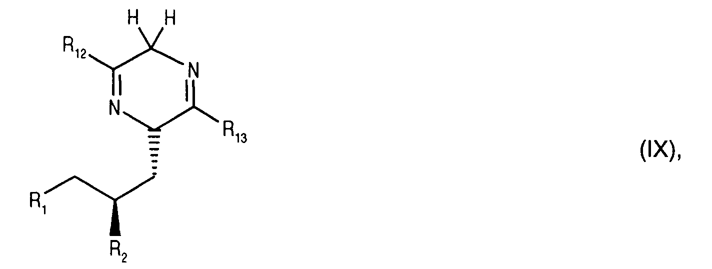 worin R₁₂ und R₁₃ unabhängig voneinander für aliphatisch, araliphatisch oder aromatisch verethertes Hydroxy, insbesondere für Niederalkoxy, stehen, R₁ und R₂ die vorstehend unter der Formel I angegebenen Bedeutungen haben, unter Aufspaltung des Pyrazinringes zur entsprechenden Verbindung der Formel I hydrolysiert, worin R₃ für aliphatisch, araliphatisch oder aromatisch verestertes Carboxy der Formel -C(=O)-R₁₃ steht, und gewünschtenfalls eine verfahrensgemäß erhältliche Verbindung in eine andere Verbindung der Formel I umwandelt, gegebenenfalls erhältliche Isomerengemische auftrennt und/oder eine verfahrensgemäß erhältliche Verbindung der Formel I mit mindestens einer salzbildenden Gruppe in ein Salz oder ein verfahrensgemäß erhältliches Salz in die freie Verbindung oder in ein anderes Salz überführt.

26. Verbindungen der Formel IX 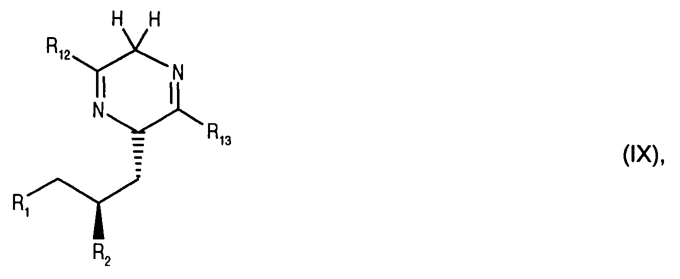 worin R₁ einen aliphatischen, cycloaliphatischen, cycloaliphatisch-aliphatischen, aromatischen oder heteroaromatischen Rest, eine aliphatisch, araliphatisch oder heteroarylaliphatisch veretherte oder durch eine Hydroxyschutzgruppe geschützte Hydroxygruppe oder eine aliphatisch veretherte Mercaptogruppe und
R₂ einen aliphatischen, cycloaliphatischen, cycloaliphatisch-aliphatischen, araliphatischen oder heteroarylaliphatischen Rest bedeutet oder
R₁ und R₂ gemeinsam einen zweiwertigen aliphatischen Rest darstellen und
R₁₂ und R₁₃ unabhängig voneinander für aliphatisch, araliphatisch oder aromatisch verethertes Hydroxy, insbesondere für Niederalkoxy, stehen, und ihre Salze.

27. Verbindungen gemäß Anspruch 26 der Formel IX, worin
R₁ Niederalkyl, Niederalkenyl, 3- bis 8-gliedriges Cycloalkyl, 3- bis 8-gliedriges Cycloalkylniederalkyl, einen unsubstituierten oder durch Aminoniederalkoxy, Aminoniederalkyl, Arylniederalkoxy, Carbamoylniederalkoxy, Carbamoylniederalkyl, Carboxyniederalkoxy, Carboxyniederalkyl, Cyanoniederalkoxy, Cyanoniederalkyl, 3- bis 8-gliedriges Cycloalkoxy, 3- bis 8-gliedriges Cycloalkoxyniederalkoxy, 3- bis 8-gliedriges Cycloalkoxyniederalkyl, 3- bis 8-gliedriges Cycloalkyl, Diniederalkylamino, Diniederalkylaminoniederalkoxy, Diniederalkylaminoniederalkyl, Pyridylniederalkoxy, Tertrahydropyridylniederalkoxy, N-Oxidopyridylniederalkoxy, Thiazolylniederalkoxy, Morpholinoniederalkoxy, Pyridylthioniederalkoxy, N-Oxidopyridylthioniederalkoxy, Pyridylthioniederalkyl, N-Oxidopyridylthioniederalkyl, Halogen(hydroxy)niederalkoxy, Halogen, Hydroxy, Hydroxyniederalkoxy, Hydroxyniederalkyl, Imidazolylthioniederalkoxy, Imidazolylthioniederalkyl, Morpholinoniederalkoxy, Morpholinoniederalkyl, N-Mono- oder N,N-Diniederalkylcarbamoylniederalkoxy, N-Mono- oder N,N-Diniederalkylcarbamoylniederalkyl, gegebenenfalls N-oxidierte Pyridylniederalkyl, N'-Niederalkylpiperazino- bzw. N'-Niederalkanoylpiperazinoniederalkoxy, N'-Niederalkylpiperazino- bzw. N'-Niederalkanoylpiperazinoniederalkyl, Naphthyl, Naphthylniederalkoxy, Niederalkanoylaminoniederalkoxy, Niederalkanoylaminoniederalkyl, Niederalkanoylniederalkoxy, Niederalkanoyloxyniederalkyl, Niederalkansulfonyl(hydroxy)niederalkoxy, Niederalkansulfonylaminoniederalkoxy, Niederalkansulfonylaminoniederalkyl, Niederalkansulfonylniederalkoxy, Niederalkansulfonylniederalkyl, Niederalkenyloxy, Niederalkenyloxyniederalkoxy, Niederalkenyloxyniederalkyl, Niederalkoxy, Niederalkoxycarbonylaminoniederalkoxy, Niederalkoxycarbonylaminoniederalkyl, Niederalkoxycarbonylniederalkoxy, Niederalkoxycarbonylniederalkyl, Niederalkoxyiminoniederalkyl, Niederalkoxyniederalkenyl, Niederalkoxyniederalkenyloxy, Niederalkoxyniederalkoxy, Niederalkoxyniederalkoxyniederalkyl, Niederalkoxyniederalkyl, Niederalkyl, Niederalkoxy, Niederalkylaminoniederalkoxy, Niederalkylaminoniederalkyl, Niederalkylthio(hydroxy)niederalkoxy, Niederalkylthioniederalkoxy, Niederalkylthioniederalkoxy, Niederalkylthioniederalkyl, Oxoniederalkoxy, Priperazinoniederalkoxy, Piperazinoniederalkyl, Piperidinoniederalkoxy, Piperidinoniederalkyl, Polyhalogenniederalkansulfonylaminoniederalkoxy, Polyhalogenniederalkansulfonylaminoniederalkyl, Polyhalogenniederalkoxy, Polyhalogenniederalkyl, Pyrimidinylthioniederalkoxy, Pyrimidinylthioniederalkyl, Pyrrolidinoniederalkoxy, Pyrrolidinoniederalkyl, S,S-Dioxothiomorpholinoniederalkoxy, S,S-Dioxothiomorpholinoniederalkyl, S-Oxothiomorpholinoniederalkoxy, S-Oxothiomorpholinoniederalkyl, Thiazolylthioniederalkoxy, Thiazolinylthioniederalkoxy, Thiazolylthioniederalkyl, Thiazolinylthioniederalkyl und/oder Thiomorpholino mono-, di- oder trisubstituierten Phenyl- oder Naphthyl- oder durch einen ankondensierten Benzo- oder Cyclohexenoring disubstituierten Phenylrest, einen unsubstituierten oder durch Niederalkyl, Niederalkoxy, Halogen und/oder Trifluormethyl substituierten Furyl-, Thienyl-, Pyridyl-, Pyrimidinyl-, Indolyl- oder Chinolinylrest, Niederalkoxy, Niederalkenyloxy, unsubstituierte oder im Phenylteil durch Niederalkyl, Niederalkoxy, Halogen und/oder Trifluormethyl substituiertes Phenylniederalkoxy, unsubstituiertes oder im Ring durch Niederalkyl, Niederalkoxy, Halogen und/oder Trifluormethyl substituierte Furylniederalkoxy-, Thienylniederalkoxy-, Pyridylniederalkoxy- oder Chinolinylniederalkoxygruppen, Niederalkanoyloxy, Trihalogenniederalkanoyloxy, eine unsubstituierte oder durch Niederalkyl, Niederalkoxy, Halogen, Trifluormethyl und/oder Nitro substituierte Benzoyloxy- oder Phenylniederalkoxycarbonyloxygruppe, Triniederalkylsilyloxy, Benzyl(diniederalkyl)silyloxy oder Niederalkylthio bedeutet,
R₂ Niederalkyl, 3- bis 8-gliedriges Cycloalkyl, 3- bis 8-gliedriges Cycloalkylniederalkyl, unsubstituiertes oder im Phenyl-, Naphthyl- bzw. Heteroarylteil durch Niederalkyl, Niederalkoxy, Halogen und/oder Trifluormethyl substituierten Phenylniederalkyl-, Naphthylniederalkyl-, Furylniederalkyl-, Thienylniederalkyl-, gegebenenfalls partiell hydriertes oder N-oxidierten Pyridylniederalkyl-, Pyrimidinylniederalkyl- oder Chinolinylniederalkylrest bedeutet oder R₁ und R₂ gemeinsam Niederalkylen, dessen freie Valenzen von benachbarten oder zueinander 1,3-, 1,4- oder 1,5-ständigen C-Atomen ausgehen, darstellen und
R₁₂ und R₁₃ unabhängig voneinander für Niederalkoxy, Niederalkenyloxy, unsubstituiertes oder durch Niederalkyl, Niederalkoxy, Halogen und/oder Trifluormethyl substituiertes Phenyloxy oder unsubstituiertes oder im Phenyl- bzw. Naphthylteil durch Niederalkyl, Niederalkoxy, Halogen und/oder Trifluormethyl substituiertes Phenylniederalkoxy oder Naphthylniederalkoxy stehen, und ihre Salze.

28. Verbindungen gemäß Anspruch 26 der Formel IXa 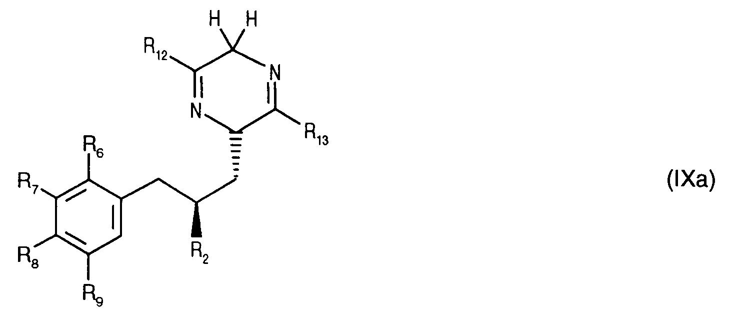 worin
R₂ Niederalkyl, 3- bis 8-gliedriges Cycloalkyl, 3- bis 8-gliedriges Cycloalkylniederalkyl, unsubstituiertes oder im Phenyl-, Naphthyl- bzw. Heteroarylteil durch Niederalkyl, Niederalkoxy, Halogen und/oder Trifluormethyl substituierten Phenylniederalkyl-, Naphthylniederalkyl-, Furylniederalkyl-, Thienylniederalkyl-, gegebenenfalls partiell hydriertes oder N-oxidierten Pyridylniederalkyl-, Pyrimidinylniederalkyl- oder Chinolinylniederalkylrest bedeutet,
R₆ Wasserstoff, Hydroxy, Niederalkoxy, Cycloalkoxy, Niederalkoxyniederalkoxy, Carboxyniederalkoxy, Niederalkoxycarbonylniederalkoxy, Carbamoylniederalkoxy oder N-Mono- oder N,N- Diniederalkylcarbamoylniederalkoxy bedeutet,
R₇ Wasserstoff, Niederalkyl, Cycloalkyl, Niederalkoxyniederalkyl, Niederalkoxyniederalkoxyniederalkyl, Cycloalkoxyniederalkyl, Hydroxy, Niederalkanoyloxyniederalkyl, Hydroxyniederalkoxy, Halogen(hydroxy)niederalkoxy, Niederalkansulfonyl(hydroxy)niederalkoxy, Aminoniederalkyl, Niederalkylaminoniederalkyl, Diniederalkylaminoniederalkyl, Niederalkanoylaminoniederalkyl, Niederalkoxycarbonylaminoniederalkyl, Niederalkoxyiminoniederalkyl, Aminoniederalkoxy, Niederalkylaminoniederalkoxy, Diniederalkylaminoniederalkoxy, Niederalkanoylaminoniederalkoxy, Niederalkoxycarbonylaminoniederalkoxy, Oxoniederalkoxy, Niederalkoxy, Cycloalkoxy, Niederalkenyloxy, Cycloalkoxyniederalkoxy, Niederalkoxyniederalkoxy, Niederalkoxyniederalkenyl, Niederalkenyloxyniederalkoxy, Niederalkoxyniederalkenyloxy, Niederalkenyloxyniederalkyl, Niederalkanoylniederalkoxy, Niederalkylthioniederalkoxy, Niederalkansulfonylniederalkoxy, Niederalkylthio(hydroxy)niederalkoxy, Arylniederalkoxy, Thiazolylthioniederalkoxy bzw. Thiazolinylthioniederalkoxy, Imidazolylthioniederalkoxy, gegebenenfalls N-oxidierte Pyridylthioniederalkoxy, Pyrimidinylthioniederalkoxy, Cyanoniederalkoxy, Niederalkoxycarbonylniederalkoxy, Carbamoylniederalkoxy, N-Mono- oder N,N-Diniederalkylcarbamoylniederalkoxy, Carboxyniederalkyl, Niederalkoxycarbonylniederalkyl, Carbamoylniederalkyl oder N-Mono- oder N,N-Diniederalkylcarbamoylniederalkyl bedeutet,
R₈ Niederalkyl, Polyhalogenniederalkyl, Niederalkoxyniederalkyl, Cycloalkoxyniederalkyl, Hydroxyniederalkyl, Niederalkylthioniederalkyl, Niederalkansulfonylniederalkyl, gegebenenfalls partiell hydriertes oder N-oxidierte Pyridylniederalkyl, Thiazolylthioniederalkyl bzw. Thiazolinylthioniederalkyl, Imidazolylthioniederalkyl, gegebenenfalls N-oxidiertes Pyridylthioniederalkyl, Pyrimidinylthioniederalkyl, Aminoniederalkyl, Niederalkylaminoniederalkyl, Diniederalkylaminoniederalkyl, Niederalkanoylaminoniederalkyl, Niederalkansulfonylaminoniederalkyl, Polyhalogenniederalkansulfonylaminoniederalkyl, Pyrrolidinoniederalkyl, Piperidinoniederalkyl, Piperazino-, N'-Niederalkylpiperazino- bzw. N'-Niederalkanoylpiperazinoniederalkyl, Morpholinoniederalkyl, Thiomorpholino-, S-Oxothiomorpholino- oder S,S-Dioxothiomorpholinoniederalkyl, Cyanoniederalkyl, Carboxyniederalkyl, Niederalkoxycarbonylniederalkyl, Carbamoylniederalkyl, N-Mono- oder N,N-Diniederalkylcarbamoylniederalkyl, Cycloalkyl, unsubstituiertes oder durch Niederalkyl, Niederalkoxy, Hydroxy, Niederalkylamino, Diniederalkylamino, Halogen und/oder Trifluormethyl mono-, di- oder trisubstituiertes Phenyl oder Naphthyl, Hydroxy, Niederalkoxy, Cycloalkoxy, Niederalkoxyniederalkoxy, Cycloalkoxyniederalkoxy, Hydroxyniederalkoxy, unsubstituiertes oder durch Niederalkyl, Niederalkoxy, Hydroxy, Niederalkylamino, Diniederalkylamino, Halogen und/oder Trifluormethyl mono-, di- oder trisubstituiertes Phenylniederalkoxy oder Naphthylniederalkoxy, Niederalkoxy, Polyhalogenniederalkoxy, Niederalkylthioniederalkoxy, Niederalkansulfonylniederalkoxy, gegebenenfalls hydriertes Heteroarylniederalkoxy, gegebenenfalls partiell oder vollständig hydriertes Heteroarylthioniederalkoxy, wie Thiazolylthioniederalkoxy bzw. Thiazolinylthioniederalkoxy, Imidazolylthioniederalkoxy, gegebenenfalls N-oxidierte Pyridylthioniederalkoxy, Pyrimidinylthioniederalkoxy, Aminoniederalkoxy, Niederalkylaminoniederalkoxy, Diniederalkylaminoniederalkoxy, Niederalkanoylaminoniederalkoxy, Niederalkansulfonylaminoniederalkoxy, Polyhalogenniederalkansulfonylaminoniederalkoxy, Pyrrolidinoniederalkoxy, Piperidinoniederalkoxy, Piperazino-, N'-Niederalkylpiperazino- bzw. N'-Niederalkanoylpiperazinoniederalkoxy, Morpholinoniederalkoxy, Thiomorpholino-, S-Oxothiomorpholino- oder S,S-Dioxothiomorpholinoniederalkoxy, Cyanoniederalkoxy, Carboxyniederalkoxy, Niederalkoxycarbonylniederalkoxy, Carbamoylniederalkoxy oder N-Mono- oder N,N-Diniederalkyl-carbamoylniederalkoxy bedeutet oder gemeinsam mit R₉ Niederalkylendioxy oder einen ankondensierten Benzo- oder Cyclohexenoring darstellt,
R₉ gemeinsam mit R₈ Niederalkylendioxy oder einen ankondensierten Benzo- oder Cyclohexenoring darstellt oder für Wasserstoff, Niederalkyl, Hydroxy, Niederalkoxy oder Cycloalkoxy steht und
R₁₂ und R₁₃ unabhängig voneinander für Niederalkoxy, Niederalkenyloxy, unsubstituiertes oder durch Niederalkyl, Niederalkoxy, Halogen und/oder Trifluormethyl substituiertes Phenyloxy oder unsubstituiertes oder im Phenyl- bzw. Naphthylteil durch Niederalkyl, Niederalkoxy, Halogen und/oder Trifluormethyl substituiertes Phenylniederalkoxy oder Naphthylniederalkoxy stehen,
und ihre Salze.

29. Verbindungen gemäß Anspruch 28 der Formel IXa, worin
R₂ für Niederalkyl, 3- bis 5-gliedriges Cycloalkylniederalkyl oder Cycloalkyl steht,
R₆ Wasserstoff bedeutet,
R₇ Niederalkyl, Niederalkoxyniederalkyl, Niederalkoxyniederalkoxy, Niederalkoxyniederalkoxyniederalkyl, unsubstituiertes oder durch Niederalkyl, Niederalkoxy, Hydroxy, Halogen, Nitro und/oder Amino substituiertes Phenylniederalkoxy, gegebenenfalls N-oxidiertes Pyridylniederalkoxy, Niederalkylthioniederalkoxy, Niederalkansulfonylniederalkoxy, Niederalkanoylniederalkoxy, gegebenenfalls N-oxidiertes Pyridylniederalkoxy, Cyanoniederalkoxy, Carboxyniederalkoxy, Niederalkoxycarbonylniederalkoxy, Carbamoylniederalkoxy Niederalkylcarbamoylniederalkoxy oder Diniederalkylcarbamoylniederalkoxy bedeutet,
R₈ Wasserstoff, Niederalkyl, Hydroxy, Niederalkoxy, oder Polyhalogenniederalkoxy bedeutet oder gemeinsam mit R₉ Niederalkylendioxy darstellt,
R₉ Wasserstoff bedeutet oder gemeinsam mit R₈ Niederalkylendioxy darstellt und
R₁₂ und R₁₃ unabhängig voneinander für Niederalkoxy, Niederalkenyloxy, unsubstituiertes oder durch Niederalkyl, Niederalkoxy, Halogen und/oder Trifluormethyl substituiertes Phenyloxy oder unsubstituiertes oder im Phenylteil durch Niederalkyl, Niederalkoxy, Halogen und/oder Trifluormethyl substituiertes Phenylniederalkoxy stehen, und ihre Salze.

30. Verbindungen gemäß Anspruch 28 der Formel IXa, worin
R₂ für verzweigtes C₁-C₄-Alkyl oder 3- bis 5-gliedriges oder Cycloalkyl-C₁-C₄-alkyl steht,
R₆ Wasserstoff bedeutet,
R₇ C₁-C₄-Alkyl, C₁-C₄-Alkoxy-C₁-C₄-alkyl, C₁-C₄-Alkoxy-C₁-C₄-alkoxy, C₁-C₄-Alkoxy-C₁-C₄-alkoxy-C₁-C₄-alkyl, Phenyl-C₁-C₄-alkoxy, Pyridyl-C₁-C₄-alkoxy, C₁-C₄-Alkylthio-C₁-C₄-alkoxy, C₁-C₄-Alkansulfonyl-C₁-C₄-alkoxy, C₁-C₄-Alkanoyl-C₁-C₄-alkoxy, C₁-C₄-Alkoxycarbonyl-C₁-C₄-alkoxy, Carbamoyl-C₁-C₄-alkoxy oder Di-C₁-C₄-alkylcarbamoyl-C₁-C₄-alkoxy bedeutet,
R₈ Wasserstoff, C₁-C₄-Alkyl, Hydroxy oder C₁-C₄-Alkoxy bedeutet oder gemeinsam mit R₉ C₁-C₄-Alkylendioxy darstellt,
R₉ Wasserstoff bedeutet oder gemeinsam mit R₈ C₁-C₄-Alkylendioxy darstellt und
R₁₂ und R₁₃ gleiche oder verschiedene C₁-C₄-Alkoxy- oder C₂-C₄-Alkenyloxygruppe bedeuten,
und ihre Salze.

31. 2(S)-{2(S)-[4-Methoxy-3-(3-methoxypropoxy)-benzyl]-3-methylbutyl}-3,6-diethoxy-2,5-dihydro-pyrazin oder ein Salz davon.

32. 2(S)-[2(S)-Benzyloxymethyl-3-methylbutyl]-3,6-diethoxy-2,5-dihydro-pyrazin oder ein Salz davon.

33. Verfahren zur Herstellung von Verbindungen der Formel IX 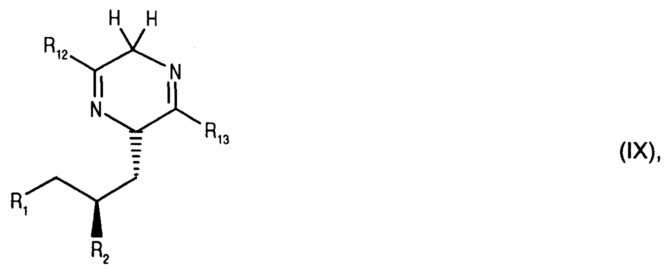 worin R₁ einen aliphatischen, cycloaliphatischen, cycloaliphatisch-aliphatischen, aromatischen oder heteroaromatischen Rest, eine aliphatisch, araliphatisch oder heteroarylalipha-tisch veretherte oder durch eine Hydroxyschutzgruppe geschützte Hydroxygruppe oder eine aliphatisch veretherte Mercaptogruppe und
R₂ einen aliphatischen, cycloaliphatischen, cycloaliphatisch-aliphatischen, araliphatischen oder heteroarylaliphatischen Rest bedeutet oder
R₁ und R₂ gemeinsam einen zweiwertigen aliphatischen Rest darstellen und
R₁₂ und R₁₃ unabhängig voneinander für aliphatisch, araliphatisch oder aromatisch verethertes Hydroxy, insbesondere für Niederalkoxy, stehen, und ihrer Salze, dadurch gekennzeichnet, daß man eine Verbindung der Formel 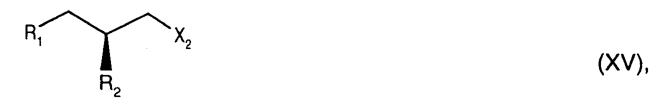 worin X₂ reaktionsfähiges verestertes Hydroxy, insbesondere Halogen, beispielsweise Brom, ist, mit einem Metallsalz einer Verbindung der Formel XVI 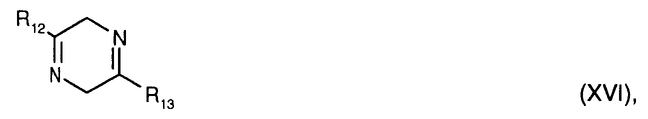 erhältlich aus derselben durch Behandlung mit einer Metallbase kondensiert und gewünschtenfalls eine erfindungsgemäße Verbindung der Formel IX in eine andere erfindungsgemäße Verbindung der Formel IX umwandelt, oder gegebenenfalls erhältliche Isomerengemische auftrennt und/oder die verfahrensgemäß erhältliche Verbindung der Formel IX in ein Salz oder ein verfahrensgemäß erhältliches Salz in die freie Verbindung oder in ein anderes Salz überführt.

34. Verwendung von Verbindungen der Formel IXa 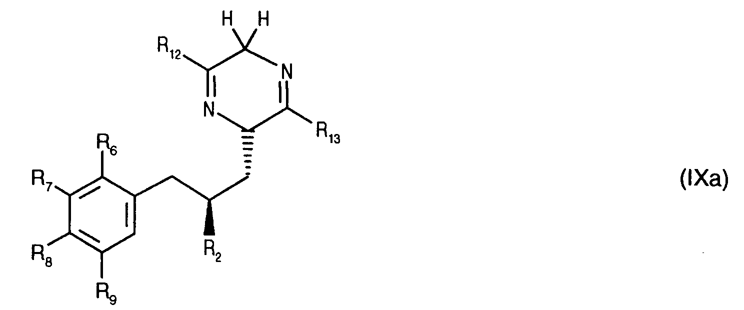 bedeutet, worin
R₂ Niederalkyl, 3- bis 8-gliedriges Cycloalkyl, 3- bis 8-gliedriges Cycloalkylniederalkyl, unsubstituiertes oder im Phenyl- , Naphthyl- bzw. Heteroarylteil durch Niederalkyl, Niederalkoxy, Halogen und/oder Trifluormethyl substituierten Phenylniederalkyl-, Naphthylniederalkyl-, Furylniederalkyl-, Thienylniederalkyl-, gegebenenfalls partiell hydriertes oder N-oxidierten Pyridylniederalkyl-, Pyrimidinylniederalkyl- oder Chinolinylniederalkylrest bedeutet,
R₆ Wasserstoff, Hydroxy, Niederalkoxy, Cycloalkoxy, Niederalkoxyniederalkoxy, Carboxyniederalkoxy, Niederalkoxycarbonylniederalkoxy, Carbamoylniederalkoxy oder N-Mono- oder N,N- Diniederalkylcarbamoylniederalkoxy bedeutet,
R₇ Wasserstoff, Niederalkyl, Cycloalkyl, Niederalkoxyniederalkyl, Niederalkoxyniederalkoxyniederalkyl, Cycloalkoxyniederalkyl, Hydroxy, Niederalkanoyloxyniederalkyl, Hydroxyniederalkoxy, Halogen(hydroxy)niederalkoxy, Niederalkansulfonyl(hydroxy)niederalkoxy, Aminoniederalkyl, Niederalkylaminoniederalkyl, Diniederalkylaminoniederalkyl, Niederalkanoyl-aminoniederalkyl, Niederalkoxycarbonylaminoniederalkyl, Niederalkoxyiminoniederalkyl, Aminoniederalkoxy, Niederalkylaminoniederalkoxy, Diniederalkylaminoniederalkoxy, Niederalkanoylaminoniederalkoxy, Niederalkoxycarbonylaminoniederalkoxy, Oxoniederalkoxy, Niederalkoxy, Cycloalkoxy, Niederalkenyloxy, Cycloalkoxyniederalkoxy, Niederalkoxyniederalkoxy, Niederalkoxyniederalkenyl, Niederalkenyloxyniederalkoxy, Niederalkoxyniederalkenyloxy, Niederalkenyloxyniederalkyl, Niederalkanoylniederalkoxy, Niederalkylthioniederalkoxy, Niederalkansulfonylniederalkoxy, Niederalkylthio(hydroxy)niederalkoxy, Arylniederalkoxy, Thiazolylthioniederalkoxy bzw. Thiazolinylthioniederalkoxy, Imidazolylthioniederalkoxy, gegebenenfalls N-oxidierte Pyridylthioniederalkoxy, Pyrimidinylthioniederalkoxy, Cyanoniederalkoxy, Niederalkoxycarbonylniederalkoxy, Carbamoylniederalkoxy, N-Mono- oder N,N-Diniederalkylcarbamoylniederalkoxy, Carboxyniederalkyl, Niederalkoxycarbonylniederalkyl, Carbamoylniederalkyl oder N-Mono- oder N,N-Diniederalkylcarbamoylniederalkyl bedeutet,
R₈ Niederalkyl, Polyhalogenniederalkyl, Niederalkoxyniederalkyl, Cycloalkoxyniederalkyl, Hydroxyniederalkyl, Niederalkylthioniederalkyl, Niederalkansulfonylniederalkyl, gegebenenfalls partiell hydriertes oder N-oxidierte Pyridylniederalkyl, Thiazolylthioniederalkyl bzw. Thiazolinylthioniederalkyl, Imidazolylthioniederalkyl, gegebenenfalls N-oxidiertes Pyridylthioniederalkyl, Pyrimidinylthioniederalkyl, Aminoniederalkyl, Niederalkylaminoniederalkyl, Diniederalkylaminoniederalkyl, Niederalkanoylaminoniederalkyl, Niederalkansulfonylaminoniederalkyl, Polyhalogenniederalkansulfonylaminoniederalkyl, Pyrrolidinoniederalkyl, Piperidinoniederalkyl, Piperazino-, N'-Niederalkylpiperazino- bzw. N′-Niederalkanoylpiperazinoniederalkyl, Morpholinoniederalkyl, Thiomorpholino-, S-Oxothiomorpholino- oder S,S-Dioxothiomorpholinoniederalkyl, Cyanoniederalkyl, Carboxyniederalkyl, Niederalkoxycarbonylniederalkyl, Carbamoylniederalkyl, N-Mono- oder N,N-Diniederalkylcarbamoylniederalkyl, Cycloalkyl, unsubstituiertes oder durch Niederalkyl, Niederalkoxy, Hydroxy, Niederalkylamino, Diniederalkylamino, Halogen und/oder Trifluormethyl mono-, di- oder trisubstituiertes Phenyl oder Naphthyl, Hydroxy, Niederalkoxy, Cycloalkoxy, Niederalkoxyniederalkoxy, Cycloalkoxyniederalkoxy, Hydroxyniederalkoxy, unsubstituiertes oder durch Niederalkyl, Niederalkoxy, Hydroxy, Niederalkylamino, Di- niederalkylamino, Halogen und/oder Trifluormethyl mono-, di- oder trisubstituiertes Phenylniederalkoxy oder Naphthylniederalkoxy, Niederalkoxy, Polyhalogenniederalkoxy, Niederalkylthioniederalkoxy, Niederalkansulfonylniederalkoxy, gegebenenfalls hydriertes Heteroarylniederalkoxy, gegebenenfalls partiell oder vollständig hydriertes Heteroarylthioniederalkoxy, wie Thiazolylthioniederalkoxy bzw. Thiazolinylthioniederalkoxy, Imidazolylthioniederalkoxy, gegebenenfalls N-oxidierte Pyridylthioniederalkoxy, Pyrimidinylthioniederalkoxy, Aminoniederalkoxy, Niederalkylaminoniederalkoxy, Diniederalkylaminoniederalkoxy, Niederalkanoylaminoniederalkoxy, Niederalkansulfonylaminoniederalkoxy, Polyhalogenniederalkansulfonylaminoniederalkoxy, Pyrrolidinoniederalkoxy, Piperidinoniederalkoxy, Piperazino-, N'-Niederalkylpiperazino- bzw. N'-Niederalkanoylpiperazinoniederalkoxy, Morpholinoniederalkoxy, Thiomorpholino-, S-Oxothiomorpholino- oder S,S-Dioxothiomorpholinoniederalkoxy, Cyanoniederalkoxy, Carboxyniederalkoxy, Niederalkoxycarbonylniederalkoxy, Carbamoylniederalkoxy oder N-Mono- oder N,N-Diniederalkylcarbamoylniederalkoxy bedeutet oder gemeinsam mit R₉ Niederalkylendioxy oder einen ankondensierten Benzo- oder Cyclohexenoring darstellt,
R₉ gemeinsam mit R₈ Niederalkylendioxy oder einen ankondensierten Benzo- oder Cyclohexenoring darstellt oder für Wasserstoff, Niederalkyl, Hydroxy, Niederalkoxy oder Cycloalkoxy steht und
R₁₂ und R₁₃ unabhängig voneinander für Niederalkoxy, Niederalkenyloxy, unsubstituiertes oder durch Niederalkyl, Niederalkoxy, Halogen und/oder Trifluormethyl substituiertes Phenyloxy oder unsubstituiertes oder im Phenyl- bzw. Naphthylteil durch Niederalkyl, Niederalkoxy, Halogen und/oder Trifluormethyl substituiertes Phenylniederalkoxy oder Naphthylniederalkoxy stehen, und ihre Salze zur Herstellung von Verbindung der Formel IIa 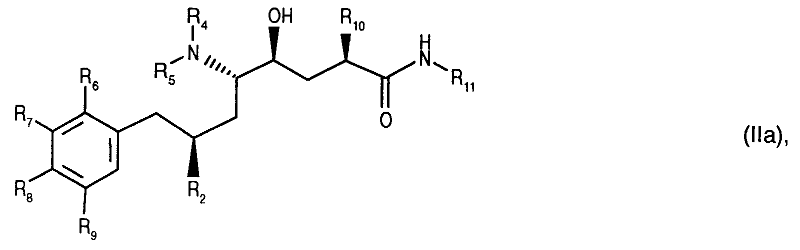 worin R₂, R₆, R₇, R₈ und R₉ die vorstehend angegebenen Bedeutungen haben R₄ Wasserstoff, Niederalkyl oder Niederalkanoyl, insbesondere Wasserstoff, ist, R₅ Wasserstoff oder, sofern R₄ für Niederalkyl steht, Wasserstoff oder Niederalkyl ist R₁₀ Niederalkyl, Niederalkenyl, Cycloalkyl oder Arylniederalkyl bedeutet und R₁₁ Niederalkyl, Cycloalkyl, gegebenenfalls aliphatisch verestertes oder verethertes Hydroxyniederalkyl, gegebenenfalls N-niederalkanoyliertes, N-mono- oder N,N-diniederalkyliertes oder durch Niederalkylen, Hydroxy-, Niederalkoxy- oder Niederalkanoyloxyniederalkylen, gegebenenfalls N'-niederalkanoyliertes bzw. N'-niederalkyliertes Azaniederalkylen, Oxaniederalkylen oder gegebenenfalls S-oxidiertes Thianiederalkylen N,N-disubstituiertes Aminoniederalkyl, gegebenenfalls verestertes oder amidiertes Carboxyniederalkyl, gegebenenfalls verestertes oder amidiertes Dicarboxyniederalkyl, gegebenenfalls verestertes oder amidiertes Carboxy(hydroxy)niederalkyl, gegebenenfalls verestertes oder amidiertes Carboxycyloalkylniederalkyl, Cyanoniederalkyl, Niederalkansulfonylniederalkyl, gegebenenfalls N-mono- oder N,N-diniederalkyliertes Thiocarbamoylniederalkyl, gegebenenfalls N-mono- oder N,N-diniederalkyliertes Sulfamoylniederalkyl oder einen über ein C-Atom gebundenen und gegebenenfalls hydrierten und/oder oxosubstituerten Heteroarylrest oder einen durch einen über ein C-Atom gebundenen und gegebenenfalls hydrierten und/oder oxosubstituerten Heteroarylrest substituiertes Niederalkyl darstellt, dadurch gekennzeichnet, daß man in einer Verbindung der Formel 1, worin R₁ für Formyl, gegebenenfalls verestertes Carboxy oder Hydroxymethyl steht, R₄ eine Aminoschutzgruppe und R₅ Wasserstoff bedeutet, die gegebenenfalls vorhandene freie oder veresterte Carboxygruppe in üblicher Weise zu Formyl reduziert, bzw. eine gegebenenfalls vorhandene Hydroxymethylgruppe in üblicher Weise zu Formyl oxidiert, den entsprechenden Aldehyd der Formel 1, worin R₃ Formyl bedeutet, in einem etherartigen Lösungsmittel in Gegenwart von 1,2-Dibrombutan mit Magnesium und einer Verbindung der Formel 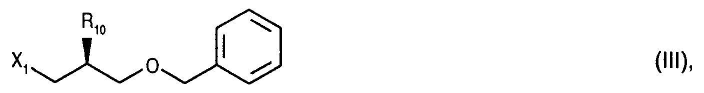 worin X₁ Halogenbedeutet, umsetzt, die erhaltene Verbindung der Formel IV 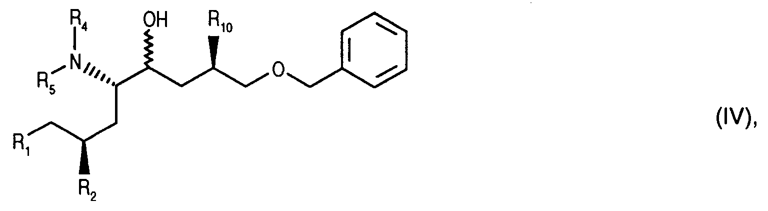 an der Amino- und Hydrogruppe durch eine zweiwertige Schutzgruppe X₂ intermediär schützt, das erhaltene Diatereomerengemisch in üblicher Weise, in die Komponenten auftrennt und aus dem Diastereomeren der Formel V 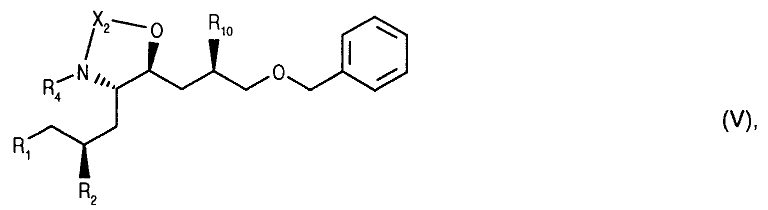 die Benzylgruppe in üblicher Weise abspaltet, die so freigesetzte terminale Hydroxymethylgruppe in üblicher Weise zu Formyl oxidiert, die gebildete Formylgruppe in üblicher Weise zu Carboxy oxidiert, die erhaltene Säure der Formel VI 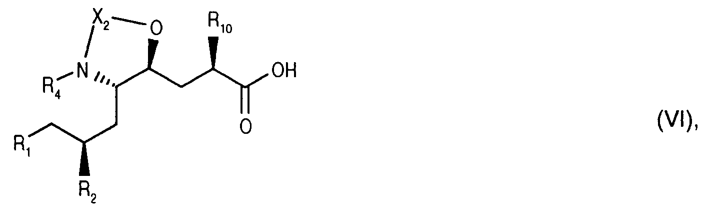 in üblicher Weise mit einem Amin der Formel VII
H₂N-R₁₁ (VII)
kondensiert und aus der erhaltenen Verbindung der Formel VIII 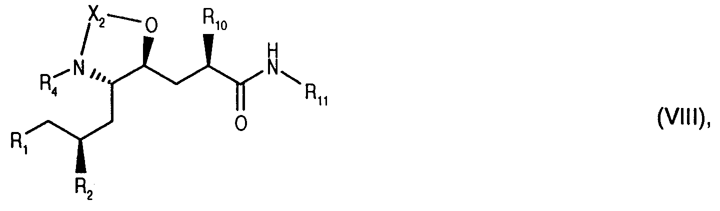 die Schutzgruppen in üblicher Weise abspaltet und gewünschtenfalls eine verfahrensgemäß erhältliche Verbindung in eine andere Verbindung der Formel IIa umwandelt, gegebenenfalls erhältliche Isomerengemische auftrennt und/oder eine verfahrensgemäß erhältliche Verbindung der Formel IIa mit mindestens einer salzbildenden Gruppe in ein Salz oder ein verfahrensgemäß erhältliches Salz in die freie Verbindung oder in ein anderes Salz überführt.
